# EUROPÄISCHE PATENTSCHRIFT

(11) **EP 1 902 037 B1**
(45) Veröffentlichungstag und Bekanntmachung des Hinweises auf die Patenterteilung: **02.09.2009**
(21) Anmeldenummer: 06763989.8
(22) Anmeldetag: 30.06.2006
(51) Int. Cl.: C07D 239/48, A61K 31/505, A61P 35/00, A61P 31/00

(54) **2,4-DIAMINO-PYRIMIDINE ALS AURORA INHIBITOREN**
2,4-DIAMINO-PYRIMIDINES USED AS AURORA INHIBITORS
2,4-DIAMINO-PYRIMIDINES COMME INHIBITEURS D'AURORA

(30) Priorität: 01.07.2005 EP 05106007
(43) Veröffentlichungstag der Anmeldung: 26.03.2008
(73) Patentinhaber: Boehringer Ingelheim International GmbH, 55216 Ingelheim am Rhein (DE); Boehringer Ingelheim Pharma GmbH & Co. KG, 55216 Ingelheim (DE)
(72) Erfinder: ZAHN, Stephan Karl, A-1130 Wien (AT); BOEHMELT, Guido, A-2531 Gaaden (AT); MANTOULIDIS, Andreas, A-1200 Wien (DE); REISER, Ulrich, A-1130 Wien (AT); TREU, Matthias, A-1080 Wien (AT); GUERTLER, Ulrich, A-1120 Wien (AT); SCHOOP, Andreas, 82061 Neuried (AT); SOLCA, Flavio, A-1230 Wien (AT); TONTSCH-GRUNT, Ulrike, A-2500 Baden (AT); BRUECKNER, Ralph, A-1130 Wien (AT); REITHER, Charlotte, A-1230 Wien (AT); HERFURTH, Lars, 55437 Appenheim (DE); KRAEMER, Oliver, A-1050 Wien (AT); STADTMUELLER, Heinz, A-1120 Vienna (AT); ENGELHARDT, Harald, A-2483 Ebreichsdorf (AT)
(74) Vertreter: Hammann, Heinz
(86) Internationale Anmeldenummer: PCT/EP2006/063736
(87) Internationale Veröffentlichungsnummer: WO 2007/003596

(56) Entgegenhaltungen:
- WO-A-02/096888
- WO-A-03/032997

## Beschreibung

Die vorliegende Erfindung betrifft neue 2,4-Diamino-Pyrimidine der allgemeinen Formel (1) 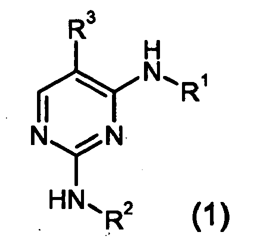 wobei die Reste R¹ bis R³ die in den Ansprüchen und der Beschreibung genannten Bedeutungen haben, deren Isomere, Verfahren zur Herstellung dieser Pyrimidine sowie deren Verwendung als Arzneimittel.

### Hintergrund der Erfindung

Tumorzellen entziehen sich teilweise oder völlig der Regulation und Kontrolle durch den Organismus und zeichnen sich durch ein unkontrolliertes Wachstum aus. Dies beruht einerseits auf dem Verlust von Kontroll-Proteinen, wie z.B. Rb, p16, p21 und p53 als auch auf der Aktivierung von so genannten Beschleunigern des Zellzykluses, den cyclin-abhängigen Kinasen (CDK's).

Studien in Modellorganismen wie *Schizosaccharomyces pombe, Drosophila melanogaster* oder *Xenopus* sowie Untersuchungen in menschlichen Zellen haben gezeigt, dass der Übergang von der G2 Phase zur Mitose durch die CDKl/Cyclin B Kinase reguliert wird (Nurse, 1990). Diese Kinase, die auch als "mitosis promoting factor" (MPF) bezeichnet wird, phosphoryliert und reguliert eine Vielzahl von Proteinen, wie z.B. nukleäre Lamine, Kinesin-ähnliche Motorproteine, Kondensine und Golgi Matrix Proteine, die eine wichtige Rolle beim Abbau der Kernhülle, bei der Zentrosomen Separation, dem Aufbau des mitotischen Spindelapparates, der Chromosomen Kondensation und Abbau des Golgi Apparates spielen (Nigg, 2001). Die Behandlung von humanen Tumorzellen mit Inhibitoren gegen CDKl/Cyclin B, wie z.B. Butyrolacton, führt zu einem Arrest in der G2/M Phase und anschließender Apoptose (Nishio et al., 1996).
Neben den Cyclin-abhängigen Kinasen spielen des weiteren die so genannten Polo-like Serin/Threonin-Kinasen (PLK-1, PLK-2, PLK-3 und PLK-4) eine wichtige Rolle bei der Regulation des eukaryontischen Zellzykluses. Besonders für PLK-1 wurde eine zentrale Rolle in der Regulation der Mitosephase gezeigt. PLK-1 ist für die Reifung der Zentrosomen, für die Aktivierung der Phosphatase Cdc25C, sowie für die Aktivierung des Anaphase Promoting Complex verantwortlich (Glover et al., 1998; Qian et al., 2001). Die Injektion von PLK-1 Antikörpern führt zu einem G2 Arrest in nicht transformierten Zellen, während Tumorzellen in der Mitosephase arretieren (Lane und Nigg, 1996).

Des Weiteren kann ein Arrest in der G2/M Phase auch durch Inhibition von bestimmten Motorproteinen, den so genannten Kinesinen wie z.B. Eg5 (Mayer et al., 1999), oder durch Mikrotubuli stabilisierende oder destabilisierende Agenzien (z.B. Colchicin, Taxol, Etoposid, Vinblastin, Vincristin) ausgelöst werden (Schiff und Horwitz, 1980).

Die Ser/Thr Kinasen der Aurora Familie regulieren verschiedene Prozessse der Zellteilung. Hierzu gehören Chromosomen-Kondensation, Spindeldynamik, Kinetochor-Mikrotubulus Wechselwirkungen, Chromosomen-Orientierung, Einrichten der Metaphaseplatte und Zytokinese (Meraldi et al., 2004; Carmena und Earnshaw, 2003; Andrews et al., 2003). Drei Familienmitglieder sind in Säugern beschrieben - Aurora A, B und C. Aurora Kinasen des A- und B-Typs existieren auch in *Caenorhabditis elegans* und *Drosophila melanogaster,* wohingegen Hefen lediglich ein einziges Aurora-Gen enthalten, das unter dem Namen IPL1 (in *S cerevisiae*), bzw. ARK1 (in *S.pombe*) bekannt ist. Allen Aurora Proteinen ist eine ähnliche Gesamtstruktur gemeinsam, die einen variablen N-Terminus, eine gut konservierte mittlere Kinasedomaine und einen kurzen C-terminalen Teil umfasst. Trotz ihrer Sequenzähnlichkeit zeigen die Kinasen der Aurora Familie unterschiedliche subzelluläre Lokalisation, die mit spezialisierten Funktionen verbunden ist.

So ist Aurora A in der Interphase an Zentrosomen zu finden und während der Mitose sowohl an Zentrosomen als auch an Spindelmikrotubuli in der Nähe der Pole.

Entsprechend - und durch RNA Interferenz-Experimente bestätigt- ist Aurora A essentiell für den Eintritt in die Mitose, da Zentrosom-Reifung und -Trennung bei Verlust von Aurora A nicht mehr erfolgen können. Es gibt verschiedene Aktivatoren für Aurora A, wie z.B. TPX2, Ajuba oder Proteinphosphataseinhibitor-2. TPX2 scheint verantwortlich für die korrekte zeitliche und räumliche Aktivierung von Aurora A an polnahen Spindelmikrotubuli zu sein (Hirota et al., 2003; Bayliss et al., 2003; Eyers und Maller, 2004; Kufer et al., 2002; Satinover et al., 2004).

Aurora B assoziiert in der frühen Prophase mit kondensierenden Chromosomen, lokalisiert in der Metaphase an Zentromeren, re-lokalisiert danach in der mittleren Zone der Zentralspindel und konzentriert sich zum Zeitpunkt der Zytokinese schließlich am sogenannten Flemming- oder Mittelkörper, einem eng begrenzten Bereich zwischen den entstehenden Tochterzellen. Diese charakteristischen räumlichen Änderungen während des Ablaufs der Mitose rechtfertigen Aurora B als sogenanntes "chromosomal passenger" Protein zu bezeichnen. Es sind zumindest drei weitere "chromosomal passenger' Proteine bekannt, die mit Aurora B einen Komplex bilden. Diese sind INCENP (inner centromere protein), Survivin und Borealin (Andrews et al., 2003; Carmena and Earnshaw, 2003; Meraldi et al., 2004). Ein wichtiger Kontaktpunkt zwischen Aurora B und diesem Komplexes wird durch den C-Terminus von INCENP, die sogenannte "IN-box", vermittelt. Die "IN-box" ist die am stärksten konservierte Region von INCENP. Sie bindet und aktiviert Aurora B und wird von dieser Kinase phosphoryliert (Adams et al., 2000; Bishop und Schumacher, 2002; Kaitna et al., 2000; Bolton et al., 2002; Honda et al., 2003).

Aurora C ist innerhalb der Aurora-Familie das am wenigsten charakterisierte Mitglied. Aurora C bindet ebenfalls an INCENP und verhält sich als "chromosomal passenger" Protein, wobei jedoch maximale Expressionsspiegel nach denen von Aurora B gemessen werden. Aurora C kann vermutlich einige Funktionen von Aurora B übernehmen, da z.B. der mehrkernige Phänotyp Aurora B-depletierter Zellen durch Expression von Aurora C normalisiert werden kann (Sasai et al., 2004; Li et al., 2004).

Aurora B phosphoryliert Histon H3 an Ser10 und Ser28. Obwohl diese Phosphorylierung mit dem Zeitpunkt der Chromosomenkondensation zusammenfällt, ist die Auswirkung dieses Ereignisses erst in einer späteren Zellzyklusphase relevant. Hierfür spricht, das Histone H3 mit Ser10-Phosphorylierung und gleichzeitiger Lys9- Dreifachmethylierung an zentromernahem Heterochromatin in mitotischen Chromosomen angereichert ist. Derartig modifiziertes Histon H3 verhindert die Bindung von heterochromatin protein 1 (HP1) und erlaubt den Zugriff des "chromosomal passenger" Protein Komplexes an zentromere Kinetochorregionen (Hirota T. et al. Manuscript in Preparation).

Eine Funktion von Aurora B, die durch Inhibition von Aurora B evident wird, liegt im Zusammenführen verschiedener Proteine am Kinetochor während der Metaphase (Ditchfield et al., 2003; Hauf et al., 2003; Murata-Hori und Wang, 2002; Vigneron et al., 2004). Aurora B spielt hierbei eine zentrale Rolle in einem Signalweg, der syntelische (fehlerhafte, da nur von einem Spindelpol ausgehende) Kinetochor-Anknüpfungen von Mikrotubuli aufspürt und korrigiert (Andrews et al., 2003; Carmena und Earnshaw, 2003; Meraldi et al., 2004). Wird dieser Anknüpfungszustand nicht korrigiert, kommt es zu Fehlern in der Chromosomensegregation. Die Aurora B-vermittelte Phosphorylierung der Mikrotubulus-Depolymerase MCAK wird mit diesem Korrekturmechanismus in Verbindung gebracht (Gorbsky, 2004).

Aurora B phosphoryliert auch Proteine, die für die Ausbildung der Teilungsfurche und Zytokinese wichtig sind, wie z.B. MgcRacGAP, die leichte regulatorische Kette von Myosin II, vimentin, desmin, GFAP (glial fibrillary acidic protein), sowie die Kinesine MKLP1 und MKLP2, von denen MKLP2 vermutlich dafür verantwortlich ist, den Transfer des "chromosomal passenger" Protein Komplexes von den Kinetochoren zum Mittelkörper zu vollziehen (Gruneberg et al., 2004).

Aufgrund der verschiedenen Aufgaben von Aurora B im Zellzyklus, war die Erkenntnis überraschend, dass Inhibition von Aurora B in Tumorzellen nicht einen mitotischen Arrest, sondern ein Fortführen des Zellzyklus ohne Zytokinese bewirkt (Hauf et al., 2003). Wegen der Anhäufung von syntelischen Mikrotubulus-Kinetochor Anknüpfungen und folglich Chromosomen-Fehlsegregationen kommt es zu massiver Polyploidie, die letztlich in Apoptose mündet. Auch gleichzeitige Inhibition von Aurora A kann diesen Phänotyp nicht beeinflussen (Keen und Taylor, 2004).

Anfänglich gab es vornehmlich Hinweise für die onkogene Aktivität von Aurora A (z.B. Transformation von Mausfibroblasten nach Überexpression), wohingegen für Aurora B derartige Hinweise nur indirekt vorlagen (Zhou et al., 1998; Bischoff et al., 1998; Katayama et al., 1999). Dies hat sich mit dem Befund geändert, dass Überexpression von Aurora B in embryonalen Hamsterzellen und deren Verwendung in Xenograft-Experimenten direkt die Tumor-Inzidenz, -Größe und Invasivität erhöht. Entsprechende Tumore zeigten Chromsomeninstabilität und erhöhte Histon H3 Ser10-Phosphorylierung (Ota et al., 2002). Diese Ergebnisse untermauern die Wichtigkeit von Aurora B während der Tumorgenese.

Pyrimidine sind allgemein als Inhibitoren von Kinasen bekannt. So werden zum Beispiel substituierte Pyrimidine mit einem nicht-aromatischen Rest in 4-Position als aktive Komponenten mit Antikrebswirkung in den Internationalen Patentanmeldung WO 02/096888 und WO 03/032997 beschrieben.

Der vorliegenden Erfindung liegt die Aufgabe zugrunde, neue Wirkstoffe aufzuzeigen, welche zur Vorbeugung und/oder Behandlung von Krankheiten, die durch exzessive oder anomale Zellproliferation charakterisiert sind, eingesetzt werden können.

### Detaillierte Beschreibung der Erfindung

Es wurde nun überraschenderweise gefunden, dass Verbindungen der allgemeinen Formel (1), worin die Reste R¹, R² und R³ die nachstehend genannten Bedeutungen haben, als Inhibitoren spezifischer Zellzykluskinasen wirken. Somit können die erfindungsgemäßen Verbindungen beispielsweise zur Behandlung von Erkrankungen, die mit der Aktivität spezifischer Zellzykluskinasen in Zusammenhang stehen und durch exzessive oder anomale Zellproliferation charakterisiert sind, verwendet werden.

Die vorliegende Erfindung betrifft Verbindungen der allgemeinen Formel (1) 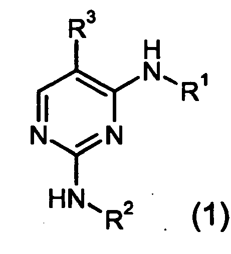 worin
R¹ ein Rest, substituiert mit R⁵ und gegebenenfalls mit einem oder mehreren R⁴, ausgewählt aus der Gruppe bestehend aus C₃₋₁₀Cycloalkyl und 3-8 gliedriges Heterocycloalkyl;
R² ein Rest, gegebenenfalls substituiert mit einem oder mehreren R⁴, ausgewählt aus der Gruppe bestehend aus C₁₋₆Alkyl, C₃-₁₀Cycloalkyl, 3-8 gliedriges Heterocycloalkyl, C₆₋₁₅Aryl und 5-12 gliedriges Heteroaryl;
R³ ein Rest ausgewählt aus der Gruppe bestehend aus Wasserstoff, Halogen, -CN, -NO₂, C₁₋₄Alkyl, C₁₋₄Haloalkyl, C₃₋₁₀Cycloalkyl, C₄₋₁₆Cycloalkylalkyl und C₇₋₁₆Arylalkyl;
R⁴ ein Rest ausgewählt aus der Gruppe bestehend aus R^{a}, R^{b} und R^{a} substituiert mit einem oder mehreren, gleich oder verschiedenen R^{c} und/oder R^{b};
R⁵ ein geeigneter Rest ausgewählt aus der Gruppe bestehend aus -C(O)R^{c}, -C(O)NR^{c}R^{c}, -S(O)₂R^{c}, -N(R^{f})S(O)₂R^{c}, -N(R^{f})C(O)R^{c}, -N(R^{f})C(O)OR^{c}, und -N(R^{f})C(O)NR^{c}R^{c};
jedes R^{a} ist unabhängig voneinander ausgewählt aus der Gruppe bestehend aus C₁₋₄Alkyl, C₃₋₁₀Cycloalkyl, C₄₋₁₆Cycloalkylalkyl, C₆₋₁₀Aryl, C₇₋₆Arylalkyl, 2-6 gliedriges Heteroalkyl, 3-8 gliedriges Heterocycloalkyl, 4-14 gliedriges Heterocycloalkylalkyl, 5-12 gliedriges Heteroaryl und 6-18 gliedriges Heteroarylalkyl;
jedes R^{b} ist eine geeigneter Rest und jeweils unabhängig voneinander ausgewählt aus der Gruppe bestehend aus =O, -OR^{c}, C₁₋₃Haloalkyloxy, -OCF₃, =S, -SR^{c}, =NR^{c}, =NOR^{c}, -NR^{c}R^{c}, Halogen, -CF₃, -CN, -NC, -OCN, -SCN, -NO₂, -S(O)R^{c}, -S(O)₂R^{c}, -S(O)₂OR^{c},-S(O)NR^{c}R^{c}, -S(O)₂NR^{c}R^{c}, -OS(O)R^{c}, -OS(O)₂R^{c}, -OS(O)₂OR^{c}, -OS(O)₂NR^{c}R^{c}, -C(O)R^{c},-C(O)OR^{c}, -C(O)NR^{c}R^{c}, -CN(R^{f})NR^{c}R^{c}, -CN(OH)R^{c}, -CN(OH)NR^{c}R^{c}, -OC(O)R^{c}, -OC(O)OR^{c}, -OC(O)NR^{c}R^{c}, -OCN(R^{f})NR^{c}R^{c}, -N(R^{f})C(O)R^{c}, -N(R^{f})C(S)R^{c},-N(R^{f})S(O)₂R^{c}, -N(R^{f})C(O)OR^{c}, -N(R^{f})C(O)NR^{c}R^{c}, -[N(R^{f})C(O)]₂R^{c}, -N[C(O)]₂R^{c}, -N[C(O)]₂OR^{c}, - [N(R^{f})C(O)]₂OR^{c} und -N(R^{f})CN(R^{f})NR^{c}R^{c};
jedes R^{c} ist unabhängig voneinander Wasserstoff oder ein gegebenenfalls mit einem oder mehreren, gleich oder verschiedenen R^{d} und/oder R^{e} substituierter Rest ausgewählt aus der Gruppe bestehend aus C₁₋₆Alkyl, C₃-₁₀Cycloalkyl, C₄₋₁₁Cycloalkylalkyl, C₆₋₁₀Aryl, C₇₋₁₆Arylalkyl, 2-6 gliedriges Heteroalkyl, 3-8 gliedriges Heterocycloalkyl, 4-14 gliedriges Heterocycloalkylalkyl, 5-12 gliedriges Heteroaryl und 6-18 gliedriges Heteroarylalkyl,
jedes R^{d} ist unabhängig voneinander Wasserstoff oder ein gegebenenfalls mit einem oder mehreren, gleich oder verschiedenen R^{e} und/oder R^{f} substituierter Rest ausgewählt aus der Gruppe bestehend aus C₁₋₆Alkyl, C₃₋₈Cycloalkyl, C₄₋₁₁Cycloalkylalkyl, C₆₋₁₀Aryl, C₇₋₁₆Arylalkyl, 2-6 gliedriges Heteroalkyl, 3-8 gliedriges Heterocycloalkyl, 4-14 gliedriges Heterocycloalkylalkyl, 5-12 gliedriges Heteroaryl und 6-18 gliedriges Heteroarylalkyl;
jedes R^{e} ist eine geeigneter Rest und jeweils unabhängig voneinander ausgewählt aus der Gruppe bestehend aus =O, -OR^{f}, C₁₋₃Haloalykloxy, -OCF₃, =S, -SR^{f}, =NR^{f}, =NOR^{f}, -NR^{f}R^{f}, Halogen, -CF₃, -CN, -NC, -OCN, -SCN, -NO₂, -S(O)R^{f}, -S(O)₂R^{f}, -S(O)₂OR^{f},-S(O)NR^{f}R^{f}, -S(O)₂NR^{f}R^{f}, -OS(O)R^{f}, -OS(O)₂R^{f}, -OS(O)₂OR^{f} -OS(O)₂NR^{f}R^{f}, -C(O)R^{f},-C(O)OR^{f}, -C(O)NR^{f}R^{f}, -CN(R^{g})NR^{f}R^{f}, -CN(OH)R^{f}, -C(NOH)NR^{f}R^{f}, -OC(O)R^{f}, -OC(O)OR^{f}, -OC(O)NR^{f}R^{f}, -OCN(R^{g})NR^{f}R^{f}, -N(R^{g})C(O)R^{f}, -N(R^{g})C(S)R^{f}, -N(R^{g})S(O)₂R^{f}, -N(R^{d})C(O)OR^{f}, -N(R^{g})C(O)NR^{f}R^{f}, und -N(R^{g})CN(R^{f})NR^{f}R^{f};
jedes R^{f} ist unabhängig voneinander Wasserstoff oder ein gegebenenfalls mit einem oder mehreren, gleich oder verschiedenen R^{g} substituierter Rest ausgewählt aus der Gruppe bestehend aus , C₁₋₆Alkyl, C₃₋₈Cycloalkyl, C₄₋₁₁Cycloalkylalkyl, C₆₋₁₀Aryl, C₇₋₁₆Arylalkyl, 2-6 gliedriges Heteroalkyl, 3-8 gliedriges Heterocycloalkyl, 4-14 gliedriges Heterocycloalkylalkyl, 5-12 gliedriges Heteroaryl und 6-18 gliedriges Heteroarylalkyl;
jedes R^{g} ist unabhängig voneinander Wasserstoff, C₁₋₆Alkyl, C₃₋₈Cycloalkyl, C₄₋₁₁Cycloalkylalkyl, C₆₋₁₀Aryl, C₇₋₁₆Arylalkyl, 2-6 gliedriges Heteroalkyl, 3-8 gliedriges Heterocycloalkyl, 4-14 gliedriges Heterocycloalkyl, 5-12 gliedriges Heteroaryl und 6-18 gliedriges Heteroarylalkyl bedeuten, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Ein Aspekt der Erfindung sind Verbindungen der allgemeinen Formel (1), wobei R³ ein Rest ausgewählt aus der Gruppe bestehend aus Halogen und C₁₋₄Haloalkyl bedeutet.

Ein weiterer Aspekt der Erfindung sind Verbindungen der allgemeinen Formel (1), wobei R³ -CF₃ bedeutet.

Ein weiterer Aspekt der Erfindung sind Verbindungen der allgemeinen Formel (1), wobei R² C₆₋₁₀Aryl oder 5-12 gliedrige Heteroaryl, gegebenenfalls substituiert mit einem oder mehreren R⁴, bedeutet.

Ein weiterer Aspekt der Erfindung sind Verbindungen der allgemeinen Formel (1), wobei R² Phenyl, gegebenenfalls substituiert mit einem oder mehreren R⁴, bedeutet.

Ein weiterer Aspekt der Erfindung sind Verbindungen der allgemeinen Formel (1A), 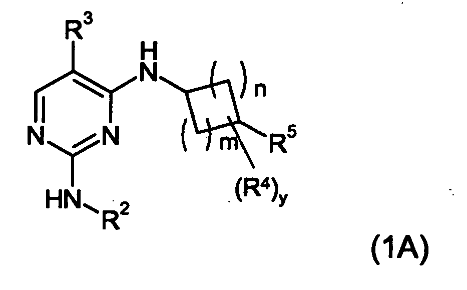 wobei
n gleich 0 oder 1, und
m gleich 1-5, und
y gleich 0 bis 6, bedeutet und die übrigen Reste wie vorstehend definiert sind.

Ein weiterer Aspekt der Erfindung sind Verbindungen der allgemeinen Formel (1A), wobei R³ ein Rest ausgewählt aus der Gruppe bestehend aus Halogen und C₁₋₄Haloalkyl bedeutet.

Ein weiterer Aspekt der Erfindung sind Verbindungen der allgemeinen Formel (1A), wobei R³ CF₃ bedeutet.

Ein weiterer Aspekt der Erfindung sind Verbindungen der allgemeinen Formel (1A), wobei R² C₆₋₁₀Aryl oder 5-12 gliedriges Heteroaryl, gegebenenfalls substituiert mit einem oder mehreren R⁴, bedeutet.

Ein weiterer Aspekt der Erfindung sind Verbindungen der allgemeinen Formel (1A), wobei R² Phenyl, gegebenenfalls substituiert mit einem oder mehreren R⁴, bedeutet.

Ein weiterer Aspekt der Erfindung sind Verbindungen, oder deren pharmazeutisch wirksamen Salze, der allgemeinen Formel (1) oder (1A), zur Verwendung als Arzneimittel.

Ein weiterer Aspekt der Erfindung sind Verbindungen, oder deren pharmazeutisch wirksamen Salze, der allgemeinen Formel (1) oder (1A), zur Herstellung eines Arzneimittels mit antiproliferativer Wirkung.

Ein weiterer Aspekt der Erfindung sind pharmazeutische Zubereitungen, enthaltend als Wirkstoff eine oder mehrere Verbindungen der allgemeinen Formel (1) oder (1A) oder deren physiologisch verträglichen Salze gegebenenfalls in Kombination mit üblichen Hilfs- und/oder Trägerstoffen.

Ein weiterer Aspekt der Erfindung ist die Verwendung von Verbindungen der allgemeinen Formel (1) oder (1A) zur Herstellung eines Arzneimittels zur Behandlung und/oder Prävention von Krebs, Infektionen, Entzündungs- oder Autoimmunerkrankungen.

Ein weiterer Aspekt der Erfindung sind pharmazeutische Präparation umfassend eine Verbindung der allgemeinen Formel (1) oder (1A) und mindestens eine weitere zytostatische oder zytotoxische, von Formel (1) verschiedene Wirksubstanz, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

### Definitionen

Wie hierin verwendet treffen folgenden Definitionen zu, falls nicht anders beschrieben.

Unter Alkyl-Substitutenten sind jeweils gesättigte, ungesättigte, unverzweigte oder verzweigte aliphatische Kohlenwasserstoffreste (Alkylrest) zu verstehen und umfasst sowohl gesättigte Alkylreste als auch ungesättigte Alkenyl- und Alkinylreste. Alkenyl-Substituenten sind jeweils unverzweigt oder verzweigte, ungesättigte Alkylreste, die mindestens eine Doppelbindung aufweisen. Unter Alkinyl-Substituenten sind jeweils unverzweigt oder verzweigte, ungesättigte Alkylreste, die mindestens eine Dreifachbindung aufweisen, zu verstehen.

Heteroalkyl repräsentiert unverzweigt oder verzweigte aliphatische Kohlenwasserstofiketten, die 1 bis 3 Heteroatome enthalten, wobei jedes der verfügbaren Kohlenstoff- und Heteroatome in der Heteroalkylkette gegebenenfalls jeweils unabhängig voneinander substituiert sein kann und die Heteroatome unabhängig voneinander ausgewählt werden aus der Gruppe bestehend aus O, N, P, PO, PO₂, S, SO und SO₂ (z. B. Dimethylaminomethyl, Dimethylaminoethyl, Dimethylaminopropyl, Diethylaminomethyl, Diethylaminoethyl, Diethylaminopropyl, 2-Disopropylaminoethyl, Bis-2-methoxyethylamino, [2-(Dimethylamino-ethyl)-ethyl-amino]-methyl, 3-[2-(Dimethylamino-ethyl)-ethyl-amino]-propyl, Hydroxymethyl, 2-Hydroxyethyl, 3-Hydroxypropyl, Methoxy, Ethoxy, Propoxy, Methoxymethyl, 2-Methoxyethyl).

Halogenalkyl bezieht sich auf Alkylreste, in denen ein oder mehrere Wasserstoffatome durch Halogenatome ersetzt sind. Halogenalkyl umfasst sowohl gesättigte Alkylreste als auch ungesättigte Alkenyl- und Alkinylreste, wie beispielsweise -CF₃, -CHF₂, -CH₂F, -CF₂CF₃,-CHFCF₃, -CH₂CF₃, -CF₂CH₃, -CHFCH₃, -CF₂CF₂CF₃, -CF₂CH₂CH₃, -CF=CF₂, -CCl=CH₂, -CBr=CH₂, -CJ=CH₂, -C=C-CF₃, -CHFCH₂CH₃ und -CHFCH₂CF₃. Halogen bezieht sich auf Fluor-, Chlor-, Brom- und/oder Jodatome.

Unter Cycloalkyl ist ein mono- oder polyzyklischer Ring zu verstehen, wobei das Ringsystem ein gesättigter Ring aber auch ein ungesättigter, nichtaromatischer Ring beziehungsweise eine Spiroverbindung sein kann, welcher gegebenenfalls auch Doppelbindungen enthalten kann, wie zum Beispiel Cyclopropyl, Cyclopropenyl, Cyclobutyl, Cyclobutenyl, Cyclopentyl, Cyclopentenyl, Cyclohexyl, Cyclohexenyl, Cycloheptanyl, Cycloheptenyl, Norbornyl, Norbornenyl, Indanyl, Adamantyl Spiroheptanyl und Spiro[4.2]heptanyl.

Cycloalkylalkyl umfasst eine nicht-zyklische Alkylgruppe, in der ein an einem Kohlenstoffatom gebundenes Wasserstoffatom durch eine Cycloalkylgruppe ersetzt ist.

Aryl bezieht sich auf monozyklische oder bizyklische Ringe mit 6 -12 Kohlenstoffatomen wie beispielsweise Phenyl und Naphthyl.
Arylalkyl umfasst eine nicht-zyklische Alkylgruppe, in der ein an einem Kohlenstoffatom gebundenes Wasserstoffatom durch eine Arylgruppe ersetzt ist.

Unter Heteroaryl sind mono- oder polyzyklische Ringe zu verstehen, welche anstelle eines oder mehrere Kohlenstoffatome ein oder mehrere, gleich oder verschiedene Heteroatome enthalten, wie z.B. Stickstoff-, Schwefel- oder Sauerstoffatome. Beispielsweise genannt seien Furyl, Thienyl, Pyrrolyl, Oxazolyl, Thiazolyl, Isoxazolyl, Isothiazolyl, Pyrazolyl, Imidazolyl, Triazolyl, Tetrazolyl, Oxadiazolyl, Thiadiazolyl, Pyridyl, Pyrimidyl, Pyridazinyl, Pyrazinyl und Triazinyl. Beispiele für bizyklische Heteroarylreste sind Indolyl, Isoindolyl, Benzofuranyl, Benzothienyl, Benzoxazolyl, Benzothiazolyl, Benzisoxazolyl, Benzisothiazolyl, Benzimidazolyl, Indazolyl, Isoquinolinyl, Quinolinyl, Quinoxalinyl, Cinnolinyl, Phthalazinyl, Quinazolinyl und Benzotriazinyl, Indolizinyl, Oxazolopyridinyl, Imidazopyridinyl, Naphthyridinyl, Indolinyl, Isochromanyl, Chromanyl, Tetrahydroisochinolinyl, Isoindolinyl, Isobenzotetrahydrofuranyl, Isobenzotetrahydrothienyl, Isobenzothienyl, Benzoxazolyl, Pyridopyridinyl, Benzotetrahydrofuranyl, Benzotetrahydrothienyl, Purinyl, Benzodioxolyl, Triazinyl, Phenoxazinyl, Phenothiazinyl, Pteridinyl, Benzothiazolyl, Imidazopyridinyl, Imidazothiazolyl, Dihydrobenzisoxazinyl, Benzisoxazinyl, Benzoxazinyl, Dihydrobenzisothiazinyl, Benzopyranyl, Benzothiopyranyl, Coumarinyl, Isocoumarinyl, Chromonyl, Chromanonyl, Pyridinyl-*N*-oxid Tetrahydroquinolinyl, Dihydroquinolinyl, Dihydroquinolinonyl, Dihydroisoquinolinonyl, Dihydrocoumarinyl, Dihydroisocoumarinyl, Isoindolinonyl, Benzodioxanyl, Benzoxazolinonyl, Pyrrolyl-*N-*oxid, Pyrimidinyl-*N*-oxid, Pyridazinyl-*N*-oxid, Pyrazinyl-*N*-oxid, Quinolinyl-*N*-oxid, Indolyl-*N*-oxid, Indolinyl-*N*-oxid, Isoquinolyl-*N*-oxid, Quinazolinyl-*N*-oxid, Quinoxalinyl-*N*-oxid, Phthalazinyl-*N*-oxid, Imidazolyl-*N*-oxid, Isoxazolyl-*N*-oxid, Oxazolyl-*N*-oxid, Thiazolyl-*N*-oxid, Indolizinyl-*N*-oxid, Indazolyl-*N*-oxid, Benzothiazolyl-*N*-oxid, Benzimidazolyl-*N*-oxid, Pyrrolyl-*N*-oxid, Oxadiazolyl-*N*-oxid, Thiadiazolyl-*N*-oxid, Triazolyl-*N*-oxid, Tetrazolyl-*N*-oxid, Benzothiopyranyl-*S-*oxid und Benzothiopyranyl-*S,S-*dioxid.

Heteroarylalkyl umfasst eine nichtzyklische Alkylgruppe, in der ein an einem Kohlenstoffatom gebundenes Wasserstoffatom durch eine Heteroarylgruppe ersetzt ist.

Heterocycloalkyl bezieht sich auf 3-12 Kohlenstoffatome umfassende gesättigte oder ungesättigte, nicht aromatische mono-, polyzyklische oder überbrückte polyzyklische Ringe oder Spiroverbindungen, welche anstelle eines oder mehrere Kohlenstoffatome Heteroatome, wie Stickstoff, Sauerstoff oder Schwefel, tragen. Beispiele für solche Heterocylylreste sind Tetrahydrofuranyl, Pyrrolidinyl, Pyrrolinyl, Imidazolidinyl, Imidazolinyl, Pyrazolidinyl, Pyrazolinyl, Piperidinyl, Piperazinyl, Indolinyl, Isoindolinyl, Morpholinyl, Thiomorpholinyl, Homomorpholinyl, Homopiperidinyl, Homopiperazinyl, Homothiomorpholinyl, Thiomorpholinyl-*S-*oxid, Thiomorpholinyl-*S,S*-dioxid, Tetrahydropyranyl, Tetrahydrothienyl, Homothiomorpholinyl-*S,S*-Dioxid, Oxazolidinonyl, Dihydropyrazolyl, Dihydropyrrolyl, Dihydropyrazinyl, Dihydropyridinyl, Dihydropyrimidinyl, Dihydrofuryl, Dihydropyranyl, Tetrahydrothienyl-*S-*oxid, Tetrahydrothienyl-*S,S*-dioxid, Homothiomorpholinyl-*S*-oxid, 2-Oxa-5-azabicyclo[2.2.1]heptan, 8-Oxa-3-aza-bicyclo[3.2.1]octan, 3,8-Diaza-bicyclo[3.2.1]octan, 2,5-Diaza-bicyclo[2.2.1]heptan, 3,8-Diaza-bicyclo[3.2.1]octan, 3,9-Diaza-bicyclo[4.2.1]nonan und 2,6-Diaza-bicyclo[3.2.2]nonan.

Heterocycloalkylalkyl.bezieht sich auf eine nicht-zyklische Alkylgruppe, in der ein an einem Kohlenstoffatom gebundenes Wasserstoffatom durch eine Heterocycloalkylgruppe ersetzt ist.

**Abkürzungsliste**

| | | | |
|---|---|---|---|
| Äq.,eq | Äquivalent(e) | IR | Infrarotspektroskopie |
| Ac | Acetyl | Kat., kat | Katalysator, katalytisch |
| Boc | t-Butyloxycarbonyl | konz. | konzentriert |
| Bu | Butyl | Kp., Sdp. | Koch- oder Siedepunkt |
| BuLi | n-Butyllithium | LC | liquid chromatography |
| c | Konzentration | Hünig-Base | *N*-Ethyl-diisopropylamin |
| cHex | Cyclohexan | i | iso |
| CDI | Carbonyldiimidazol | mCPBA | meta-Chlorperbenzoesäure |
| CSI | Chlorsulfonylisocyanat | min | Minuten |
| DC, TLC | Dünnschichtchromatographie | Me | methyl |
| DCC | Dicyclohexylcarbodiimid | MS | Massenspektrometrie |
| DCM | Dichlormethan | NMP | N-Methylpyrrolidone |
| DIPEA | Ethyldiisopropylamin (Hünig Base) | NMP | nuclear magnetic resonance |
| DMAP | *N,N*-Dimethylaminopyridin | Ph | phenyl |
| DMF | *N,N-*Dimethylformamid | Pr | propyl |
| DMA | *N,N-*Dimethylacetamid | rac | racemisch |
| DMSO | Dimethylsulfoxid | R_{f} (Rf) | Retentionsfaktor |
| EE | Ethylacetat (Essigsäureethylester) | RP | Reversed Phase |
| ESI | electron spray ionization RT | | Raumtemperatur oder Retentionszeit (HPLC) |
| Et | Ethyl | *t* | tertiär |
| h | Stunde | THF | Tetrahydrofuran |
| Hex | hexyl | TBTU | *O*-(Benzotriazol-1-yl)-*N,N,N',N'*-tetramethyluronium tetrafluorborat |
| HPLC | high performance liquid chromatography | UV | Ultraviolett |
| LDA | Lithiumdiisopropylamid | | |

Die nachfolgenden Beispiele illustrieren die vorliegende Erfindung ohne sie jedoch in ihrem Umfang einzuschränken.

### Allgemeines

Alle Reaktionen werden - sofern nicht anders beschrieben - in kommerziell erhältlichen Apparaturen nach im chemischen Laboratorien gängigen Verfahren durchgeführt.
Die benutzten Lösungsmittel werden in *pro analysi* Qualität gekauft und ohne weitere Reinigung eingesetzt. Alle Reagenzien werden ebenfalls direkt ohne weitere Reinigung in der Synthese verwendet.

Luft- und/oder feuchtigkeitsempfindliche Ausgangsstoffe werden unter Argon aufbewahrt und entsprechende Reaktionen und Manipulationen mit letzteren unter Schutzgas durchgeführt (Stickstoff oder Argon).

### Chromatographie

Für die präparative Mitteldruck-Chromatographie (MPLC, Normalphase) wird Kieselgel der Firma Millipore (Bezeichnung: Granula Silica Si-60A 35-70 µm) oder C-18 RP-Kieselgel (RP-Phase) der Firma Macherey Nagel (Bezeichnung: Polygoprep 100-50 C18) eingesetzt.
Die Dünnschichtchromatographie erfolgte auf DC-Fertigplatten Kieselgel 60 auf Glas (mit Fluoreszenzindikator F-254) der Fa. Merck.
Für die präparative Hochdruck-Chromatographie (HPLC) werden Säulen der Firma Waters (Bezeichnung: XTerra Prep. MS C18, 5 µM, 30*100 mm bzw. XTerra Prep. MS C18, 5 µm, 50*100 mm OBD oder Symmetrie C 18, 5 µm, 19*100mm), die analytische HPLC (Reaktionskontrolle) wird mit Säulen der Firma Agilent (Bezeichnung: Zorbax SB-C8, 5 µm, 21,2*50mm) durchgeführt.
Für die chirale Hochruckchromatographie (HPLC) werden Säulen der Firma Daicel Chemical Industries, LTD. (Bezeichnung: Chiralpak AD-H oder Chiralpak AS oder Chiracel OD-RH oder Chiracel OD-H oder Chiracel OJ-H in diversen Größen und 5 µm Material) verwendet.

### Kernresonanz Spektroskopie (NMR)

Die Kernresonanz-Spektren werden in deuteriertem Dimethylsulfoxid-d6 als Lösungsmittel aufgenommen. Werden andere Lösungsmittel verwendet, sind diese explizit in den Beispielen oder in den Methoden vermerkt. Die chemische Verschiebung wird relativ zu dem Standard Tetramethylsilan (δ= 0.00 ppm) angegeben. Die Messungen erfolgt auf einem Avance 400 (400MHz-NMR-Spektrometer) oder einem Avance 500 (500MHz-NMR-Spektrometer) der Firma Bruker Biospin GmbH.

### HPLC-Massenspektroskopie/UV-Spektrometrie

Die Retentionszeiten/MS-ESI⁺ zur Charakterisierung der Beispiele werden mit Hilfe einer HPLC-MS Anlage (high performance liquid chromatography mit Massendetektor) der Firma Agilent erzeugt.
Die Anlage ist so aufgebaut, dass anschließend an die Chromatographie (Säule: XTerra MS C18, 2,5 µm, 2,1*30mm, Fa. Waters oder Synergi POLAR-RP 80A; 4µm, Fa. Phenomenex) ein Diodenarry-Detektor (G1315B von Fa. Agilent) und ein Massendetektor (1100 LS-MSD SL; G1946D; Fa. Agilent) in Reihe geschaltet sind.
Diese Anlage wird mit einem Fluß von 1.1 ml/min betrieben. Für einen Trennvorgang wird ein Gradient innerhalb von 3.1 min durchlaufen (Gradient Anfang: 95% Wasser und 5% Acetonitril; Gradient Ende: 5% Wasser und 95% Acetonitril; den beiden Lösungsmitteln wird jeweils 0,1 %Ameisensäure beigemischt).

### Schmelzpunkte

Schmelzpunkte wurden an einem Gerät der Fa. Büchi vom Typ B-540 ermittelt und sind nicht korrigiert.

Soweit die Herstellung der Ausgangsverbindungen nicht beschrieben wird, sind diese kommerziell erhältlich oder analog zu bekannten Verbindungen oder hier beschriebenen Verfahren herstellbar.

### Herstellung der erfindungsgemäßen Verbindungen

Die Herstellung der erfindungsgemäßen Verbindungen kann nach den im Folgenden beschriebenen Syntheseverfahren erfolgen, wobei die Substituenten der allgemeinen Formeln die zuvor genannten Bedeutungen haben. Diese Verfahren sind als Erläuterung der Erfindung zu verstehen ohne selbige auf deren Gegenstand und den Umfang der beanspruchten Verbindungen auf diese Beispiele zu beschränken.

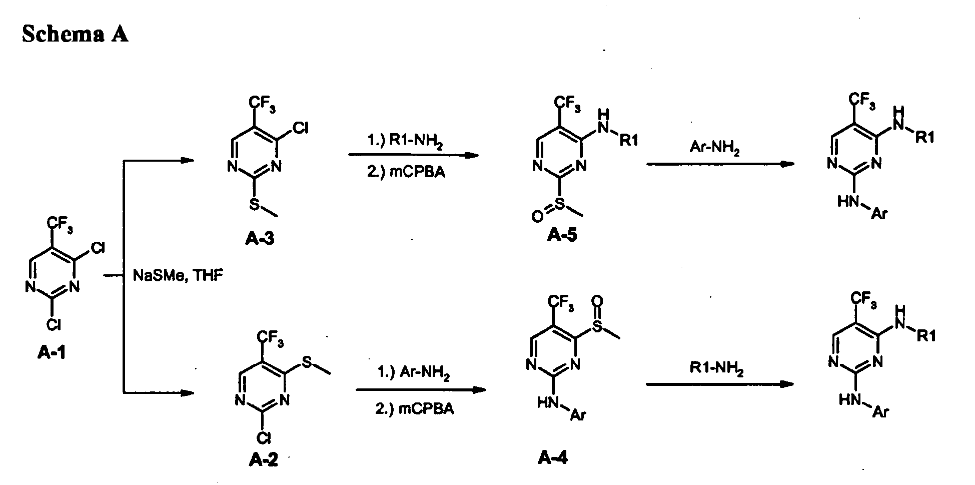

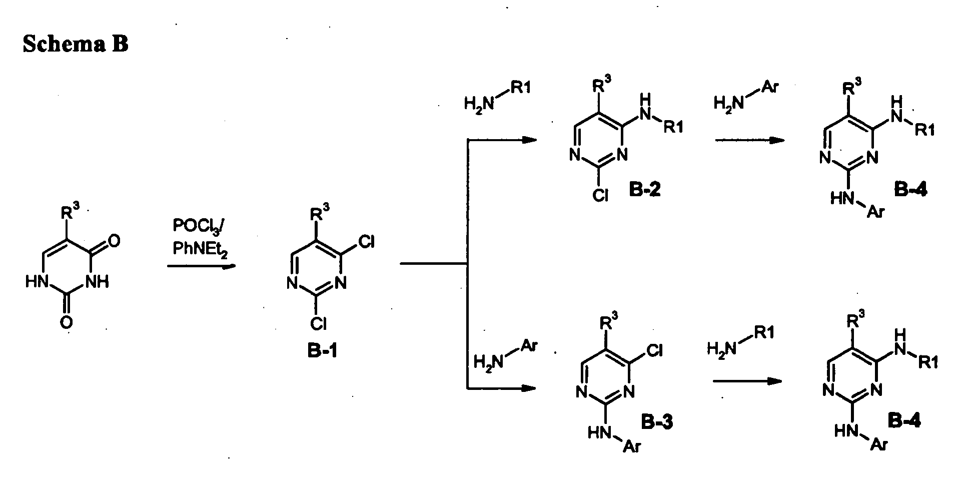

Optional ist nach dem Aufbau des Diaminopyrimidins noch eine Transformation einer oder mehrerer funktioneller Gruppen möglich.

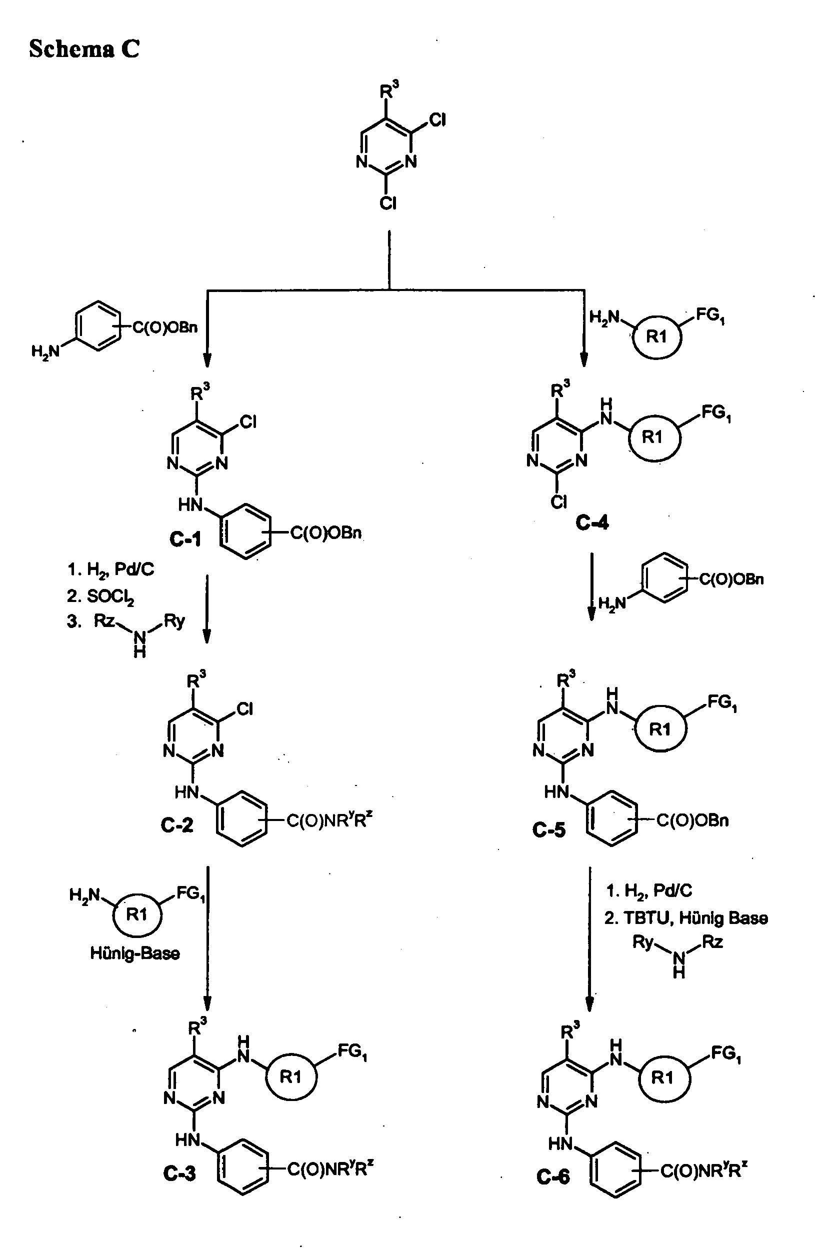

Optional ist nach dem Aufbau des Diaminopyrimidins noch eine Transformation einer oder mehrerer funktioneller Gruppen (FG) möglich. Dies ist in den Beispielen beschrieben, sofern relevant.

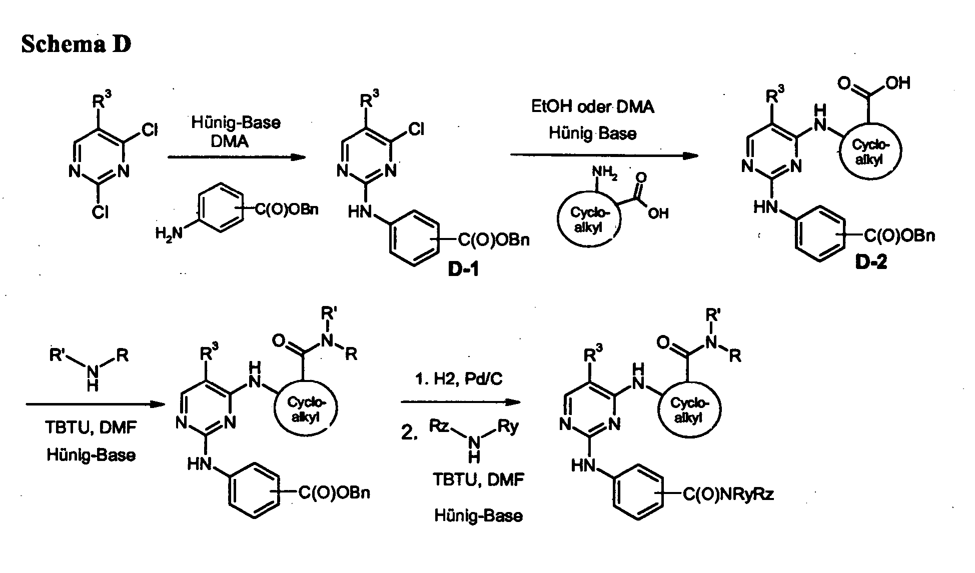

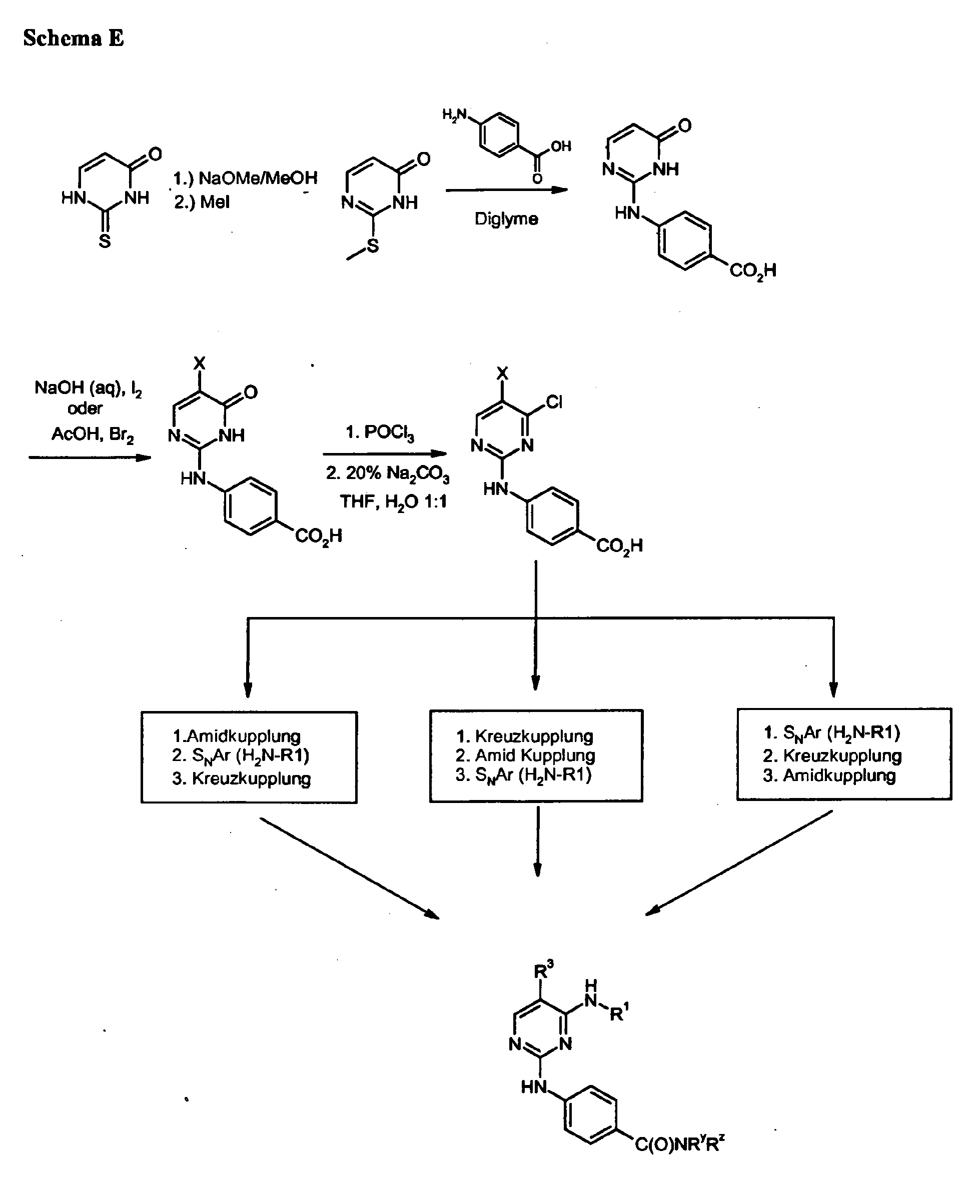

### Herstellung_von Ausgangsverbindungen

Falls nicht anders beschrieben werden alle Ausgangsmaterialien bei kommerziellen Anbietern gekauft und direkt in den Synthesen eingesetzt In der Literatur beschriebene Substanzen werden nach den publizierten Syntheseverfahren hergestellt.

### A-1) 2,4-Dichlor-5-trifluormethyl-pyrimidin

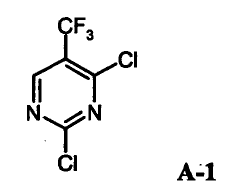

48 g (267 mmol) 5-Trifluormethyluracil werden unter Feuchtigkeitsausschluss in 210 mL Phosphoroxychlorid (POCl₃) suspendiert. Zu dieser Suspension werden 47.7 g (320 mmol, 1.2 eq) Diethylanilin so langsam zugetropft, dass die Temperatur zwischen 25 °C und 30 °C bleibt. Nach beendeter Zugabe wird noch 5-10 min im Wasserbad gerührt und der Ansatz für 5 - 6 h unter Feuchtigkeitsausschluss bei 80 - 90 °C erwärmt. Das überschüssige POCl₃ wird durch Einrühren in ca. 1200 g schwefelsaures Eiswasser zerstört und die wässrige Phase sofort 3 x mit jeweils 500 ml Ether oder t-Butyl-methyl-ether extrahiert. Die vereinigten etherischen Extrakte werden 2 x mit je 300 mL schwefelsaurem Eiswasser (ca. 0.1 M) sowie mit kalter Kochsalzlösung gewaschen und sofort über Natriumsulfat getrocknet. Man filtriert das Trocknungsmittel ab und entfernt das Lösungsmittel im Vakuum. Der Rückstand wird im Vakuum (10 mbar) über eine kurze Kolonne (20 cm) destilliert (Kopftemperatur: 65 - 70 °C), wobei man 35.3 g (0.163 mol, 61%) einer farblosen Flüssigkeit erhält, die unter Argon abgefüllt und gelagert wird.
DC: R_{f} = 0.83 (cHex:EE = 3:1)

### A-2) 2-Chlor-4-methylsulfanyl-5-trifluormethyl-pyrimidin und

### A-3) 4-Chlor-2-methylsulfanyl-5-trifluormethyl-pvrimidin

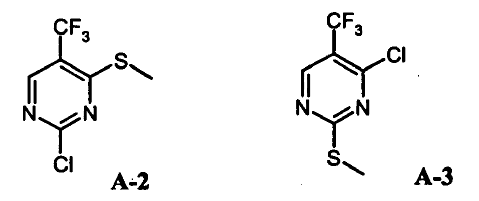

5 g (23 mmol) 2,4-Dichlor-5-trifluormethyl-pyrimidin werden in 40 mL THF gelöst, die Lösung auf -25 °C temperiert und 1.8 g (25.3 mmol, 1.1 eq) Natriumthiomethylat zugeben. Es wird 1 h bei -25 °C und dann ohne Kühlung über Nacht bei RT gerührt. Anschließend wird mit Dichlormethan verdünnt und 3 x mit 1 N HCl gewaschen. Die organische Phase wird über Magnesiumsulfat getrocknet und im Vakuum eingeengt. Das Rohprodukt wird säulenchromatographisch gereinigt (Kieselgel, Cyclohexan/Dichlormethan; von 90/10 auf 80/20% in ca. 20 min). Es werden 1.56 g (6.8 mmol, 30%) des Produktes **A-3** und 1.46 g (6.4 mmol, 28%) des Produktes **A-2** als farblose Öle isoliert. Daneben lassen sich 0.24 g (4%) des 2,4-Bis-methylsulfanyl-5-trifluormethyl-pyrimidins als farbloser Feststoff isolieren.

| | Produkt **A-3** | Produkt **A-2** |
|---|---|---|
| R_{f} (cHex:CH₂Cl₂ 1:1) | 0.48 | 0.40 |

Die Strukturaufklärung erfolgt mittels chemischer Derivatisierung und anschließender NMR Spektroskopie. Dazu werden **A-2** und **A-3** zunächst separat in THF bei 100 °C, 5 bar H₂, Pd/C und Pd(OH)₂ je 1:1 1 dehalogeniert. Aufgrund der unterschiedlichen Symmetrieeigenschaften der entstehenden Produkte ist eine eindeutige Identifizierung der Regioisomeren möglich.

### 4-Amino-N-methyl-N-phenyl-benzolsulfonamid (Edukt in Beispiel 1)

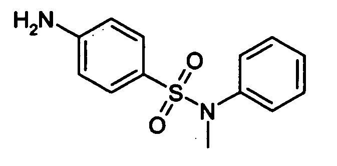

Es werden 9.5 ml (85.7 mmol, 98%) *N*-Methylanilin in 100 mL Dichlormethan gelöst und bei 0 °C 20 g (85.7 mmol, 95%) 4-Nitrobenzolsulfonylchlorid, gelöst in 150 mL Dichlormethan, zugetropft und noch 1.5 h weiter gerührt. Die organische Phase wird mit gesättigter, wässriger Natriumcarbonatlösung gewaschen und über Natriumsulfat getrocknet. Abschließend filtriert man über Kieselgel und erhält nach Entfernung aller flüchtigen Bestandteile im Vakuum 24.6 g des rohen *N*-Methyl-4-nitro-*N*-phenyl-benzolsulfonamids.
14.6 g (49.9 mmol) des Nitrosulfonsäureamids werden in 100 mL THF/MeOH 1/1 gelöst. Nach Zugabe von Pd/C (10%) wird unter 5 bar H₂-Druck 16 h bei 50 °C gerührt. Nach Zugabe von Molekularsieb zum Binden von Wasser, weiterer Zugabe von Pd/C und erneutem Rühren unter Hydrierbedingungen (5 bar H₂-Druck, 60 °C) für 16 h erhält man 13.1 g (48.9 mmol, 100%) rohes **A-4a** als beigen Feststoff. Dieses Rohprodukt wird ohne weitere Reinigung in der Synthese eingesetzt.

In analoger Weise werden 4-Amino-*N*-phenyl-benzolsulfonamid und 4-Amino-*N,N-*dimethyl-benzolsulfonamid hergestellt (Edukte in Beispiel 2 und 3). Die beschriebene Methode stellt ein generell anwendbares Verfahren zur Herstellung von substituierten oder unsubstituierten Aminobenzolsulfonsäureamiden aus den entsprechenden Nitrobenzolsulfonsäurechloriden dar.

### Allgemeine Vorschrift zur Synthese von Verbindungen des Typs B-2

Ein entsprechend R3-substituiertes 2,4-Dichlorpyrimidin **B-1** (käuflich bzw. hergestellt durch Chlorierung des entsprechenden Uracils wie für **A-1** beispielhaft beschrieben) wird in THF (oder Dioxan, DMA, NMP, Aceton) gelöst (ca. 2 - 5 mL pro mmol), 1-1.6 eq Hünig-Base (oder Triethylamin, Kaliumcarbonat oder eine andere geeignete Base) zugegeben und die Reaktionsmischung temperiert (-78 °C bei sehr reaktiven Pyrimidinen, RT oder erhöhte Temperatur bei eher reaktionsarmen Pyrimidinen). Nun werden ca. 0.75 - 1 eq des Amins, gelöst in dem entsprechenden Lösungsmittel (siehe oben), zugegeben und die Reaktionsmischung über einen bestimmten Zeitraum bei der entsprechenden Temperatur gerührt bzw. über einen bestimmten Zeitraum aufgetaut bzw. erwärmt, abhängig von der Reaktivität des verwendeten Pyrimidins. Nach beendeter Reaktion (Reaktionskontrolle mittels HPLC oder DC) wird die Reaktionsmischung mit Kieselgel versetzt und alle flüchtigen Bestandteile im Vakuum entfernt. Säulenchromatographische Reinigung liefert die gewünschten Substitutionsprodukte. In Abhängigkeit vom Rest R3 des Pyrimidins entstehen beide möglichen Regioisomeren in verschiedenen Verhältnissen. Sie sind in der Regel chromatographisch separierbar.

### B-2a) (±)-(1S*,2R*)-2-(2-Chlor-5-trifluormethyl-pyrimidin-4-ylamino)-cyclopentancarbonsäureamid

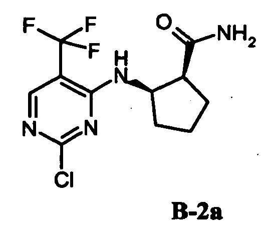

500 mg (2.3 mmol) **A-1** und 636 mg (4.6 mmol, 2 eq) Kaliumcarbonat werden in 11 mL Aceton suspendiert, auf -70 C° gekühlt, danach wird cis-(±)-(1S,2R)-2-Aminocyclopentancarbonsäureamid zugegeben. Man lässt die Reaktionsmischung über Nacht unter Rühren auf RT auftauen und rührt noch 24 Stunden bei Raumtemperatur. Dann wird mit 40 mL Kieselgel versetzt und alle flüchtigen Bestandteile im Vakuum entfernt. Säulenchromato-graphisch werden beiden regioisomeren Produkte getrennt, wobei das gewünschte Regioisomer das zuerst eluierende Produkt ist (Kieselgel, cHex/EE 40/60). Es werden 218 mg (0.71 mmol, 31%) **B-2a** isoliert sowie 297 mg (0.96 mmol, 42%) des regioisomeren Produktes **B-2'a.**
R_{f} **(B-2a)** = 0.51 (Kieselgel, EE ), [R_{f} (**B-2a**') = 0.34]
MS-ESI+: 309 (M+H)⁺

Die Aufklärung und Zuordnung der Struktur beider Regioisomere erfolgt mittels separater Dehalogenierung unter reduktiven Bedingungen und anschließender 1H-NMR-Spektroskopie der Produkte (analog zu **A-2** und **A-3**).

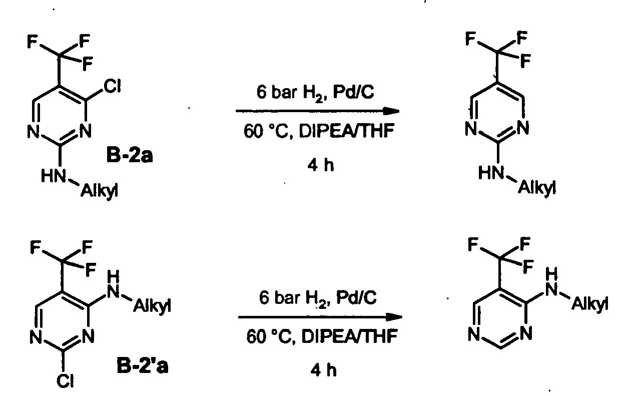

Folgende Beispiele von Verbindungen des Typs **B-2** werden analog synthetisiert.

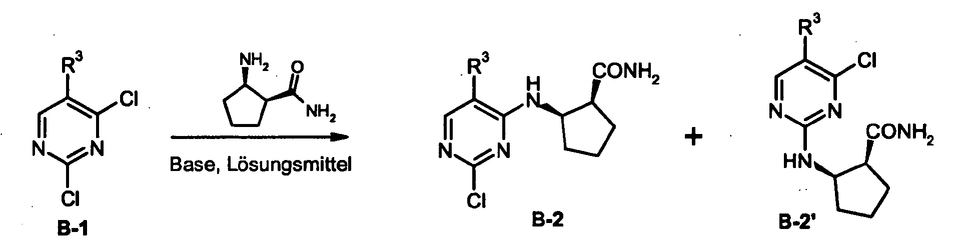

| **#** | **R³** | **Bedingungen** | **B-2 : B-2'** | **Ausbeute B-2** | **R_{f} (B-2)** | **R_{f} (B-2')** | **Laufmittel** |
|---|---|---|---|---|---|---|---|
| B-2a | CF₃ | Aceton, K₂CO₃, -70 °C - RT, 16 h | 42 : 58 | 31% | 0.51 | 0.34 | EE |
| B-2b | Me | DMA, Hünig-Base, 40°C, 24 h | > 85 : 15 | 83% | 0.25 | nicht bestimmt | EE |
| B-2c | NO₂ | Aceton, K₂CO₃ -70 °C, 16 h | > 99 : 1 | 82% | 0.54 | - | EE |
| B-2d | F | Dichlormethan, Hünig-Base, 0°C - RT, 2 Tage | > 99 : 1 | 82% | 0.43 | - | EE |
| B-2e | Cl | Dichlormethan, Hünig-Base, 0°C - RT, 1 Tag | nicht bestimmt | 60% | 0.45 | nicht bestimmt | EE |
| B-2f | *i*-Pr | DMA, Hünig-Base, 70°C, 24 h | nicht bestimmt | 60% | 0.40 | 0.28 | EE |

Die Verbindungen **B-2a** bis **B-2f** können säurekatalysiert mit Anilinen zu Verbindungen vom Typ **B-4** umgesetzt werden.

### Allgemeine Vorschrift zur Synthese von Verbindungen vom Typ B-4

Das Edukt **B-2** wird in 1-Butanol (oder Dioxan, DMA, NMP) gelöst (ca 0.5 - 4 mL pro mmol), 0.1-1 eq HCl in Dioxan zugegeben sowie 1 eq des Anilins und die Reaktionsmischung unter Rückfluss erwärmt. Nach beendeter Reaktion wird die Reaktionsmischung mit Kieselgel versetzt und alle flüchtigen Bestandteile im Vakuum entfernt. Anschließend wird säulenchromatographisch gereinigt. Oftmals fallen die Produkte auch nach beendeter Reaktion aus der Reaktionslösung aus und können direkt abgesaugt und mit 1-Butanol gewaschen werden.

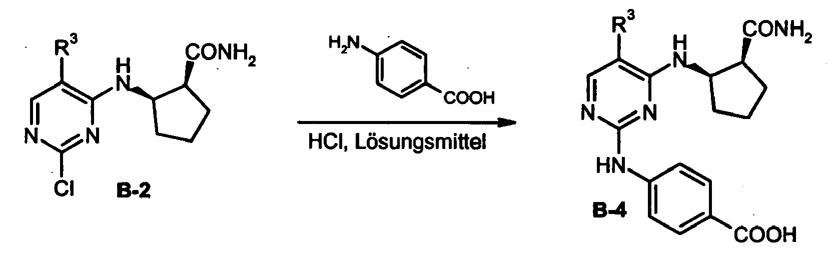

| **#** | **R³** | **Bedingungen** | **Ausbeute B-4** | **R_{f}** | **Laufmittel** |
|---|---|---|---|---|---|
| B-4a | CF₃ | Wird gem. Schema C aus **C-1** hergestellt (**C-3a≡B-4a**) | -- | 0.37 | DCM:MeOH:AcOH 9:1:0.1 |
| B-4b | Me | 1-Butanol, 0.1 eq HCl, Rückfluß 3 Stunden | 95% | 0.11 | DCM:MeOH:AcOH 9:1:0.1 |
| B-4c | NO₂ | 1-Butanol, 0.1 eq HCl, Rückfluß 4 Stunden | 66% | nicht bestimt | -- |
| B-4d | F | 1-Butanol, 0.1 eq HCl, Rückfluß 4 Stunden | 83% | 0.27 | DCM:MeOH:AcOH 9:1:0.1 |
| B-4e | Cl | 1-Butanol, 0.1 eq HCl, Rückfluß 2 Stunden | 92% | 0.31 | DCM:MeOH:AcOH 9:1:0.1 |
| B-4f | *i*-Pr | 1-Butanol, 0.1 eq HCl, Rückfluß4 Stunden | 99% | 0.08 | DCM:MeOH:AcOH 9:1:0.1 |

### (4-Amino-2-chlor-phenyl)-(4-methyl-piperazin-1-yl)-methanon (Edukt in Beispiel 70)

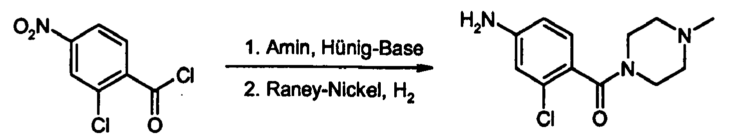

1 ml (8.84 mmol, 1.3 eq) *N*-Methylpipemzin wird in 40 mL Dichlormethan gelöst und diese Lösung mit 1.5 mL (8.84 mmol, 1.3 eq) Hünig-Base versetzt. Nun werden 1 - 5 g (6.82 mol, 1 eq) 4-Nitro-2-chlorbenzoylchlorid, gelöst in 10 mL Dichlormethan, langsam unter Kühlung zugetropft. Nach 2 h unter Rühren werden langsam 9 mL gesättigte, wässrige Natriumhydrogencarbonat-Lösung zugetropft, die organische Phase abgetrennt und das Lösungsmittel im Vakuum entfernt. Das Produkt wird säulenchromatographisch gereinigt (Kieselgel, DCM/MeOH/NH₃ 9/1/01) und man erhält 1.83 g (6.45 mmol, 95%) des Nitrobenzoesäureamids. Letzteres wird in 21 THF gelöst, 300 mg Raney-Nickel zugegeben und 16 h bei 3 bar H₂-Druck und RT gerührt. Man erhält nach Abfiltrieren des Raney-Nickels und Entfernen der flüchtigen Bestandteile im Vakuum 1.2 g (4.73 mmol, 73%) (4-Amino-2-chlor-phenyl)-(4-methyl-piperazin-1-yl)-methanon.
R_{f}= 0.38 (Kieselgel, DCM:MeOH:NH₃ = 9:1:0.1)
MS-ESI⁺: 254 (M+H)⁺

Die Methode eignet sich in analoger Weise zur Synthese substituierter und unsubstituierter Aminobenzoesäureamide, wie sie zum Beispiel in der Synthese der Beispiele 71 - 75 verwendet werden. Diese Beispiele werden analog zu Beispiel 70 hergestellt. Bei der Synthese der Beispiele 106, 107 und 144 werden m-Aminobenzoesäureamide verwendet, die nach dem gleichen Verfahren hergestellt werden.

### cis-(±)-2-Amino-cyclopentancarbonsäure-isopropylamid

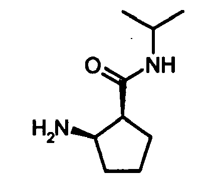

55 mg (0.43 mmol) cis-(±)-2-Amino-cyclopentancarbonsäure werden in 900 µL (25 eq) Isopropylamin suspendiert und zu dieser Suspension 205 mg (0.064 mmol, 1.5 eq) TBTU und 550 µL DMF zugegeben. Es wird 16 h gerührt und die Reaktionsmischung wird in DCM:MeOH:NH₃ 9:1:0.1 aufgenommen und mit 7 mL Kieselgel versetzt. Nach Entfernung aller flüchtigen Bestandteile im Vakuum wird chromatographiert (Kieselgel DCM:MeOH:NH₃ 9:1:0.1). Man erhält 63 mg (0.37 mmol, 86%) farblosen Feststoff. R_{f}= 0.33 (Kieselgel, DCM:MeOH:NH₃ 85:15:1.5)

### B-2g) (±)-(1S*,2R*)-2-(2-Chlor-5-trifluormethyl-pyrimidin-4-ylamino)-cyclopentancarbonsäureisopropylamid

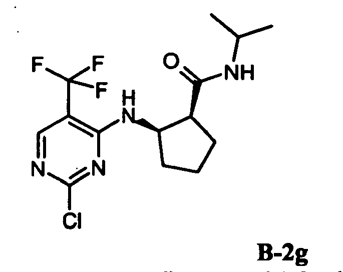

2 g (9.2 mmol) **A-1** und 1.8 ml (11.2 mmol, 1.2 eq) Hünig-Base werden in 60 mL THF gelöst, auf -78 C° gekühlt, danach wird cis-(±)-2-Amino-cyclopentancarbonsäureisopropylamid, gelöst in 60 mL THF, bei -78°C langsam zugetropft. Man lässt die Reaktionsmischung über Nacht unter Rühren auf RT auftauen. Dann wird mit 40 mL Kieselgel versetzt und alle flüchtigen Bestandteile im Vakuum entfernt. Säulenchromatographisch werden beiden regioisomeren Produkte getrennt, wobei das gewünschte Regioisomer das zuerst eluierende Produkt ist (Kieselgel, cHex/EE von 85/15 auf 80/20 innerhalb von 30 min). Es werden 590 mg (1.68 mmol, 24%) **B-2g** isoliert sowie 690 mg (1.97 mmol, 28%) des regioisomeren Produktes **B-2g'.**
R_{f} **(B-2g)** = 0.21 (Kieselgel, cHex:EE 3:1), [R_{f} **(B-2g')** = 0.10]
MS-ESI+: 351 (M+H)⁺

### UVₘₐₓ = 246 nm

### 3-Fluor-4-(4-methyl-[1,4]diazepan-1-yl)-phenylamin

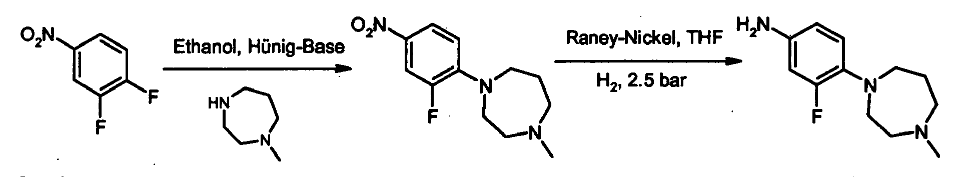

2 g (12.6 mmol) 3,4-Difluornitrobenzol werden in 1.6 ml Ethanol gelöst, 2.4 mL (15.1 1 mmol, 1.2 eq) Hünig-Base zugegeben und dann unter Eiskühlung 1.44 g (12.6 mmol, 1 eq) Hexahydro-1-methyl-1*H*-1,4-diazepin zugetropft. Nach ca. 12 h Rühren bei RT ist die Reaktion beendet. Danach wird mit Methanol und 50 mL Kieselgel versetzt, die flüchtigen
Bestandteile im Vakuum entfernt und säulenchromatographisch gereinigt (DCM/MeOH 97/3 auf 85/15 in 35 min). Man erhält 3 g (11.9 mmol, 94%) der Nitroverbindung.
R_{f}= 0.39 (Kieselgel, DCM:MeOH:NH₃ 9:1:0.1)
MS-ESI⁺: 253 (M+H)⁺
Die Nitroverbindung wird in 600 mL THF gelöst und mit ca. 300 mg Raney-Nickel versetzt. Es wird 3 h bei einem H₂-Druck von 3 bar hydriert. Das Raney-Nickel wird abfiltriert und die Lösung im Vakuum von allen flüchtigen Bestanteilen befreit. Man erhält 2.15 g (9.6 mmol, 81%) 3-Fluor-4-(4-methyl-[1,4]diazepan-1-yl)-phenylamin.
R_{f}= 0.48 (Kieselgel, DCM:MeOH:NH₃ 4:1:0.1)
MS-ESI⁺: 224 (M+H)⁺

In analoger Weise werden die Aniline hergestellt, die als Edukte in den Beispielen 142 - 143 eingesetzt werden.

### 4-Amino-benzoesäure-benzylester

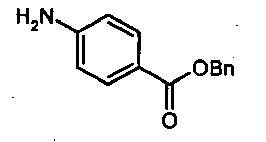

10.01 g 4-Nitrobenzoesäure werden in 500 mL Acetonitril suspendiert und nachfolgend mit 15.03 g (108.7 mmol, 1.2 eq) Kaliumcarbonat versetzt. Unter Rühren werden 15.40 g (171.0 mmol, 1 eq) Benzylbromid zugetropft und die Reaktionsmischung dann für 5 h unter Rühren auf 60 °C erwärmt. Es wird mit 750 ml destilliertem Wasser versetzt, 4 x mit je 250 mL EE extrahiert und nach Vereinigen der organischen Phasen über Natriumsulfat getrocknet. Nach Entfernen aller flüchtigen Bestandteile im Vakuum wird das Rohprodukt nacheinander 2 x in Toluol suspendiert und im Vakuum alle flüchtigen Bestandteile entfernt (Entfernung überschüssigen Benzylbromid). Man erhält 20.60 g (80.1 mmol) 4-Nitro-benzoesäurebenzylester als farblosen Feststoff, welcher in der nächsten Stufe ohne weitere Reinigung eingesetzt wird.
20.6 g des 4-Nitrobenzoesäurebenzylesters werden in 350 mL Dioxan gelöst und diese Lösung mit 6.9 g (49.9 mmol, 0.61 eq) Raney-Nickel versetzt. Unter Rühren wird bei 5 bar H₂-Druck 16 h hydriert. Der Katalysator wird abfiltriert, im Vakuum alle flüchtigen Bestandteile entfernt. Man erhält 17.0 g (74.8 mmol, 93%) 4-Aminobenzoesäurebenzylester in Form eines farblosen Feststoffs.

### C-1a) 4-(4-Chlor-5-trifluormethyl-pyrimidin-2-ylamino)-benzoesäure-benzylester

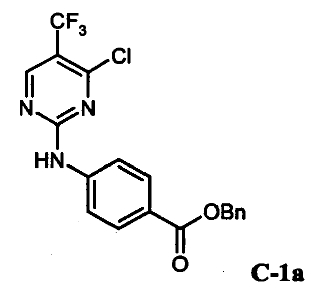

10 g (44 mmol) 4-Aminobenzoesäurebenzylester werden in 200 mL DMA gelöst, 8 mL Hünig-Base (0.97 eq) zugegeben und zur klaren Lösung 10.4 g (48.21 mmol) 2,4-Dichlor-5-trifluormethylpyrimidin, gelöst in 50 mL DMA, bei RT zugetropft. Die Reaktionslösung wird bei 60 °C über Nacht gerührt, dann mit 300 mL Dichlormethan versetzt und mit destilliertem Wasser (3 x 300 mL) ausgeschüttelt. Die organische Phase wird über Natriumsulfat getrocknet und das Lösungsmittel im Vakuum entfernt. Das Rohprodukt wird mit 100 mL MeOH versetzt, digeriert und 2 h stehen gelassen. Anschließend wird 10 min gerührt, der Niederschlag abfiltriert und mit Methanol nachgewaschen (methanolisches Filtrat enthält das nicht gewünschte Regioisomer der nukleophilen Substitution). Abschließend wird das Rohprodukt nochmals in Methanol suspendiert, abfiltriert, mit wenig Methanol nachgewaschen und im Vakuumtrockenschrank bei 60 °C getrocknet. Man erhält 8.5 g (20.7 mmol, 43%) von **C-1a** in Form eines hellgelben Feststoffs.
R_{f}= 0.71 (Kieselgel, cHex:EE 1:2)
MS-ESI⁺: 408 (M+H)⁺

### C-2a) [4-(4-Chlor-5-trifluormethyl-pyrimidin-2-ylamino)-phenyl]-(4-methyl-piperazin-1-yl)-methanon

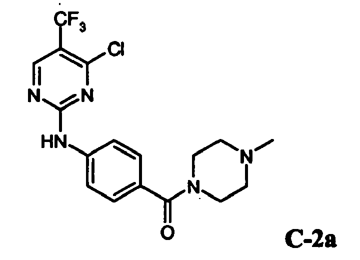

2.74 g (6.71 mmol) **C-1a** werden in 120 mL Dioxan gelöst, 300 mg Palladiumhydroxid (20% w/w Pd, 2.14 mmol, 0.32 eq) zugegeben und 16 h bei 3 bar H₂-Druck und RT gerührt. Die Reaktionsmischung wird über Celite filtriert, im Vakuum das Lösungsmittel entfernt und 1.87 g (5.89 mmol, 88%) 4-(4-Chlor-5-trifluormethyl-pyrimidin-2-ylamino)-benzoesäure als farbloser Feststoff erhalten, der ohne weitere Reinigung eingesetzt wird. 1.1 g (3.46 mmol) der Benzoesäure werden mit 20 mL Toluol und 301 µL (4.16 mmol, 1.2 eq) Thionylchlorid versetzt und 1.5 h unter Rückfluss erhitzt. Alle flüchtigen Bestandteile werden im Vakuum entfernt und das rohe Benzoesäurechlorid direkt weiter umgesetzt. 536 mg (1.6 mmol) davon werden in 4 mL THF gelöst und mit 410 µL (1.5 eq) Hünig-Base versetzt. Nach Zugabe von 179 µL (1 eq) *N*-Methylpiperazin wird die Lösung 16 h bei RT gerührt. Das Reaktionsgemisch wird in ca. 40 mL destilliertes Wasser gegossen, 30 min gerührt und die wässrige Phase 3 x mit je 50 ml Ethylacetat extrahiert. Nach Trocknung der organischen Phase über Magnesiumsulfat, Filtration und Entfernung der flüchtigen Bestandteile im Vakuum werden 645 mg (1.5 mmol, 94%) **C-2a** als Feststoff erhalten.
R_{f}= 0.69 (Kieselgel, CH₂Cl₂:MeOH:NH₃ 5:1:0.1)
MS-ESI+: 400 (M+H)⁺

### C-2b) 4-(4-Chlor-5-trifluormethyl-pyrimidin-2-ylamino)-N-methyl-N-(1-methyl-piperidin-4-yl)-benzamid

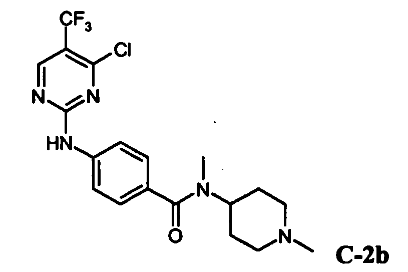

R_{f}= 0.30 (Kieselgel, CH₂Cl₂:MeOH:NH₃ 5:1:0.1)
MS-ESI+: 428 (M+H)⁺

**C-2b** wird analog zu **C-2a** unter Verwendung von Methyl-(1-methyl-piperidin-4-yl)-amin hergestellt.

### (±)-((1S*,2R*)-2-Amino-cyclohexyl)-carbaminsäurebenzylester

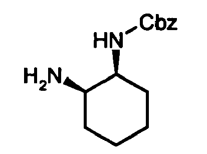

2 mL (16.2 mmol) cis-1,2-Diaminocyclohexan und 2.42 g (19.4 mmol, 1.2 eq) 9-Borabicyclo[3.3.1]nonan (9-BBN) werden in 8 mL THF/NMP 1/1 gelöst und bei RT 45 min lang gerührt. Zu der leicht trüben Lösung werden 2.4 mL (16.2 mmol, 1 eq) Benzylchlorformiat (Cbz-Chlorid) gegeben. Nach ca. 1 h wird das Reaktionsgemisch mit destilliertem Wasser versetzt und einige Minuten lang gerührt. Anschließend wird die wässrige Lösung mit Ethylacetat versetzt und die wässrige Phase 3 x mit je ca. 50 mL Ethylacetat gewaschen. Das Produkt befindet sich zur Gänze in der wässrigen Phase Verunreinigungen in der organischen Phase. Die wässrige Phase wird mit NaHCO₃ alkalisch gestellt (pH 8), mit Dichlormethan versetzt, 3 x mit je 10 mL Dichlormethan extrahiert, die vereinigten organischen Phasen über Magnesiumsulfat getrocknet und das Lösungsmittel im Vakuum entfernt. Man erhält 2.29 g (9.22 mmol, 57%) (±)-((1S*,2R*)-2-Amino-cyclohexyl)-carbaminsäurebenzylester als farblose ölige Flüssigkeit.
R_{f}= 0.45 (Kieselgel, CH₂Cl₂:MeOH:NH₃ 9:1:0.1)
MS-ESI⁺: 249 (M+H)⁺

### C-3a) (±)-(1S*,2R*)-{2-[4-(4-Methyl-piperazin-1-carbonyl)-phenylamino]-5-trifluormethyl-pyrimidin-4-ylamino}-cyclohexyl)-carbaminsäurebenzylester

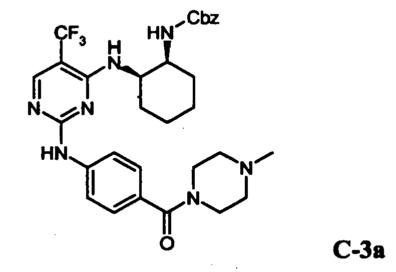

800 mg (2 mmol) **C-2a** werden mit in 1 mL NMP gelöst, 569 mg (2.4 mmol, 1.2 eq) (±)-((1S*,2R*)-2-Amino-cyclohexyl)-carbaminsäurebenzylester und anschließend 521 µL (3 mmol, 1.5 eq) Hünig-Base zugegeben. Nach 48 h bei 70 °C ist die Reaktion abgeschlossen. Nach Entfernen des Lösungsmittels im Vakuum wird das Rohprodukt säulenchromatographisch gereinigt (DCM/MeOH/NH₃ von 19/1/0.1 auf 9/1/0.1) und 826 mg (1.35 mmol, 68%) des Produktes in Form eines farblosen Harzes erhalten.
MS-ESI⁺: 612 (M+H)⁺

### C-3b) (±)-{4-[4-((1R*,2S*)-2-Amino-cyclohexylamino)-5-trifluormethyl-pyrimidin-2-ylamino]-phenyl}-(4-methyl-piperazin-1-yl)-methanon

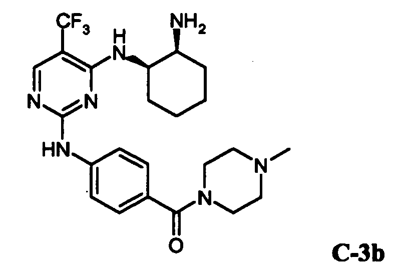

112 mg (0.18 mmol) **C-3a** wird in DMF (10 mL) gelöst und mit destilliertem Wasser (1 mL) versetzt. Nun werden abermals 9 mL DMF zugegeben, die Lösung in eine Hydrierapparatur überführt und mit Pd/C (200 mg, 5% Pd) versetzt. Die Reaktionslösung wird 12 h bei einem H₂-Druck von 4 bar gerührt. Das Reaktionsgemisch wird in Dichlormethan aufgenommen und mit 10 mL RP-Gel versetzt und alle flüchtigen Bestandteile im Vakuum entfernt. Die Reinigung erfolgt über Säulenchromatographie (RP-Phase, Acetonitril/Wasser von 5/95 auf 95/5 in 20 min). Nach Vereinigung der Produktfraktionen und Gefriertrocknung erhält man 27 mg (0.06 mmol, 30%) des gewünschten Produkts als farblosen Feststoff.
MS-ESI⁺: 478 (M+H)⁺

### C-3c) (±)-(1S*,2R*)-2-{2-[4-(4-Methyl-piperazin-1-carbonyl)-phenylamino]-5-trifluormethyl-pyrimidin-4-ylamino}-cycloheptancarbonsäure

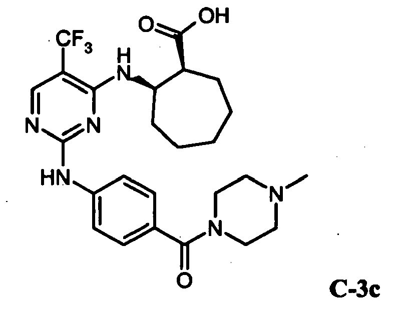

440 mg (1.1 mmol) **C-2a** werden in 500 µL NMP gelöst und mit 565 µL Hünig-Base (3.3 mmol, 3 eq) sowie 256 mg cis-2-Aminocycloheptancarbonsäure (racemisch) versetzt. Die Reaktionsmischung wird in ein auf 100°C temperiertes Ölbad gegeben und bei dieser Temperatur für 8 h unter Rühren erwärmt. Das Reaktionsgemisch wird nach Abschluss der Reaktion in Methanol aufgenommen, mit 20 mL RP-Gel versetzt und alle flüchtigen Bestandteile im Vakuum entfernt. Die Reinigung erfolgt über Umkehrphase (Laufmittel: Acetonitril/Wasser (15/85 auf 35/65 in 15 min). Nach Vereinigung der Produktfraktionen und Gefriertrocknung erhält man 160 mg (0.31 mmol, 28%) des gewünschten Produkts als farblosen Feststoff.
MS-ESI⁺: 521 (M+H)⁺

### C-3d) (±)-(1S*,2R*)-2-{2-[4-(4-Methyl-piperazin-1-carbonyl)-phenylamino]-5-trifluormethyl-pyrimidin-4-ylamino} -cyclopentancarbonsäure

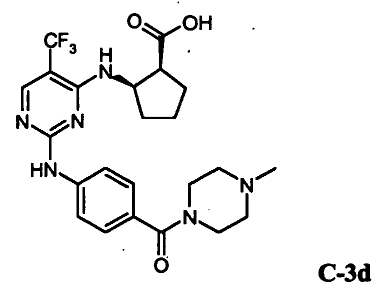

563 mg (1.13 mmol) **C-2a** werden in 5 mL 1-Butanol gelöst und dazu 163 mg cis-2-Amino-1-cyclopentancarbonsäure (racemisch) gegeben. Nach Zugabe von 540 µL Hünig-Base wird ca. 60 min bei 110 °C erwärmt (Mikrowelle, CEM, 100 W). Das Reaktionsgemisch wird im Vakuum eingeengt, mit ca. 100 mL Wasser verrührt und 3 x mit je 50 mL Ethylacetat ausgeschüttelt. Die vereinigten organischen Phasen werden über Magnesiumsulfat getrocknet und das Lösungsmittel im Vakuum entfernt. Man erhält 530 mg (1.08 mmol, 96%) **C-3d.**
MS-ESI⁺: 493 (M+H)⁺

Die Herstellung von C-3e erfolgt analog unter Verwendung von DMA als Lösungsmittel und **C-2b** als Ausgangsmaterial. 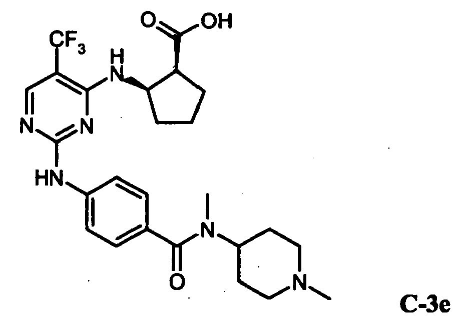 MS-ESI⁺: 521 (M+H)⁺

### C-3f) (1S,3R)-3-(2-{4-[Methyl-(1-methyl-piperidin-4-yl)-carbamoyl]-phenylamino}-5-trifluormethyl-pyrimidin-4-ylamino)-cyclopentancarbonsäure

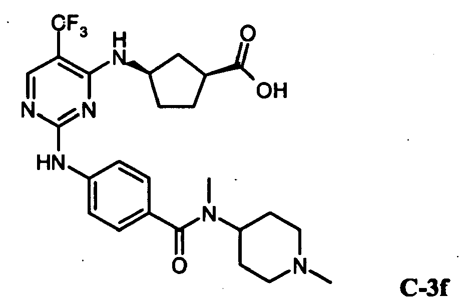

200 mg **C-2b** werden in 750 µL DMA gelöst und 160 µL (0.93 mmol, 2 eq) Hünig-Base zugegeben. Nun werden 72 mg (0.56 mmol, 1.2 eq) (1S,3R)-3-Aminocyclopentancarbonsäure zugegeben und die Reaktionsmischung 40 min lang auf 120 °C erwärmt. Die Reaktionsmischung wird mit RP-Gel versetzt, die flüchtigen Bestandteile im Vakuum entfernt und das Produkt säulenchromatographisch über eine RP-Phase gereinigt und isoliert (von 85% Wasser (+0.2% HCOOH) und 15% Acetonitril (+0,2% HCOOH) auf 76% Wasser und 24% Acetonitril in 20 min). Entsprechende Produktfraktionen werden vereinigt, mittels Gefriertrocknung vom Lösungsmittel befreit und 150 mg (0.29 mmol, 62%) C-3f als farblosen Film erhalten.

### (±)-trans-2-Aminocyclopentancarbonsäureamid

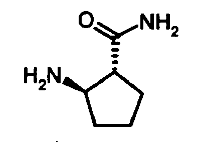

Die Verbindung wird gemäß Literatur (Csomos et al., 2002) hergestellt.

### D-2a) 4-[4-((1R,2S)-2-Carboxy-cyclopentylamino)-5-trifluormethyl-pyrimidin-2-ylamino]-benzoesäure-benzylester

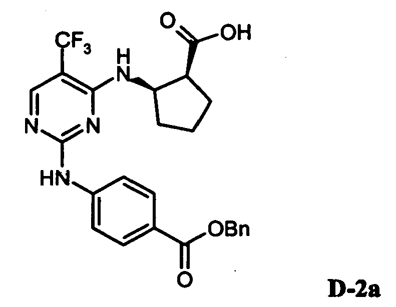

2.05 g (5 mmol) **C-1a** und 1 g (1S,2R)-(+)-2-Amino-1-cyclopentancarbonsäure Hydrochlorid (6 mmol, 1.2 eq) werden in 18 mL Ethanol vorgelegt. 7.3 ml (42.5 mmol, 3.4 eq) Hünig-Base zugegeben und 4 h bei 70 °C gerührt. Die Reaktionsmischung wird in 275 mL Wasser eingerührt, vom Ungelösten abfiltriert, das Filtrat mit gesättigter, wässriger KHSO₄-Lösung auf pH 2 gestellt, 5 min gerührt und die entstehende Ausfällung abgesaugt. Das Rohprodukt wird mit Wasser gewaschen, im Vakuum getrocknet und 2.37 g (4.74 mmol, 94%) **D-2a** in Form eines leicht beige gefärbten Feststoffs erhalten.
MS-ESI⁺: 501 (M+H)⁺

Die Synthese mit (1R,2S)-(-)-2-Amino-1-cyclopentancarbonsäure- bzw. (1R*,2S*)-(±)-2-Amino-1-cyclopentancarbonsäurederivat wird analog durchgeführt. Die entsprechenden Produkte tragen die Benennung **D-2b** (chiral, Enantiomer zu **D-2a**) und **D-2c** (rac).

### Garstellung von (1S,2R)-2-Aminocyclopentancarbonsäure Hydrochlorid

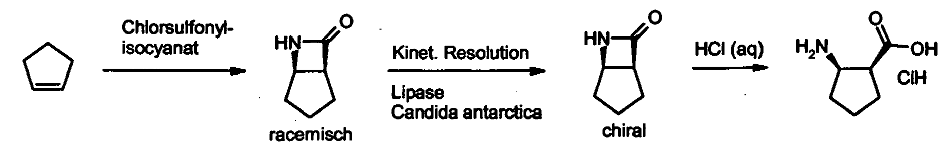

Zu 23 mL (0.273 mol, 1 eq) Cyclopenten werden bei -75 °C unter Argon 22.64 mL (0.26 mol, 0.95 eq) CSI tropfenweise zugegeben. Während der Zugabe wird die Reaktionstemperatur stets unter -65 °C gehalten. Man lässt die Reaktionsmischung binnen 2 h auf RT kommen und rührt über Nacht weiter. Die reduktive Aufarbeitung erfolgt durch Eintropfen der Reaktionslösung in eine Lösung aus 600 mL Eis/Wasser mit 60 g Natriumsulfit und 180 g NaHCO₃. Die wässrige Phase wird 4 x mit je 200 mL Dichlormethan extrahiert, die organischen Phasen vereinigt, über Magnesiumsulfat getrocknet und alle flüchtigen Bestandteile im Vakuum entfernt. Man erhält 25.75g (85%) leicht gelbliche Kristalle.
Diese werden in 400 mL Diisopropylether gelöst, 1.6 mL Wasser und 20 g harzgebundene Lipolase (Lipase acrylic resin from *candida antartica, Sigma-Aldrich*) zugegeben und 11 Tage bei 60 °C geschüttelt. Die Reaktionssuspension wird über Celite filtriert, mit Diisopropylether nachgewaschen und das Filtrat bis zur Trockene eingeengt. Das dabei erhaltene gelbliche Öl wird in 200 mL Dichlormethan aufgenommen und mit ca. 150 mL gesättigter NaHCO₃-Lösung gewaschen. Die wässrige Phase wird 3 x mit Dichlormethan rückgeschüttelt, die organischen Phasen vereinigt und über Magnesiumsulfat getrocknet. Nach Entfernen aller flüchtigen Bestandteile im Vakuum erhält man 8.93 g des chiralen Lactams in Form eines gelblichen Öls.
Man löst Produkt letzteres in 10 mL Wasser und gibt unter Eisbadkühlung und Rühren 10 mL 37% HCl (aq) zu. Nach 10 min Rühren bei 0 °C lässt man die Reaktionslösung über Nacht bei RT stehen. Die dabei ausfallenden Kristalle werden abfiltriert, mit wenig Acetonitril gewaschen und im Hochvakuum getrocknet. Die Mutterlauge wird bis fast zur Trockene eingeengt, die dabei ausfallenden Kristalle abfiltriert, mit Acetonitril gewaschen und ebenfalls im Hochvakuum getrocknet. Man erhält 11.74 g (70.9 mmol, 31% bezogen auf das recemische Lactam) farbloser Kristalle des Hydrochlorids der (1S,2R)-2-Aminocyclopentanearbonsäure.
(Die enantiomere Säure ist auf der Stufe der kinetischen Resolution ausgefallen und im Niederschlag enthalten, der mittels Filtration über Celite abgetrennt wurde).
Die Synthesesequenz ist in der Literatur (Forro and Fueloep, 2003) beschrieben.

### D-3a) 4-[4-((1R,2S)-2-lsopropylcarbamoyl-cyclopentylaminol)-5-trifluormeth-pyrimidin-2-ylamino]-benzoesäurebenzylester

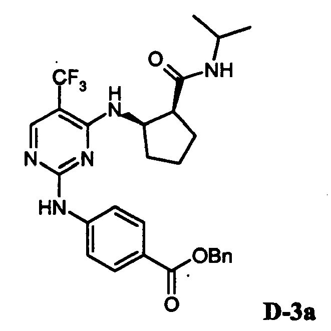

2.59 g (4.9 mmol) **D-2a,** 2.21 g (6.9 mmol, 1.4 eq) TBTU und 4.21 mL (24.6 mmol, 5 eq) Hünig-Base werden in 75 mL DMF gelöst und 20 min bei RT gerührt. Daraufhin werden 0.63 ml (7.38 mmol; 1.5 eq) Isopropylamin zugegeben und über Nacht bei RT gerührt. Es wird über basisches Aluminiumoxid abgesaugt, mit DMF nachgewaschen und die Mutterlauge in 400 mL Wasser eingerührt, weitere 30 min gerührt und die Ausfällung abgesaugt. Das Rohprodukt wird mit Wasser gewaschen und im Vakuum getrocknet. Zur Reinigung wird mit 50 ml Acetonitril 30 min bei 5 °C verrührt, abgesaugt, mit etwas kaltem Acetonitril nachgewaschen und der Rückstand im Vakuum getrocknet. Man erhält 2.13 g (3.9 mmol, 80%) **D-3a** in Form eines hellbeigen Feststoffs.
R_{f}= 0.53 (Kieselgel, cHx:EE 1:1)
MS-ESI⁺: 542 (M+H)⁺

### D-4a) 4-[4-((1R,2S)-2-Isopropylcarbamoyl-cyclopentylamino)-5-trifluormethyl-pyrimidin-2-ylaminol]-benzoesäure

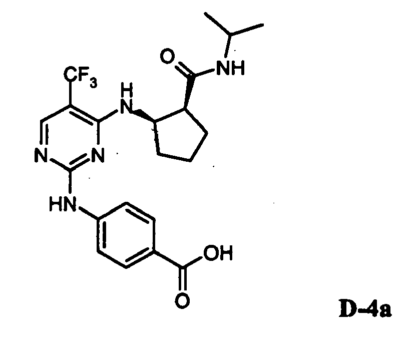

2.13 g (3.9 mmol) **D-3a** werden in 150 mL THF gelöst und 250 mg Palladiumhydroxid/C-Katalysator (20 Gew.% Pd auf Kohle) zugegeben. 16 h wird bei einem H₂-Druck von 6 bar unter Rühren bei RT hydriert. Danach werden 30 mL Methanol zugegeben, der Katalysator über Kieselgur abfiltriert, mit Methanol nachgewaschen und das Filtrat eingeengt. Der Rückstand wird mit 45 mL Ethanol aufgekocht, langsam auf 5 °C abgekühlt, 1 h weiter gerührt und dann abgesaugt und mit kaltem Ethanol nachgewaschen. Man erhält 2.46 g (3.2 mmol, 82%) der Säure **D-4a.**
R_{f}= 0.46 (Kieselgel, CH₂Cl₂:MeOH:AcOH 5:1:0.1)
MS-ESI⁺: 452 (M+H)⁺

Die Synthese der enantiomeren Verbindung sowie des Racemats erfolgt analog.

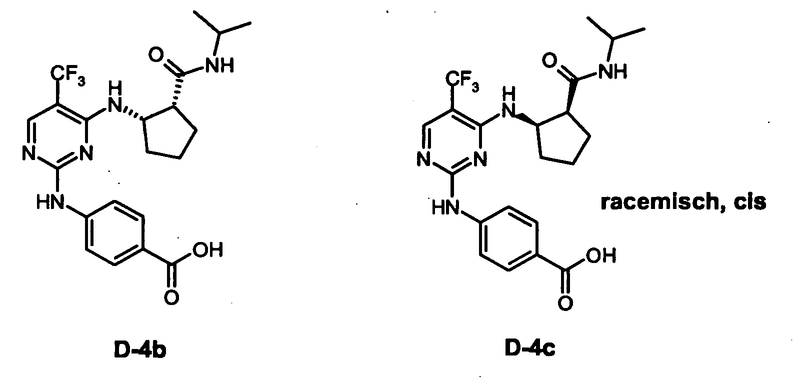

### D-5c) (±)-{4-[4-((1R*,2S*)-2-Isopropylcarbamoyl-cyclopentylamino)-5-trifluormethyl-pyrimidin-2-ylamino]-phenyl}-carbaminsäure-t-butylester

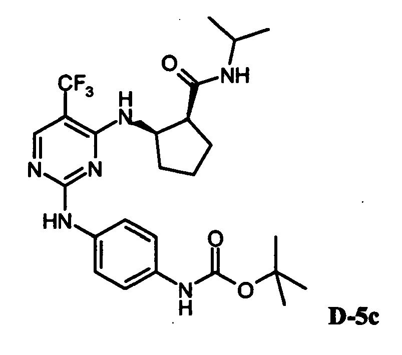

450 mg (1 mmol) **D-4c** werden in 1.8 mL trockenem Toluol gelöst und nacheinander 222 µL (1.3 mmol, 1.3 eq) Hünig-Base und 940 µL t-Butanol versetzt. Nun werden 258 µL Diphenylphosphorylazid zugegeben und 16 h auf 80 °C erwärmt. Die Reaktionsmischung wird mit 20 mL Ethylacetat versetzt, 2 x mit je 20 mL 0.5 M NaOH-Lösung gewaschen und die wässrige Phase 2 x mit je 20 ml Ethylacetat gegengewaschen. Die vereinigten organischen Phasen werden mit gesättigter, wässriger Natriumchloridlösung gewaschen, unlösliche Bestandteile abfiltriert, das Filtrat über Magnesiumchlorid getrocknet und das Lösungsmittel im Vakuum entfernt: Es werden 461 mg (0.88 mmol, 89%) **D-5c** in Form eines gelblichen Feststoffs erhalten.
MS-ESI⁺: 523 (M+H)⁺

### D-6c) (±)-(1S*,2R*)-2-[2-(4-Amino-phenylamino)-5-trifluormethyl-pyrimidin-4-ylamino]-cyclopentancarbonsäure-isopropylamid

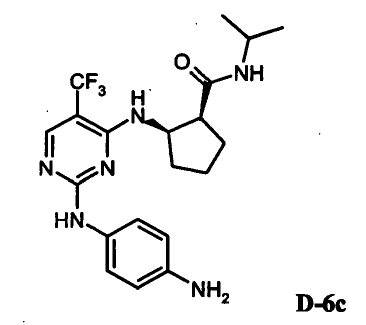

461 mg (0.88 mmol) **D-5c** werden in 5 mL Dichlormethan gelöst, 2 mL Trifluoressigsäure zugegeben und 1 h bei RT gerührt. Die Reaktionsmischung wird in 50 mL Wasser eingerührt und die wässrige Phase mit 50 mL Ethylacetat gewaschen. Die organische Phase wird noch 2 x mit 30 mL 10%iger Salzsäure extrahiert, die wässrigen Phasen vereinigt, mit 10%iger Natronlauge auf pH 10 gebracht und 3 x mit je 50 ml Ethylacetat extrahiert. Die vereinigten organischen Phasen werden über Magnesiumsulfat getrocknet, die flüchtigen Bestandteile im Vakuum entfernt und 243 mg (0.58 mmol, 65%) **D-6c** als farblosen Feststoff erhalten.
R_{f}= 0.08 (Kieselgel, cHex:EE 1:1)
MS-ESI⁺: 423 (M+H)⁺

### E-1) 2-Methylsulfanyl-1H-pyrimidin-4-on

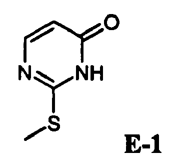

20 g (153 mmol) 2-Thiouracil werden in 250 mL Methanol suspendiert und anschließend 8.7 g (152.9 mmol, 1 eq) Natriummethanolat zugegeben. Die Lösung wird 5 min bei RT gerührt und danach 12.4 mL (198.8 mmol, 1.3 eq) Methyljodid zugetropft. Die Reaktionsmischung wird über Nacht gerührt, danach auf Wasser gegossen und 3 x mit je ca. 150 ml Chloroform extrahiert. Die vereinigten organischen Phasen werden über Magnesiumsulfat getrocknet, das Lösungsmittel im Vakuum entfernt und 16 g (121.5 mmol, 74%) **E-1** in Form eines farblosen Feststoffs erhalten.

### E-2) 4-(6-Oxo-1,6-dihydro-pyromidin-2-ylamino)-benzoesäure

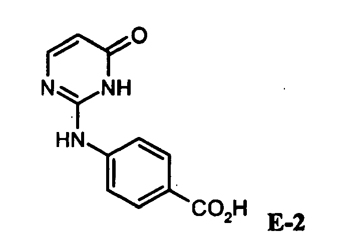

4.1 g (28.8 mmol) E-1 werden in 10 mL Diglyme (Diethylenglycoldimethylether) gelöst und diese Lösung mit 4.79 g (34.6 mmol, 1.2 eq) 4-Aminobenzoesäure versetzt. Die Reaktionsmischung wird für 16 h unter Rückfluss erhitzt. Nach Abkühlen auf RT wird der Niederschlag abgesaugt, mit wenig Diglyme, dann mit Diethylether nachgewaschen und im Vakuum getrocknet. Man erhält 5.27 g (22.8 mmol, 79%) **E-2** als farblosen Feststoff. MS-ESI⁺: 232 (M+H)⁺

### E-3a) 4-(5-Jod-6-oxo-1,6-dihydro-pyrimidin-2-ylamino)-benzoesäure

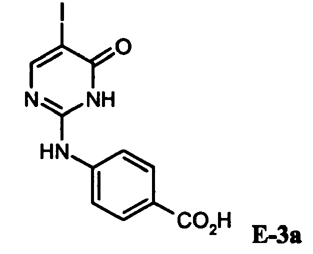

9 g (38.9 mmol) **E-2** werden in 100 mL Wasser vorgelegt, 2.18 g NaOH (54.5 mmol, 1.4 eq) zugegeben. Die Lösung wird mit 11.9 g (46.7 mol, 1.2 eq) Jod versetzt und 3 h bei 65 °C gerührt. Nach Abkühlen auf 50 °C wird Natriumthiosulfat Pentahydrat zugegeben um überschüssiges Jod zu entfernen, dann 1 h nachgerührt und dabei auf RT abgekühlt. Der bräunliche Niederschlag wird abgesaugt, mit Wasser gewaschen und im Vakuum getrocknet. Man erhält 13.7 g (38.4 mmol, 82%) **E-3a.**
MS-ESI⁺: 358 (M+H)⁺

### E-3b)4-(5-Brom-6-oxo-1,6-dihydro-pyrimidin-2-ylamino)-benzoesäure

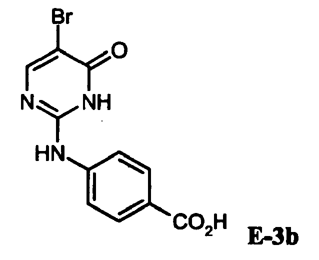

9 g (38.9 mmol) **E-2** werden in 10 mL Essigsäure vorgelegt und dazu eine Lösung von 2.1 mL (40.9 mmol 1.05 eq) Brom in 50 mL Essigsäure getropft und ca. 1 h bei RT gerührt. Die Reaktionsmischung wird in 800 mL Wasser eingerührt, die Ausfällung abgesaugt und der erhaltene bräunliche Niederschlag mit Wasser gewaschen und im Vakuum getrocknet. Man erhält 11.5 g (37.1 mmol, 95%) **E-3b** als farblosen Feststoff.
R_{f}= 0.27 (Kieselgel, EE:MeOH 7:3)
MS-ESI⁺: 309/311 (M+H)⁺ (1 x Br)

### E-4a) 4-(4-Chlor-5-iod-pyrimidin-2-ylamino)-benzoylchlorid und E-5a) 4-(4-Chlor-5-iod-pyrimidin-2-ylamino)-benzoesäure

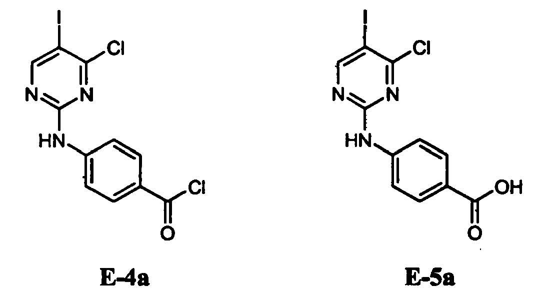

6.5 g (18.2 mmol) **E-3a** werden in 80 mL Phoshporoxychlorid suspendiert und 3 h unter Rühren unter Rückfluss erwärmt. Die Reaktionsmischung wird in 800 mL Wasser/Eis unter starkem Rühren eingetropft, weitere 30 min gerührt und das rohe Säurechlorid **E-4a** abfiltriert. Dieses wird im Vakuum getrocknet und ohne Reinigung weiter eingesetzt.
Zur Herstellung der Säure wird das rohe Säurechlorid in 200 mL THF gelöst und 200 mL 20%ige wässrige NaHCaO₃-Lösung zugegeben. Die Reaktionsmischung wird 16 h bei RT gerührt. THF wird im Vakuum entfernt, die wässrige Phase mit konzentrierter HCl auf pH 2 gestellt, 10 min nachgerührt, der entstehende Rückstand abgesaugt und mit Wasser nachgewaschen. Nach Trocknung im Vakuum erhält man 6.3 g (16.7 mmol, 92%) **E-5a** als farblosen Feststoff.
R_{f}= 0.24 (Kieselgel, Ethylacetat)
MS-ESI⁺: 427 (M+H)⁺

### E-4b) 4-(4-Chlor-5-brom-pyrimidin-2-ylamino)-benzoylchlorid und E-5b) 4-(4-Chlor-5-brom-pyrimidin-2-ylamino)-benzoesäure

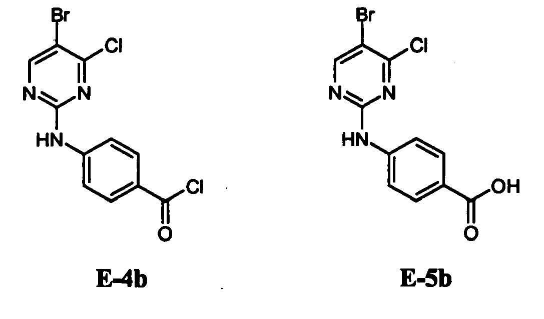

Die Herstellung erfolgt aus **E-3b** analog zur den Derivaten **E-4a** und **E-5a.**

### E-6b) [4-(5-Brom-4-chlor-pyrimidin-2-ylamino)-phenyl]-(4-methyl-piperazin-1-yl)-methanon

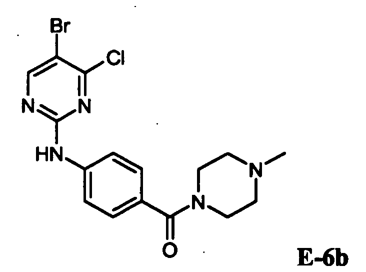

559 mg (1.6 mmol) **E-4b** werden in 5 mL THF gelöst und mit 414 µL (2.4 mmol, 1.5 eq) Hünig-Base versetzt. In diese Lösung werden 181 µL (1.6 mmol, 1 eq) *N*-Methylpiperazin getropft und 90 min bei RT gerührt. Danach werden 100 mL Wasser zugegeben und 3 x mit je 50 ml Ethylacetat extrahiert. Die vereinigten organischen Phasen werden über Magnesiumsulfat getrocknet und das Lösungsmittel im Vakuum entfernt. Man erhält 566 mg (1.4 mmol, 86%) **E-6b** in Form eines farblosen Harzes.
MS-ESI⁺: 410/412 (M+H)⁺ (1 x Br)

### E-7b) (±)-(1S*,2R*)-2-{5-Brom-2-[4-(4-methyl-piperazin-1-carbonl)-phenylamino]-pyrimidin-4-ylamino}-cyclopentancarbonsäure

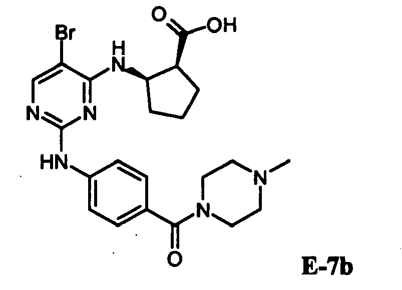

459 mg (1.1 mmol) **E-6b** werden in 5 mL 1-Butanol gelöst und mit 536 µL (3.1 mmol, 2.8 eq) Hünig-Base versetzt. Zu der Lösung werden 162 mg cis-2-Aminocyclopentancarbonsäure (racemisch) gegeben und die Reaktionsmischung 100 min bei 110 °C (CEM Mikrowelle, 100 W) gerührt. Das Reaktionsgemisch wird eingeengt, in ca 200 mL Wasser verrührt und 3 x mit je 50 mL Ethylacetat extrahiert. Die vereinigten organischen Phasen werden über Magnesiumsulfat getrocknet und das Lösungsmittel im Vakuum entfernt Man erhält 321 mg (0.64 mmol, 57%) **E-7b** in Form eines farblosen Harzes.
MS-ESI⁺: 503/505 (M+H)⁺ (1 x Br)

### E-8b)(±)-4-[5-Brom-4-((1R*,2S*)-2-carbamoyl-cyclopentylamino)-pyrimidin-2-ylamino]-benzoesäure

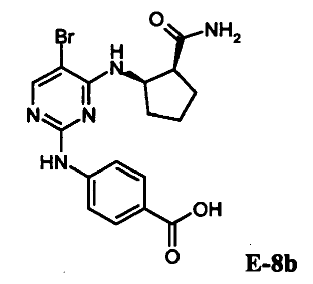

1 g (3.04 mmol) **E-5b** werden in 3.9 mL DMA gelöst und mit 1.3 µL (7.6 mmol, 1.5 eq) Hünig-Base versetzt. Zu der Lösung werden 390 mg (3.04 mmol, 1 eq) cis-2-Aminocyclopentancarbonsäureamid (racemisch) gegeben und die Reaktionsmischung 60 min bei 120 °C gerührt. Das Reaktionsgemisch wird eingeengt, der Rückstand in 5 mL 1-Butanol aufgenommen und der Niederschlag abgesaugt. Nach Waschen mit 5 mL kaltem 1-Butanol und Trocknen im Vakuum erhält man 935 mg (2.2 mmol, 73%) **E-8b** in Form eines beigen Feststoffs.
MS-ESI⁺: 420/422 (M+H)⁺ (1 x Br)

Das Jod-Derivat **E-8a** wird in analoger Weise aus **E-5a** hergestellt. Die Reaktionstemperatur beträgt dabei jedoch 80 °C.

### E-9b) (±)-4-[4-((1R*,2S*)-2-Carbamoyl-cyclopentylamino)-5-cyano-pyrimidin-2-ylamino]-benzoesäure

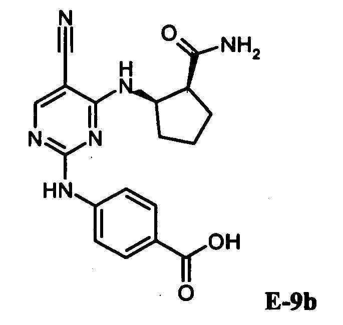

935 mg (2.23 mmol) **E-8b** werden in 8 mL DMF gelöst und unter Argon 403 mg (4.45 mmol, 2 eq) Kupfer(I)cyanid zugegeben. Die gelbe Lösung wird mit 80 mg (0.067 mmol, 3 mol%) Palladium-tetrakistriphenylphosphin versetzt und 24 h auf 145 °C erwärmt, wobei ca. 50% des Edukts umgesetzt werden. Es wird abermals die gleiche Menge Katalysator zugegeben, weitere 5 h erwärmt und die Reaktion dann aufgearbeitet. Das Reaktionsgemisch wird über eine mit Kieselgel gefüllte Fritte filtriert (Lösungsmittel: DMF), das Filtrat bis auf ca. 5 mL eingeengt und in ca. 400 mL destilliertes Wasser gegossen. Der dabei entstehende Niederschlag wird abfiltriert, mit 100 mL Wasser gewaschen und in Methanol gelöst. RP-Gel wird zugegeben und das Lösungsmittel im Vakuum entfernt. Über eine Umkehrphase wird chromatographisch gereinigt (von 5% Acetonitril (+0.2 % HCOOH) und 95 % Wasser (+0,2 % HCOOH) auf 50% Acetonitril (+0.2 % HCOOH) und 50 % Wasser (+0.2 % HCOOH)). Es werden 160 mg (0.44 mmol, 20%) **E-9b** als beigefarbener Feststoff isoliert.
R_{f}= 0.30 (Kieselgel, CH₂Cl₂:MeOH:AcOH 5:1:0.1)
MS-ESI⁺: 367 (M+H)⁺

### Beispiel 1

### (±)-(1S*,2R*)-2-{2-[4-(Methyl-phenyl-sulfamoyl)-phenylaminol]-5-trifluormethyl-pyrimidin-4-ylamino}-cyclopentancarbonamid (Syntheseschema A)

150 mg (0.6 mmol) **A-2,** 519 mg (1.98 mmol, 3 eq) 4-Amino-*N*-methyl-*N-*phenyl-benzolsulfonamid und 130 µL (0.76 mmol, 1.15 eq) *N*-Ethyldiisopropylamin werden in 3 mL *N,N*-Dimethylacetamid gelöst und die Lösung bei 180 °C 10 min gerührt (Erwärmung mittels Mikrowelle). Die Lösung wird in 30 mL Wasser eingerührt, mit 0.1 N HCl (aq) auf pH 3 gestellt, 3 x mit je 10 mL Ethylacetat extrahiert, über Magnesiumsulfat getrocknet und im Vakuum die flüchtigen Bestandteile entfernt. Der Rückstand wird säulenchromatographisch gereinigt (Cyclohexan/Ethylacetat 2/1). Man erhält 92 mg (0.2 mmol) N-Methyl-4-(4-methylsulfanyl-5-trifluormethyl-pyrimidin-2-ylamino)-*N*-phenyl-benzolsulfonamid als hellbraunen Feststoff.
85 mg (0.19 mmol) dieses Intermediats werden in 7.5 mL Dichlormethan gelöst, 64 mg (0.285 mmol, 1.5 eq, 77%) *m*-Chlorperbenzoesäure hinzu gegeben und 3 h bei RT gerührt. Die organische Phase wird 3 x mit 20 ml gesättigter, wässriger NaHCaO₃ Lösung gewaschen und damit die 3-Chlorbenzoesäure entfernt. Nach Trocknung der organischen Phase über Natriumsulfat erhält man 83 mg (0.18 mmol, 95%) 4-(4-Methansulfinyl-5-trifluormethyl-pyrimidin-2-ylamino)-*N*-methyl-*N*-phenyl-benzolsulfonamid **(A-4a),** das ohne weitere Reinigung in der nächsten Stufe eingesetzt wird.
83 mg (0.18 mmol) **A-4a,** 26 mg cis-2-Amino-1-cyclopentancarboxamid (0.2 mmol, 1.1 eq, racemisch) und 35 µL (0.2 mmol,1.1 eq) Hünig-Base werden in 2 mL DMA gelöst und 1 h bei 60 °C gerührt. Die Reaktionsmischung wird in 10 mL 0.1 N HCl (aq) eingerührt, 30 min nachgerührt, der entstandene Niederschlag abgesaugt, mit Wasser gewaschen und getrocknet. Abschließend erfolgt eine säulenchromatographische Reinigung (cHex/EE 60/40 auf 50/50 innerhalb von 20 min). Man erhält 43 mg (0.08 mmol, 45%) der Verbindung 1 als farblosen Feststoff

In analoger Weise werden **Beispiel 2** und **3** hergestellt.

### Beispiel 4

### (±)-N-((1S*2R*)-2-{2-[4-(4-Methyl-piperazin-1-carbonyl)-phenylamino]-5-trifluormethyl-Dyrimidin-4-ylamino}-cyclohexyl)-acetamid (Syntheseschema C)

38 mg (0.08 mmol) **C-3b** werden in 50 µL DMA gelöst, 25 µL (0.16 mol, 2 eq) Hünig-Base zugegeben und einige Minuten bei RT gelöst. 5 µL Acetylchlorid (1 eq) wird in wenig DMA gelöst und zu der Reaktionsmischung getropft. Nach ca. 10 min wird das Reaktionsgemisch in Dichlormethan aufgenommen, mit 10 mL RP-Gel versetzt und alle flüchtigen Bestandteile im Vakuum entfernt. Es wird chromatographisch über eine RP-Phase gereinigt (AcCN/Wasser 5/95 auf 95/5% in 20 min). Nach Vereinigung der Produktfraktionen und Gefriertrocknung erhält man 18 mg (0.034 mmol, 42%) der Verbindung **4** als farblosen Feststoff.

In analoger Weise werden **Beispiel 5-12** hergestellt.

### Beispiel 13

### (±)-1-Methy-3((1S*,2R*)-2-{2-[4-(4-methyl-piperazin-1-carbonyl)-phenylamino]-5-trifluormethyl-pyrimidin-4-ylamino}-cyclohexyl)-harnstoff (Syntheseschema C)

50 mg (0.105 mmol) **C-3b** werden in 50 µL DMF gelöst und mit 55 µL (0.315 mol, 3 eq) Hünig-Base versetzt. Zu dieser Lösung werden bei RT 6 µL Methylisocyanat (1 es) gegeben. Nach ca. 10 min wird das Reaktionsgemisch in Dichlormethan aufgenommen, mit 10 mL RP-Gel versetzt und alle flüchtigen Bestandteile im Vakuum entfernt. Es wird chromatographisch über eine RP-Phase gereinigt (AcCN/Wasser 5/95 auf 95/5% in 20 min). Nach Vereinigung der Produktfraktionen und Gefriertrocknung erhält man 24 mg (0.045 mmol, 43%) der Verbindung **13** als farblosen Feststoff.

In analoger Weise werden **Beispiele 14 - 17** hergestellt.

### Beispiel 18

### ((±)-(1S*,2R*)-2-{2-[4-(4-Methyl-piperazin-1-carbonyl)phenylaminol]-5-trifluormethyl-pyrimidin-4-ylaminol}-cyclohexyl)-carbaminäuremethylester (Syntheseschema C)

30 mg (0.063 mmol) **C-3b** werden in 50 µL DMF gelöst und mit 22 µL (0.126 mmol, 2 eq) Hünig-Base versetzt. Zu dieser Lösung werden bei RT 6 µL Chlorameisensäuremethylester (1.2 eq) gegeben. Nach ca. 10 min wird das Reaktionsgemisch in Dichlormethan aufgenommen, mit 10 mL RP-Gel versetzt und alle flüchtigen Bestandteile im Vakuum entfernt. Es wird chromatographisch über eine RP-Phase gereinigt (AcCN/Wasser 5/95 auf 95/5% in 20 min). Nach Vereinigung der Produktfraktionen und Gefriertrocknung erhält man 13 mg (0.025 mmol, 39%) der Verbindung **13** als farblosen Feststoff.

In analoger Weise werden **Beispiel 19** und **20** hergestellt.

### Beispiel 21

### [4-(4-Cyclopentylamino-5-trifluormethyl-pyrimidin-2-ylamino)phenyl]-4-methyl-piperazin-1-yl)-methanon (Syntheseschema C)

88 mg (0.22 mmol) **C-2a** werden in 290 µL DMA gelöst, 26 µL (0.26 mmol, 1.2 eq) Cyclopentylamin sowie 75 µL (0.44 mmol, 2 eq) Hünig-Base zugegeben und die Reaktionsmischung auf 120 °C erwärmt. Nach ca. 90 min wird das Reaktionsgemisch in ca. 10 mL destilliertes Wasser gegossen und der entstehende Niederschlag abfiltriert. Die Suspension wird 3 x mit je 20 mL Ethylacetat extrahiert, die vereinigten organischen Phasen mittels gesättigter wässriger NaCl-Lösung und Magnesiumsulfat getrocknet, mit 100 µL dioxanischer HCl versetzen und alle flüchtigen Bestandteile im Vakuum entfernt. Man erhält 106 mg (0.219 mmol, 99%) der Verbindung **21** in Form des Hydrochlorids.

In analoger Weise werden **Beispiel 22 - 26** hergestellt.

### Beispiel 27

### (±)-(1S*,2R*)-2-{2-[4-Methyl-piperazin-1-carbonyl)-phenylamino]-5-trifluormethyl-pyrimidin-4-ylamino}-cycloheptancarbonsäuredimethylamid (Syntheseschema C)

35 mg (0.067 mmol) **C-3d** werden in 250 µL DMF gelöst, 30 µL (0.175 mmol, 2.6 eq Hünig-Base und abschließend 35 mg (0.11 mmol, 1.6 eq) TBTU zugegeben. Die Reaktionsmischung wird 10 min bei RT gerührt und dann mit 118 µL Dimethylamin (2 M Lösung in THF, 0.235 mmol, 3.5 eq) versetzt. Es wird 4 h bei 35 °C geschüttelt, danach das Reaktionsgemisch in Acetonitril aufgenommen und mit 6 mL RP-Gel versetzt und alle flüchtigen Bestandteile im Vakuum entfernt. Die Reinigung erfolgt säulenchromatographisch über RP-Phase (Acetonitril/Wasser 12/88 auf 40/60 in 12 min). Die Produktfraktionen werden gefriergetrocknet und 19 mg (0.035 mmol, 52%) der Verbindung **27** erhalten.

In analoger Weise werden **Beispiel 28 - 30** hergestellt.

### Beispiel 31

### (+)-4-(4-((1R*,2S)-2-Isopropylcarbamoyl-cyclopentylamino)-5-trifluormethyl-pyrimidin-2-ylamino]-N-[2-(1-methyl-pyrrolidin-2-yl)-ethyl]-benzamid (Syntheseschema D)

Es werden 80 mg (0.18 mmol) **D-4c** in 2.4 mL DMF gelöst, 179 µL (1.03 mol, 1.5 eq) Hünig-Base zugegeben und die Lösung mit 83 mg (0.25 mmol, 1.4 eq) TBTU versetzt. Die Lösung wird 40 min bei RT gerührt, danach werden 38.5 µL (0.27 mmol, 1.5 eq) 2-(2-Aminoethyl)-1-methylpyrrolidin zugegeben und 2 Tage gerührt. Danach wird Kieselgel zu der Reaktionsmischung gegeben und die flüchtigen Bestandteile im Vakuum entfernt. Die Reinigung erfolgte säulenchromatographisch über eine Normalphasen-Chromatographie (DCM/MeOH/NH₃(aq) 5/1/0.1). Man erhält 70 mg (0.125 mmol, 70%) der Verbindung **31.**

In analoger Weise werden **Beispiel 32 - 58** hergestellt.

### Beispiel 59

### (±)-(1S*,2R*)-2-{2-[4-(4-Methyl-piperazin-1-carbonyl)-phenylamino]-5-trifluormethyl-pyrimidin-4-ylamino}-cyclopentancarbonsäureisopropylamid (Syntheseschema C)

Es werden 88 mg (0.18 mmol) **C-3d** in 2 mL DMF gelöst, 153 µL (0.90 mmol, 5 eq) Hünig-Base zugegeben und die Lösung mit 81 mg (0.25 mmol, 1.4 eq) TBTU versetzt. Die Lösung wird 20 min bei RT gerührt, danach werden 12 µL (0.27 mmol, 1.5 eq) Isopropylamin zugegeben und 16 h gerührt. Danach wird über basisches Aluminiumoxid filtriert und mit 20 mL Methanol nachgewaschen. Zum Filtrat wird RP-Gel gegeben und die flüchtigen Bestandteile im Vakuum entfernt. Das auf dem RP-Gel immobilisierte Rohprodukt wird über eine Umkehrphase gereinigt (von 95% Wasser (+0.2% HCOOH) und 5% Acetonitril (+0.2% HCOOH) auf 55% Wasser und 45% Acetonitril in 20 min). Entsprechende Produktfraktionen werden mit 1 eq konzentrierter Salzsäure versetzt und mittels Gefriertrocknung vom Lösemittel befreit. Zurück bleiben 14 mg (0.025 mmol, 14%) des Hydrochlorids der Verbindung **59** als farbloser Film.

### In analoger Weise werden Beispiel 60 - 69 hergestellt.

Die Beispiele **68** und **69** sind chiral, sie werden entsprechend aus **C-2a,** Verwendung der Enantiomere der cis-2-Aminocyclopentancarbonsäure und abschließender Bildung des Isopropylamids hergestellt. Alternativ können **68** und **69** auch durch präparative chirale HPLC aus **59** gewonnen werden.

### Beispiel 70

### (±)-(1S*,2R*)-2-{2-[3-Chlor-4-(4-methyl-piperazin-1-carbonyl)-phenylamino]-5-trifluormethyl-pyrimidin-4-ylamino}-cyclopentancarbonsäureisopropylamid (Syntheseschema B)

30 mg (85.5 mmol) **B-2a** werden in 100 µL NMP gelöst und mit 35 mg (0.14 mmol, 1.6 eq) (4-Amino-2-chlor-phenyl)-(4-methyl-piperazin-1-yl)-methanon versetzt. Zu dieser Reaktionsmischung werden 107 µL 4 M HCl in Dioxan (0.43 mmol, 5 eq) gegeben und **12** h bei 5 °C gerührt._Das Reaktionsgemisch wird in DCM/MeOH/NH₃ 9/1/0.1 aufgenommen und mit 6 mL RP-Gel versetzt, die flüchtigen Bestandteile im Vakuum entfernt und über eine RP-Phase chromatographisch gereinigt (von 5% Acetonitril auf 95% Acetonitril in 10 min). Entsprechende Produktfraktionen werden mittels Gefriertrocknung vom Lösemittel befreit. Zurück bleiben 35 mg (0.06 mmol, 72%) der Verbindung **70.**

In analoger Weise werden **Beispiel 71- 75** hergestellt.

### Beispiele 76 -105 (Allgemeine Arbeitsvorschrift)

1 eq der Verbindung **B-4** (für die **Beispiele 98-101** die Verbindung **E-8b** und für die **Beispiele 102-105** die Verbindung **E-8a)** wird in DMF (ca. 1-10 mL pro mmol) gelöst, 4 - 6 eq Hünig-Base und anschließend 1.3-1.5 eq TBTU zugegeben. Die Reaktionsmischung wird 10-30 min bei RT gerührt und anschließend 1 -1.5 eq des Amins oder Anilins zugegeben. Nach Ende der Reaktion wird die Reaktionsmischung mit Kieselgel versetzt, alle flüchtigen Bestandteile im Vakuum entfernt und das Produkt durch eine Säulenchromatographie (Normal- oder RP-Phase) gereinigt und isoliert.

### Beispiel 106

### (±)-(3-[4-((1R*,2S*)-2-Carbamoyl-cyclopentylamino)-5-trifluormetlyl-pyrimidin-2-ylamino]-N-phenylbenzamid (Syntheseschema A)

700 mg (3.06 mmol) **A-3** werden in 6 mL DMA gelöst. 800 µL (4.6 mmol, 1.5 eq) Hünig-Base wird zugegeben und 440 mg cis-2-Amino-1-cyclopentancarboxamid, gelöst in 24 mL DMA, zugetröpft. Die Reaktionsmischung wird bei RT gerührt. Nach 1 h wird mit 400 mL Dichlormethan verdünnt und 2 x mit je 200 mL halbgesättigter Ammoniumchloridlösung extrahiert, danach über Magnesiumsulfat getrocknet und das Lösungsmittel im Vakuum entfernt. Zurück bleiben 1.1 grobes (±)-(1S*,2R*)-2-(2-Methylsulfanyl-5-trifluormethyl-pyrimidin-4-ylamino)-cyclopentancarbonsäureamid als beigen Feststoff. Dieses wird ohne Reinigung weiter umgesetzt.
Dazu wird der Feststoff in 60 mL THF gelöst, portionsweise 1.31 g (5.5 mmol, 77% 2 eq) mCPBA zugegeben und 1 h bei RT gerührt. Die organische Phase wird 3 x mit 20 ml gesättigter, wässriger Natriumhydrogencarbonat Lösung gewaschen und damit die 3-Chlorbenzoesäure entfernt. Nach Trocknung der organischen Phase über Magnesiumsulfat erhält man 1.15 g rohes (±)-(1S*,2R*)-2-(2-Methansulfinyl-5-trifluormethyl-pyrimidin-4-ylamino)-cyclopentancarbonsäureamid, das ohne weitere Reinigung in der nächsten Stufe eingesetzt wird.
150 mg (0.45 mmol) (+)-(1S*,2R*)-2-(2-Methansulfinyl-5-trifluormethyl-pyrimidin-4-ylamino)-cyclopentancarbonsäureamid werden in 500 µl NMP gelöst, 148 mg (0.68 mmol, 1.5 eq) *m*-Aminobenzanilid zugegeben. Zu dieser Lösung werden 34 µL Salzsäure (4 M Lösung in Dioxan, 0.3 eq) gegeben und 16 h bei 50 °C gerührt. Die Reaktionsmischung wird in 30 mL Wasser eingerührt, mit 10 mL 0,1 N HCl auf pH 3 gestellt und 3 x mit je 15 mL Ethylacetat extrahiert. Die vereinigten organischen Phasen werden über Magnesiumsulfat getrocknet, alle flüchtigen Bestandteile im Vakuum entfernt und das Rohprodukt in Cyclohexan/Ethylacetat 60/40 verrührt, der Niederschlag abgesaugt und mit 2-Propanol gewaschen. Man erhält 15 mg (0.03 mmol, 7%) der Verbindung **106** als farblosen Feststoff.

In analoger Weise werden **Beispiel 107-109** hergestellt. Hier erfolgte die Reinigung säulenchromatographisch (Ethylacetat/Cyclohexan, Kieselgel).

### Beispiel 110

### (±)-((1S,2R)-2-{5-Brom-2-[4-(4-meth-piperazin-1-carbonyl)-phenylaminol-pyrimidin-4-ylaminol}-cyclopentancarbonsäure-cyclopropylamid (Syntheseschema E)

39 mg (0.077 mmol) **E-7b** werden in 500 µL DMF gelöst, 66 µL (0.39 mmol, 5 eq) Hünig-Base und 35 mg (0.11 mmol, 1.4 eq) TBTU zugegeben. Die Lösung wird 20 min bei RT gerührt und nun werden 8 µL (0.116 mmol, 1.5 eq) Cyclopropylamin zugegeben und über Nacht bei RT gerührt. Es wird über basisches Aluminiumoxid filtriert, mit ca. 20 mL Methanol nachgewaschen und das Filtrat mit 8 mL RP-Gel versetzt. Nach Entfernen der flüchtigen Bestandteile im Vakuum wird über eine Umkehrphase gereinigt. (von 95% Wasser (+0.2% HCOOH) und 5% Acetonitril (+0.2% HCOOH) auf 5% Wasser und 95% Acetonitril in 20 min). Entsprechende Produktfraktionen werden mittels Gefriertrocknung vom Lösemittel befreit. Man erhält Verbindung **110** als farblosen Film, 12 mg (0.021 mmol, 27%).
MS-ESI⁺: 542/544 (M+H)⁺(1 Br)

In analoger Weise werden **Beispiel 111-120** hergestellt.

### Beispiel 121

### N-Methyl-N-(1-methyl-piperidin-4-yl-4-{4-[(±)-1R*,2S*)-2-(pyrrolidin-1-carbonyl)-cyclopentylamino]-5-trifluormethyl-pyrimidin-2-ylamino}-benzamid (Syntheseschema C)

80 mg (0.15 mmol) **C-3e** werden in 1.4 mL DMF gelöst, 132 µL (0.77 mmol, 5 eq) Hünig-Base und 69 mg (0.22 mmol, 1.4 eq) TBTU zugegeben. Die Reaktionsmischung wird 30 min bei RT gerührt, nun werden 119 µL (0.144 mmol, 9.4 eq) Pyrrolidin zugegeben und 16 h bei RT gerührt. Es wird über basisches Aluminiumoxid filtriert, mit ca. 20 mL Methanol nachgewaschen und das Filtrat mit Kieselgel versetzt. Nach Entfernen der flüchtigen Bestandteile im Vakuum wird säulenchromatographisch gereinigt. (DCM/MeOH/NH₃ 9/1/0.1). Nach Sammlung der Produktfraktionen, Versetzen mit 100 µL HCl (4 M Lösung in Dioxan) und Entfernen des Lösungsmittels im Vakuum erhält man das Hydrochlorid der Verbindung **121** als farblosen Film, 29 mg (0.048 mmol, 31%).
MS-ESI⁺: 574 (M+H)⁺

In analoger Weise wurden **Beispiel 122 -128** hergestellt.

### Beispiel 129

### 4-[4-((1R,3S)-3-Carbamoyl-cyclopentylamino)-5-trifluormethyl-pyrimidin-2-ylamino]-N-methyl-N-(1-methyl-piperidin-4-yl)-benzamid (Syntheseschema C)

75 mg (0.14 mmol) **C-3f** werden werden in 1 mL DMF gelöst, 123 µl (0.7 mmol, 5 eq) Hünig-Base zugegeben und die Reaktionsmischung 30 min lang gerührt. Nun werden 14 µL (0.22 mmol, 1.5 eq) wässrige Ammoniaklösung (28%) zugegeben und bei RT 5 h lang gerührt. Die Lösung wird mit RP-Gel versetzt, alle flüchtigen Bestandteile im Vakuum entfernt und säulchenchromatographisch gereinigt (von 10% Acetonitril (+0.2 % HCOOH) und 90% Wasser (+0.2 % HCOOH) auf 24% Acetonitril und 76% Wasser in 12 min). Die Produktfraktionen werden mit 100 µL dioxanischer HCl versetzt und alle flüchtigen Bestandteile mittels Gefriertrocknung entfernt. Man erhält 35 mg (0.063 mol, 44%) der Verbindung **129** in Form des Hydrochlorids.

In analoger Weise wird Beispiel **130** hergestellt.

### Beispiel 131

### (±)-(1S*,2R*)-2-[2-(4-Acetylamino-phenylamino)-5-trifluormethyl-pyrimidin-4-ylamino]-cyclopentancarbonsäureisoiproipylamid (Syntheseschema D)

22 mg **D-6c** werden in 1 mL THF gelöst, mit 14 µL (0.075 mmol, 1.5 eq) Hünig-Base versetzt und nun 3 µL Acetylchlorid, gelöst in 500 µL THF, zugegeben. Nach ca. 90 min wird die Reaktionslösung mit 10 mL Methanol verdünnt und 8 mL RP-Gel zugegeben. Es erfolgt eine chromatographische Reinigung über eine Umkehrphase (von 78% Wasser (+0.2% HCOOH) und 22% Acetonitril (+0.2% HCOOH) auf 5 1 % Wasser und 49% Acetonitril in 15 min). Die entsprechenden Produktfraktionen werden vereinigt und das Lösungsmittel mittels Gefriertrocknung entfernt. Man erhält 14 mg (0.028 mmol, 54%) der Verbindung **131.**

In analoger Weise werden **Beispiel 132 - 133** hergestellt.

### Beispiel 134

### (±)-(1S*,2R*)-2-{5-Cyano-2-[4-(4-methyl-piperazin-1-carbonl)-phylaminol]-pyrimidin-4-ylamino}-cyclopentancarbonsäureamid (Syntheseschema E)

40 mg (0.11 mmol) **E-96** werden in 1.5 mL DMF gelöst, 110 µL (0.63 mmol, 5.8 eq) Hünig-Base zugegeben und die Reaktionsmischung 40 min lang gerührt. Nun werden 18 µL (0.16 mmol, 1.5 eq) *N*-Methylpiperazin zugegeben und bei RT 48 h lang gerührt. Die Lösung wird mit Kieselgel versetzt, alle flüchtigen Bestandteile im Vakuum entfernt und säulchenchromatographisch gereinigt (DCM/MeOH 9/1). Man erhält 33 mg (0.07 mol, 67%) der Verbindung **134.**

In analoger Weise werden **Beispiel 135 - 136** hergestellt.

### Beispiel 137

### (±)-(1S*,2R*)-2-{5-Cyclopropylethynyl-2-[4-methyl-piperazin-1-carbonyl)-phenylamino]-pyrimidin-4-ylamino}-cyclopentancarbonsäureamid (Syntheseschema E)

50 mg (0.09 mmol) **105** werden in 220 µL DMF gelöst und nachfolgend 15 mg Dichlorbis(triphenylphosphin)palladium (0.021 mmol, 23 mol%) sowie 10 mg (0.03 mmol, 0.58 eq) Kupfer(I)jodid zugegeben. Die Lösung wird mit 320 µL Hünig-Base versetzt und danach mit 18 mg (0.27 mmol, 3 eq) Ethinylcyclopropan. Die Reaktionsmischung wird mit einem Gemisch aus DCM/MeOH/NH₃ 4/1/0.1 über Kieselgel filtriert und nachfolgend 6 mL RP-Gel zugegeben. Nach Entfernen der flüchtigen Bestandteile erfolgt säulenchromatographische Reinigung über eine RP-Phase, (von 95% Wasser (+0.2% HCOOH) und 5% Acetonitril (+0.2% HCOOH) auf 50% Wasser und 50% Acetonitril in 20 min). Die entsprechenden Produktfraktionen werden vereinigt und das Lösungsmittel mittels Gefriertrocknung entfernt. Man erhält 32 mg (0.065 mmol, 71 %) der Verbindung **137.**

In analoger Weise werden **Beispiel 138 -139** hergestellt, wobei in Beispiel **138** die Reaktion unter einer Propinatmosphäre in einem Stickstoffkolben bei 40 °C durchgeführt wird.

### Beispiel 140

### (±)-4-[4-((1R*,2S*)-2-Carbamoyl-cyclopentylamino)-5-cyclopropyl-pyrimidin-2-ylamino]-N-(1-methyl-piperidin-4-yl)-benzamid (Syntheseschema E)

100 mg (0.15 mmol) **104** werden in 1.4 mL Dioxan suspendiert und 13 mg (0.15 mmol, 1 eq) Cyclopropylboronsäure zugegeben. Die Lösung wird im Vakuum entgast und unter Argon werden 3.5 mg (0.004 mmol, 3 mol%) Dichlor[1,1'-bis(diphenylphosphino)-ferrocen]palladium(II)-Dichlormethan Addukt (PdCl₂dppf DCM) zugegeben sowie 2 mL Natriumcarbonat-Lösung (2 M in Wasser). Das Zwei-Phasen-Gemisch wird 5 min auf 130 °C erwärmt (CEM Mikrowelle, 100 W). Die organische Phase wird abgetrennt, mit Methanol verdünnt und mit 6 mL RP-Gel versetzt. Nach Entfernen der flüchtigen Bestandteile erfolgt säulenchromatographische Reinigung über eine Umkehrphase. (von 97%, Wasser (+0.2% HCOOH) und 3% Acetonitril (+0.2% HCOOH) auf 70% Wasser und 30% Acetonitril in 12 Minuten v). Die entsprechenden Produktfraktionen werden vereinigt und das Lösungsmittel mittels Gefriertrocknung entfernt. Man erhält 2 mg (0.003 mmol, 2%) der Verbindung **140.**

### Beispiel 141

### (±)-(1S*,2R*)-2-[2-(4-[1,4]Diazepan-1-yl-3-fluor-phenylamino)-5-trifluormethyl-pyrimidin-4-ylamino]-cyclopentancarbonsäureisopropylamid (Syntheseschema B)

23 mg (0.066 mmol) **B-2a** werden in 100 µL NMP gelöst, 17 mg (0.079 mmol, 1.2 eq) 3-Fluor-4-(4-methyl-[1,4]diazepan-1-yl)-phenylamin und abschließend 46 µL HCl (0.18 mmol, 2.8 eq, 4 M Lösung in Dioxan) zugegeben. Die Reaktionsmischung wird für 12 h bei 90 °C erwärmt, mit 6 mL RP-Gel versetzt und die flüchtigen Bestandteile im Vakuum entfernt. Es erfolgt eine chromatographische Reinigung über eine Umkehrphase (von 95% Wasser (+0.2% HCOOH) und 5% Acetonitril (+0.2% HCOOH) auf 55% Wasser und 45% Acetonitril in 25 min). Die entsprechenden Produktfraktionen werden vereinigt und das Lösungsmittel mittels Gefriertrocknung entfernt. Man erhält 3 mg (0.005 mmol, 8%) der Verbindung **141.**

In analoger Weise werden **Beispiel 142 -144** hergestellt.

### Beispiel 145

### (±)-(1R*,2R*)-2-{2-[4-(4-Methyl-piperazin-1-carbonyl)-phenylaminol]-5-trifluormethyl-pyrimidin-4-ylamino}-cyclopentancarbonsäureamid (Syntheseschema C)

100 mg (0.25 mmol) **C-2a** werden in 1 mL 1-Butanol gelöst und diese Lösung mit 35 mg (0.275 mmol, 1.1 eq) racemischem trans-2-Aminocyclopentancarbonsäureamid sowie 60 µL (0.35 mmol, 1.4 eq) Hünig-Base versetzt. Bei 110 °C (100W, Mikrowelle CEM) wird 30 min bis zum vollständigen Umsatz gerührt. Man gibt zum Reaktionsgemisch ca. 20 mL Methanol, versetzt mit RP-Gel (ca. 8 mL) und entfernt alle flüchtigen Bestandteile im Vakuum. Es wird über eine RP-Säule gereinigt (von 95% Wasser (+0.2% HCOOH) und 5% Acetonitril (+0.2% HCOOH) auf 55% Wasser und 45% Acetonitril in 20 min). Entsprechende Produktfraktionen werden mit konzentrierter Salzsäure versetzt und mittels Gefriertrocknung vom Lösemittel befreit. Man erhält 77 mg (0.146 mmol, 58%) der Verbindung **145** als farblosen Feststoff.

In analoger Weise werden Beispiel **146 -147** hergestellt, wobei die Herstellung von Beispiel **148** analog zu Beispiel **129** erfolgt (nukleophile Substitution mit der β-Aminosäure ausgehend von **C-2a** und abschließende Amidknüpfung mit Ammoniak).

### Beispiele 1-148

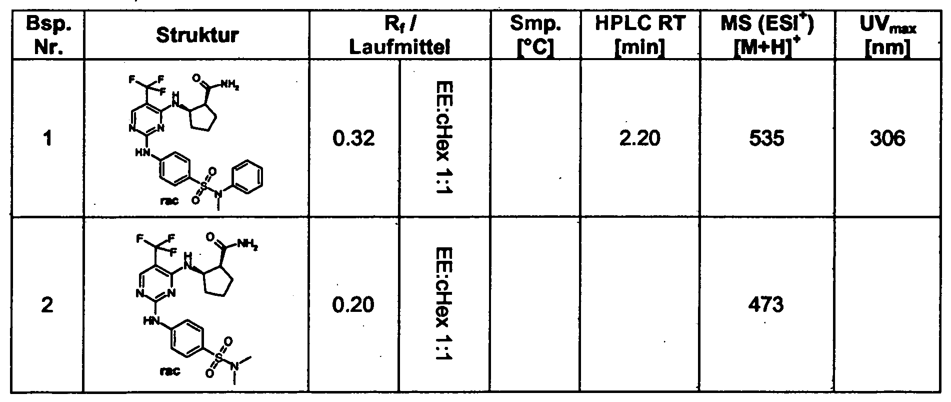

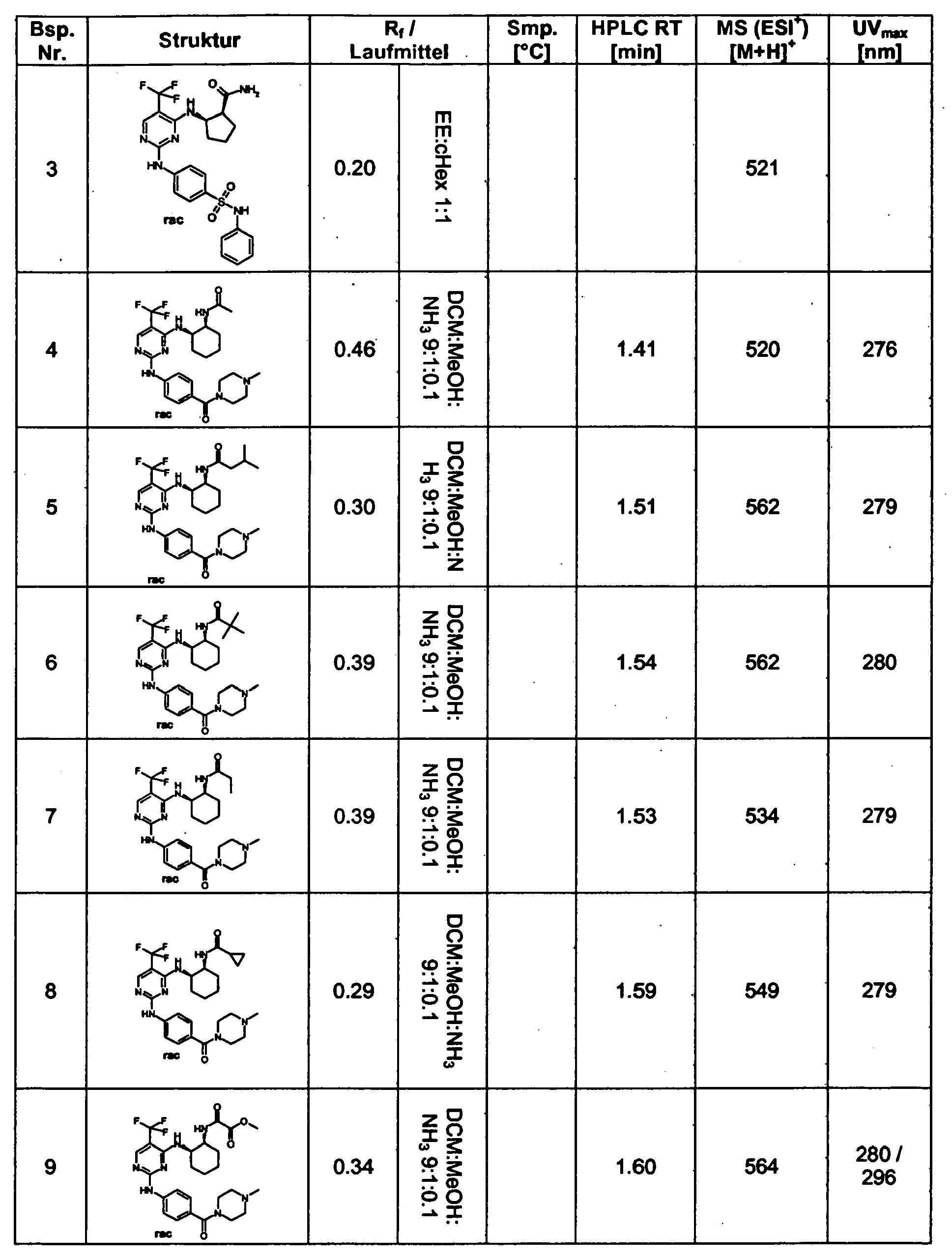

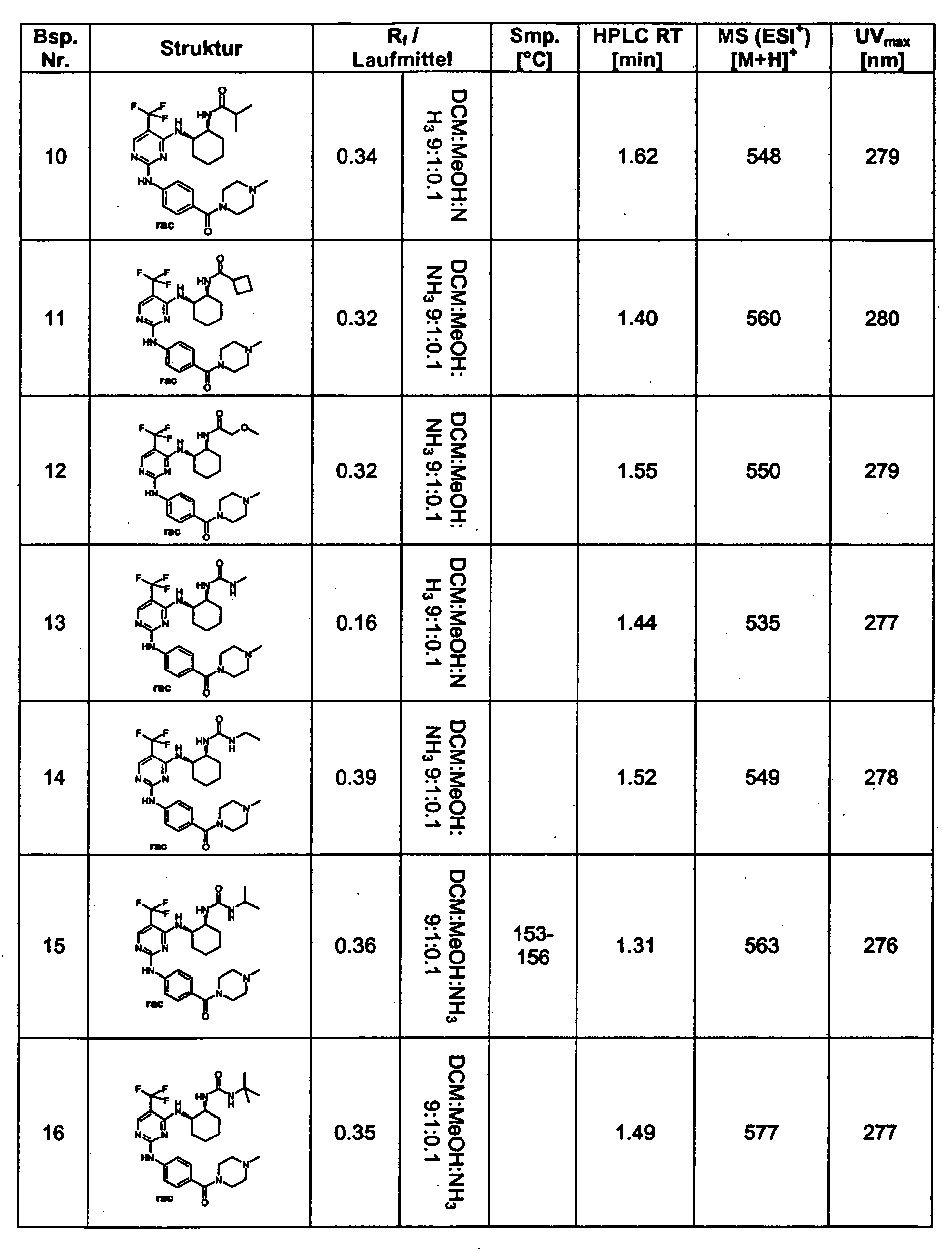

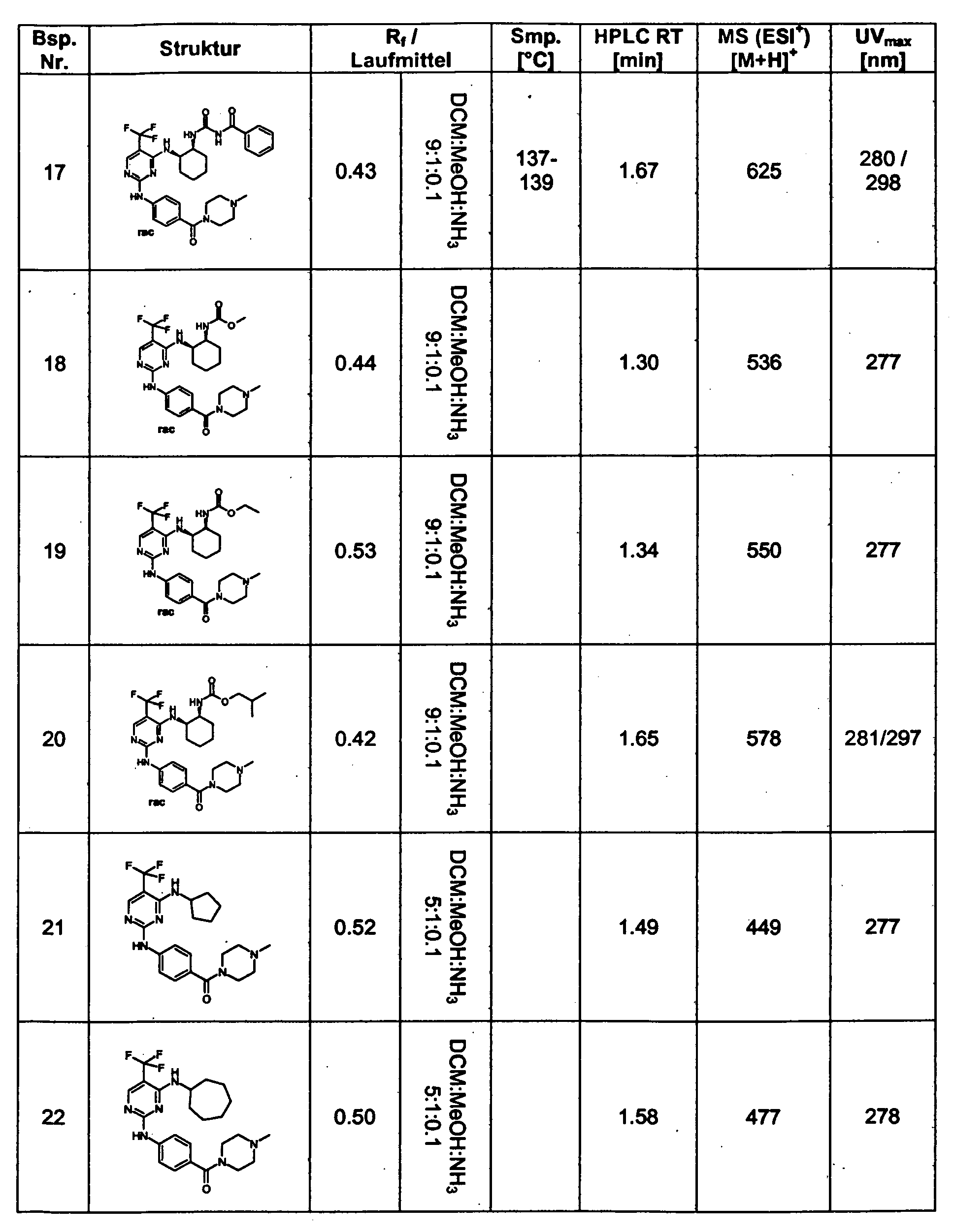

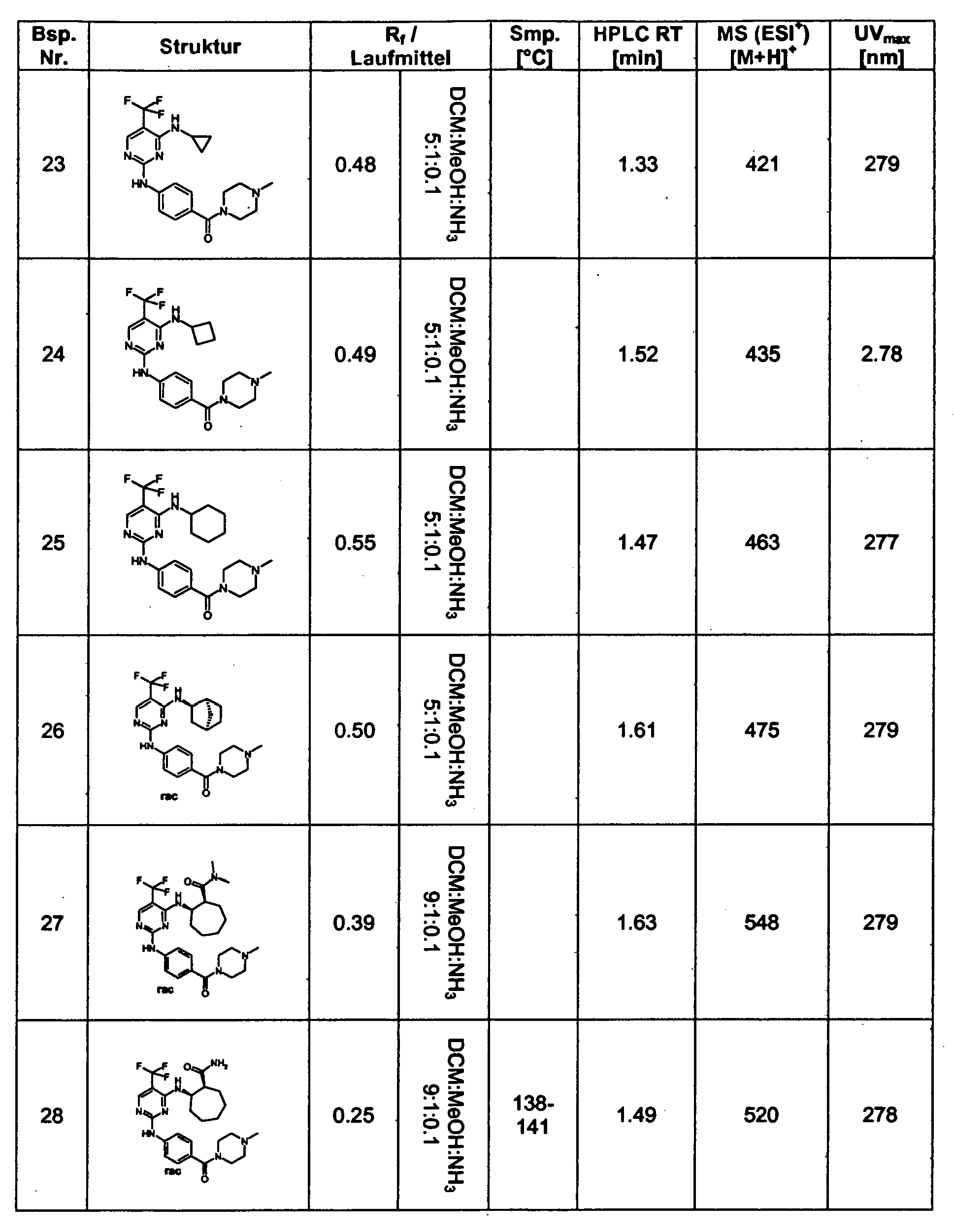

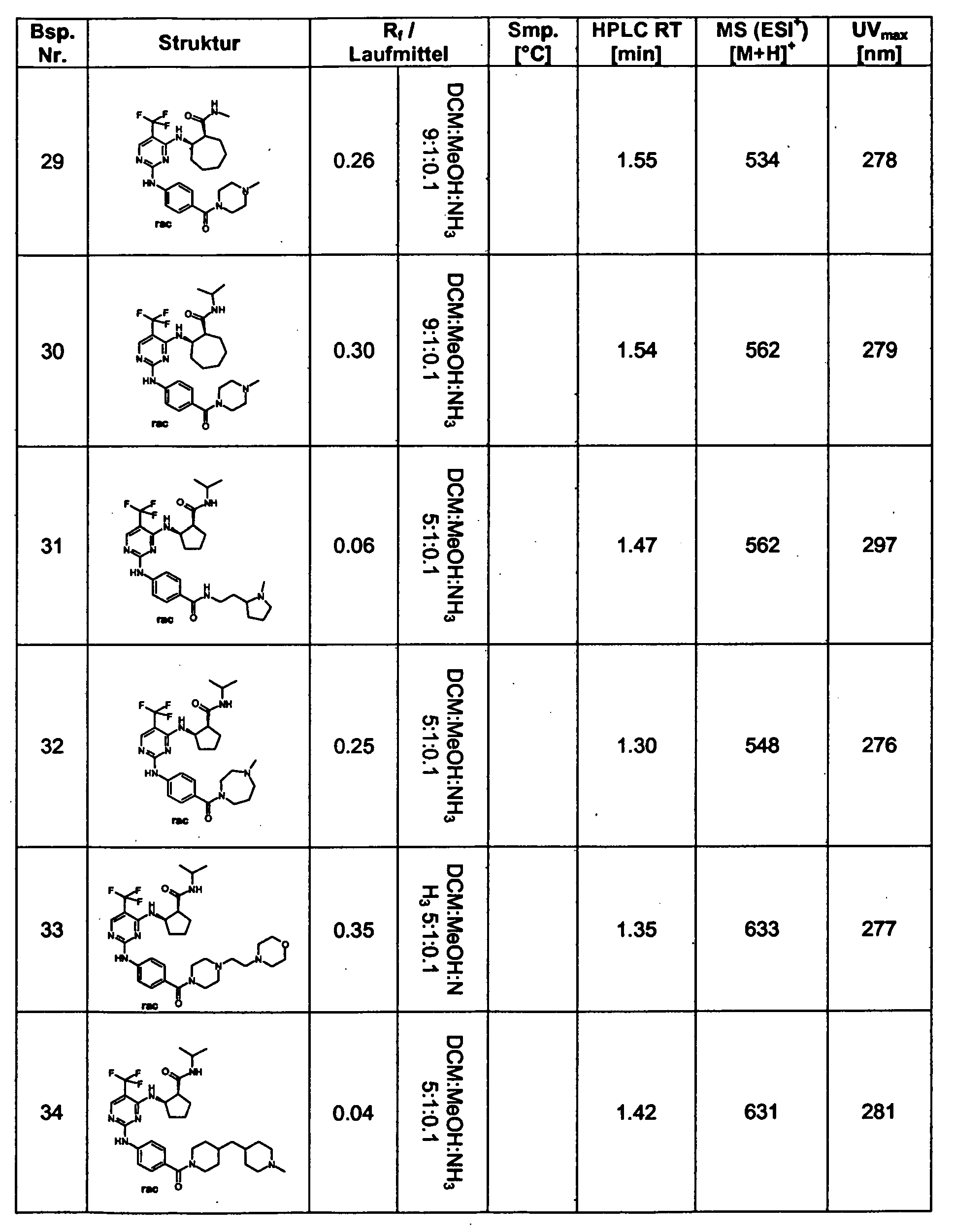

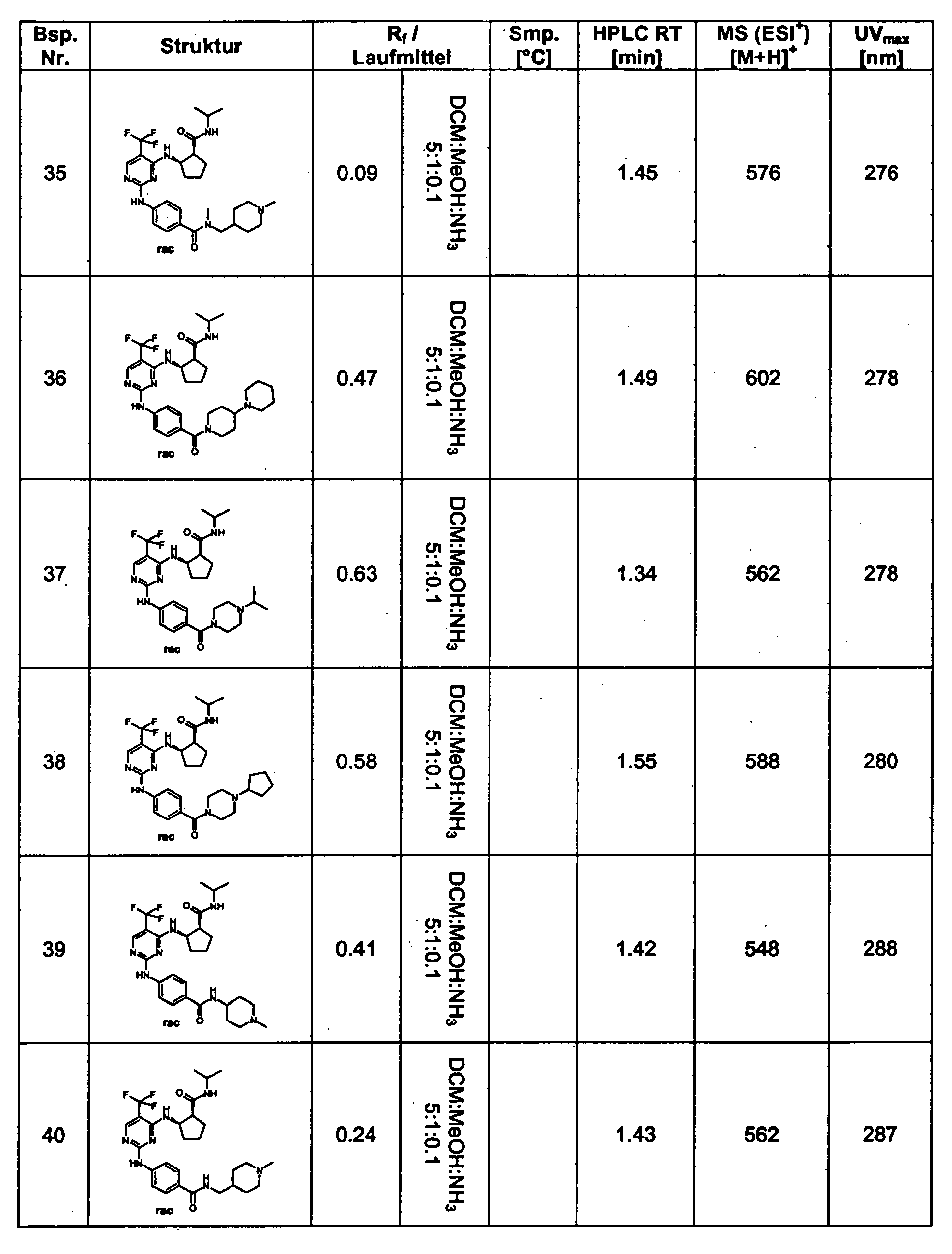

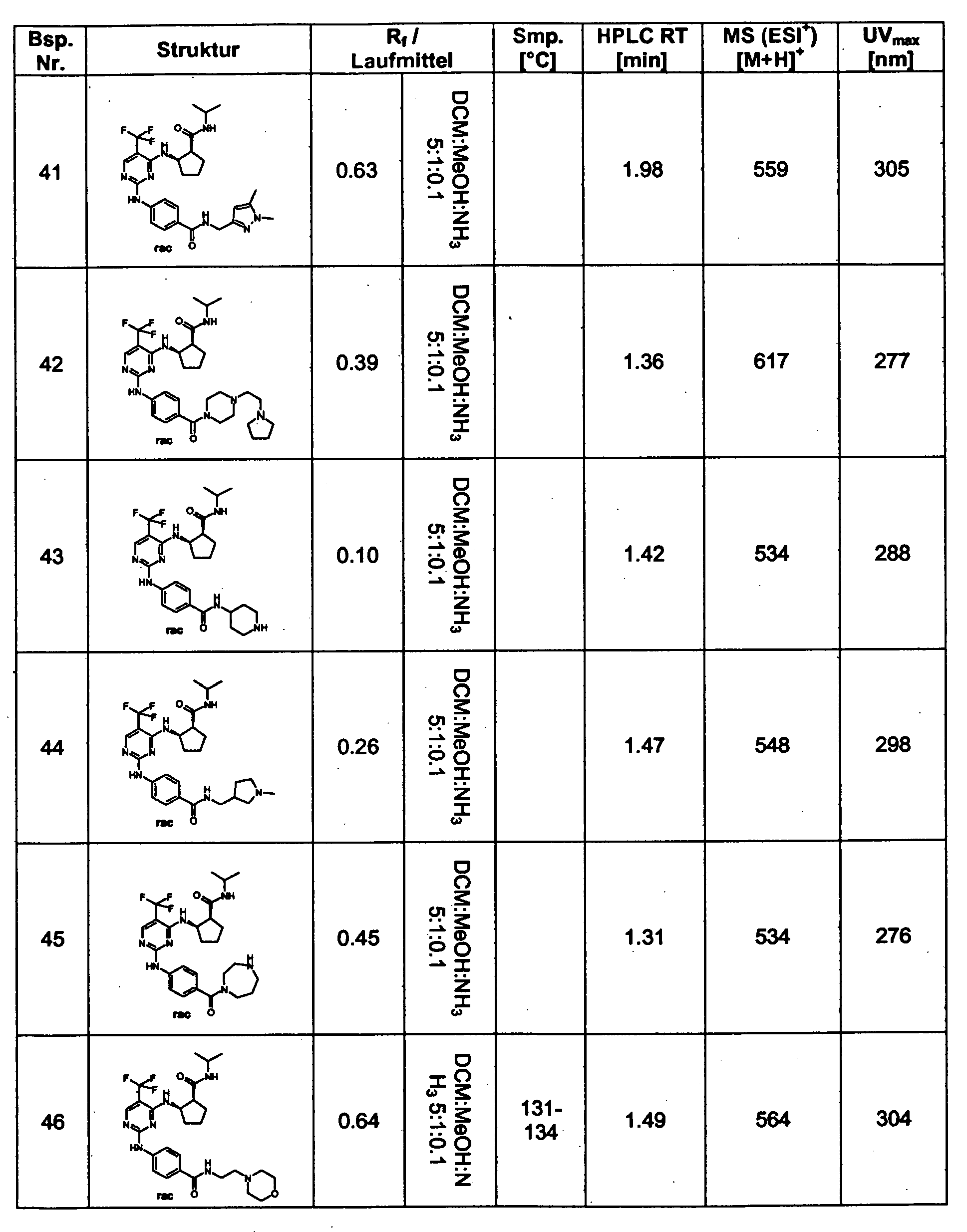

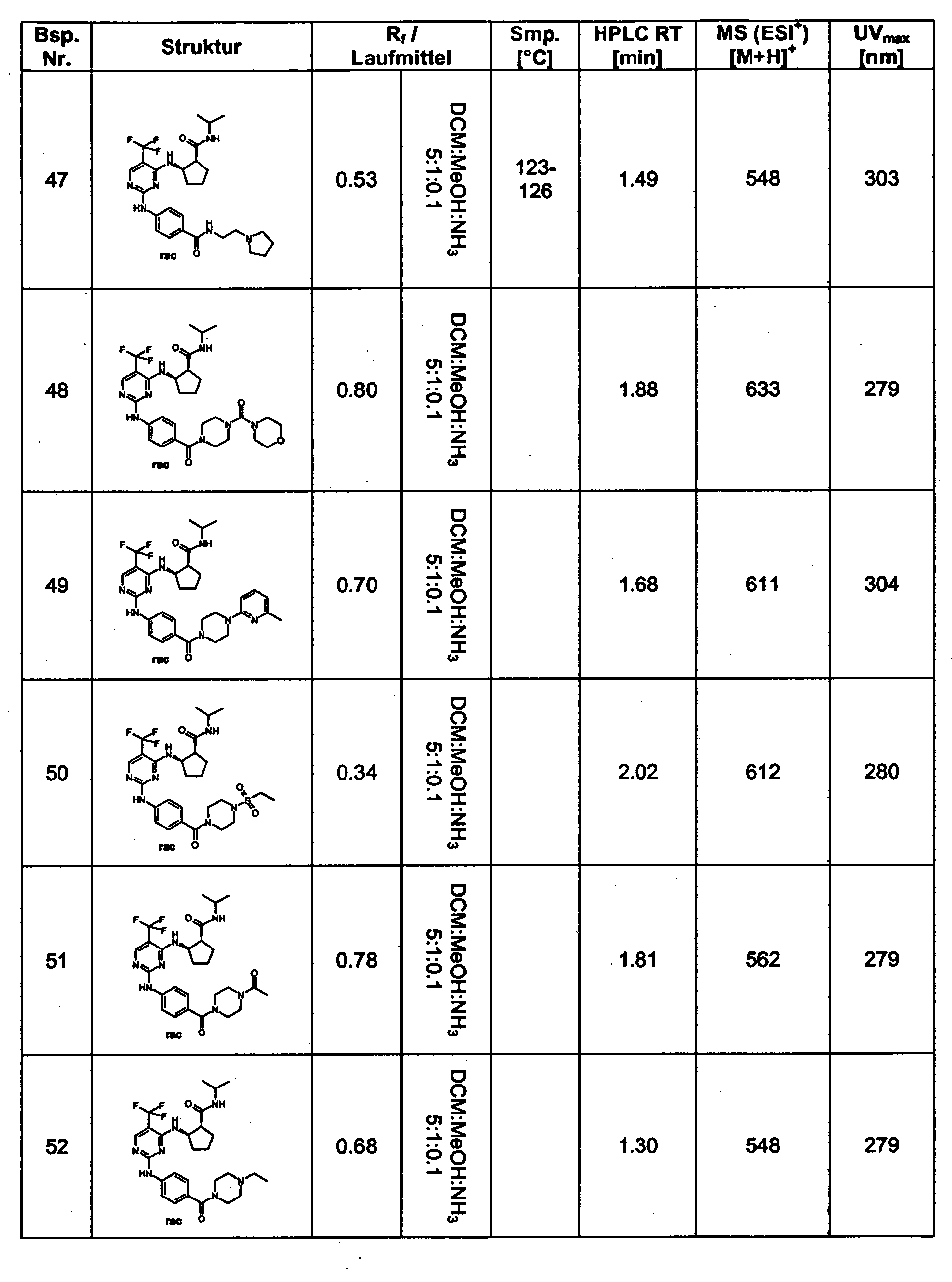

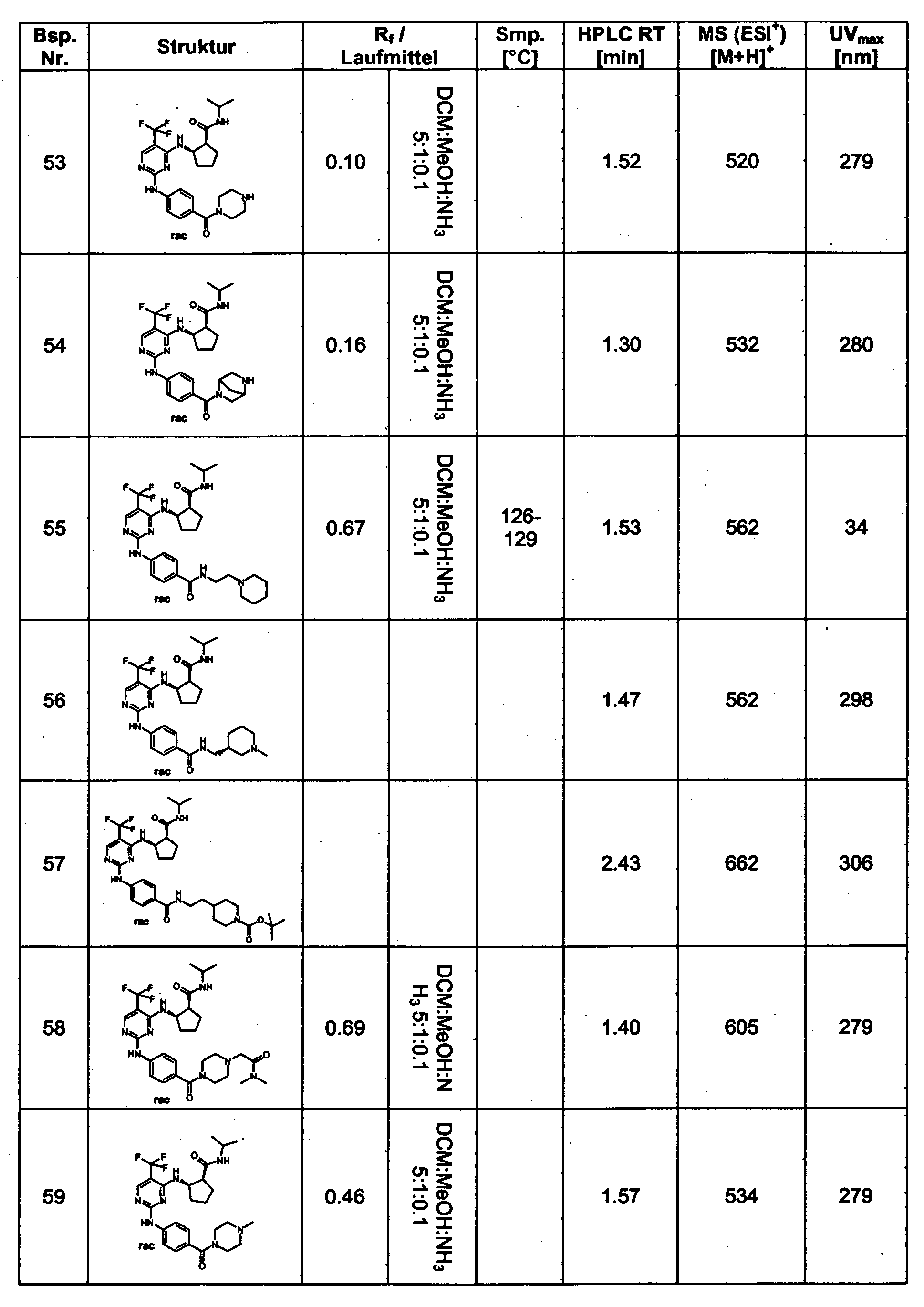

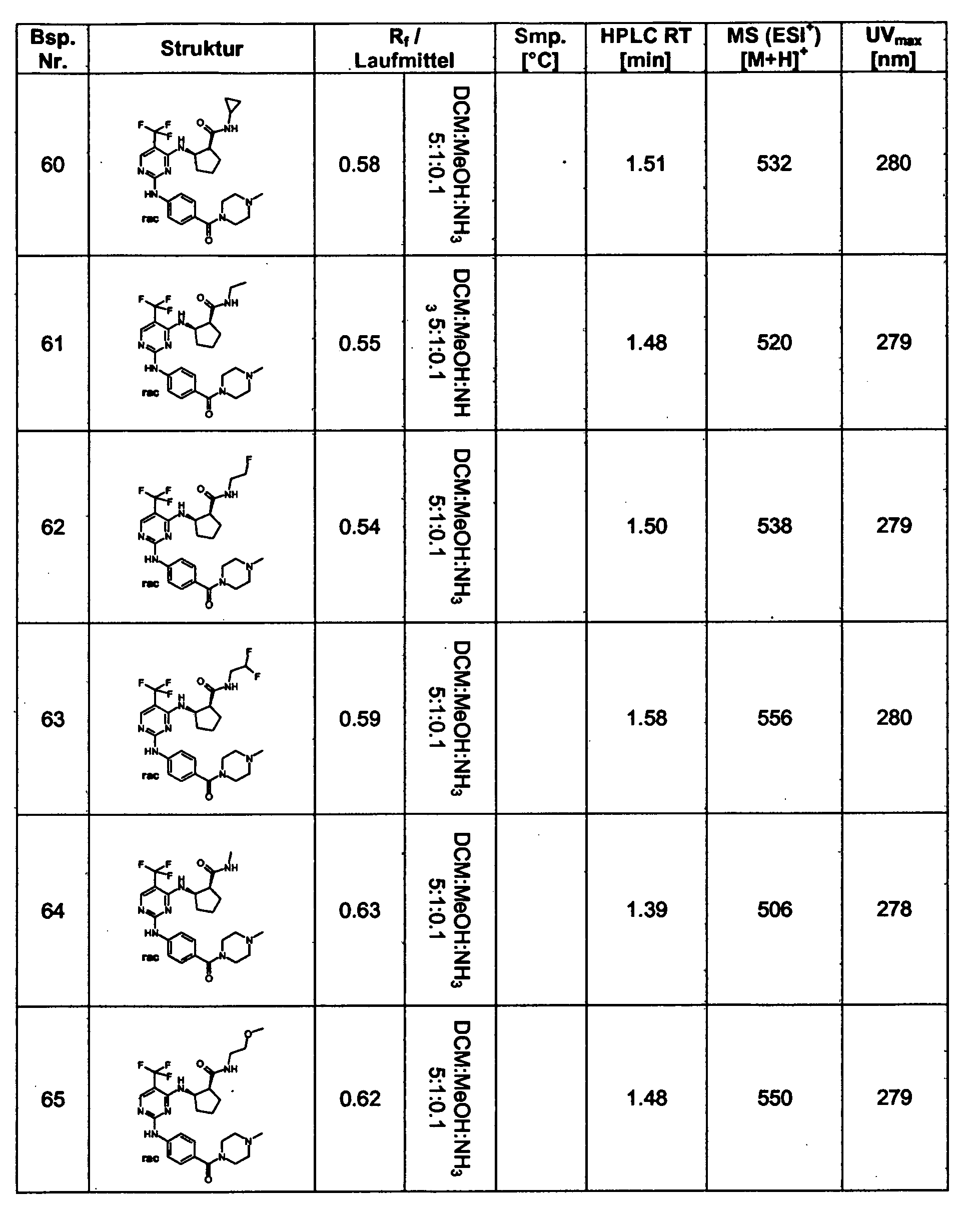

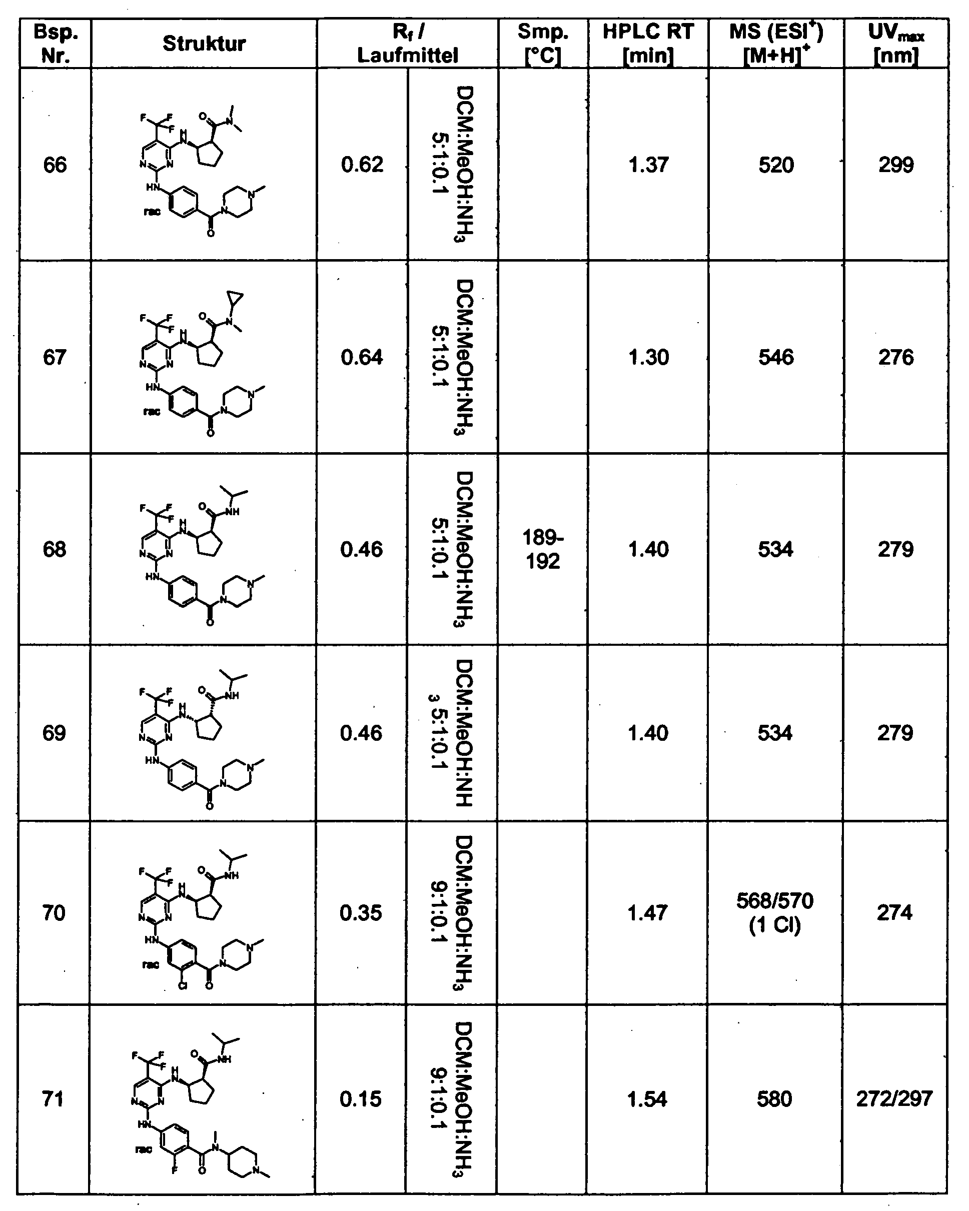

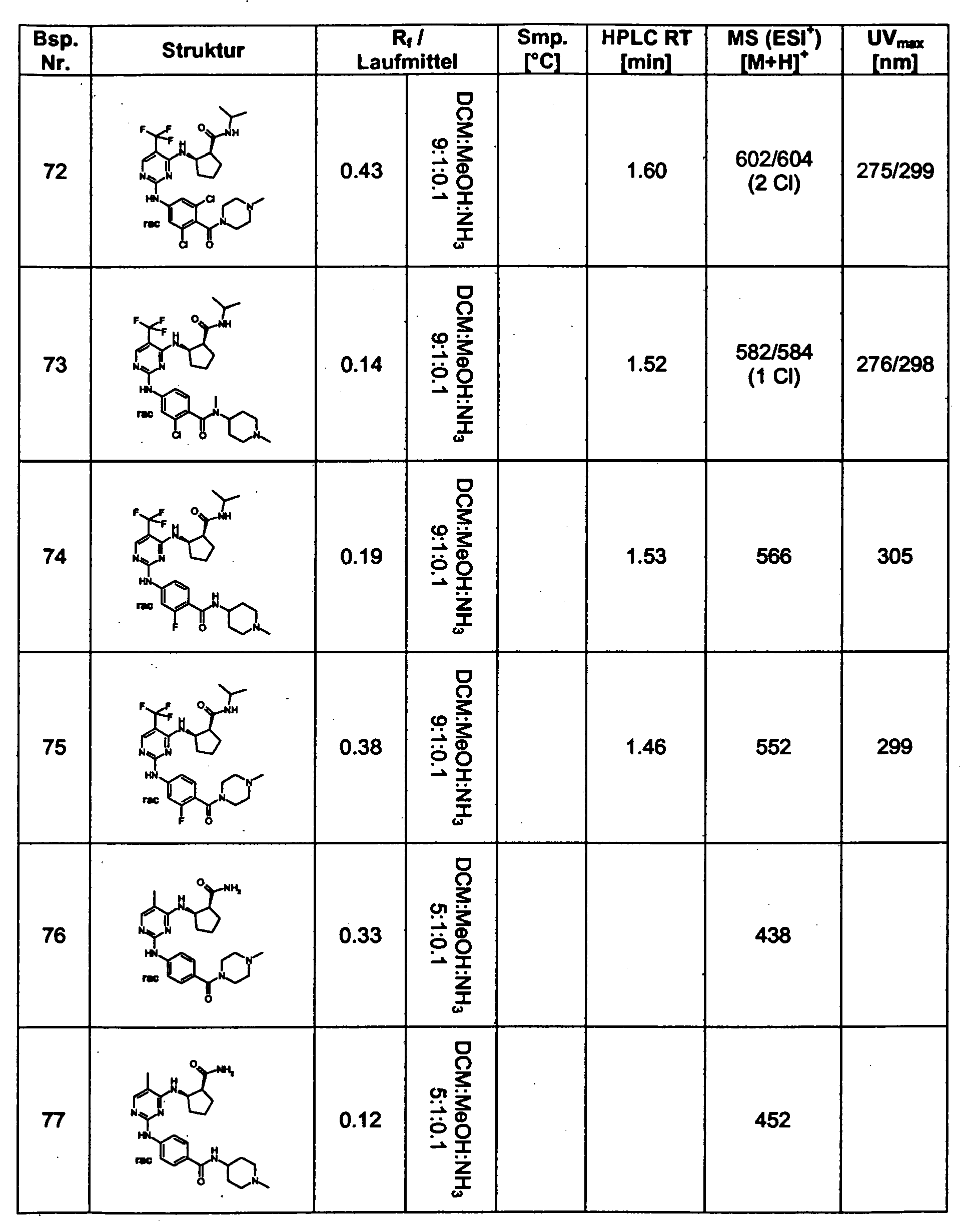

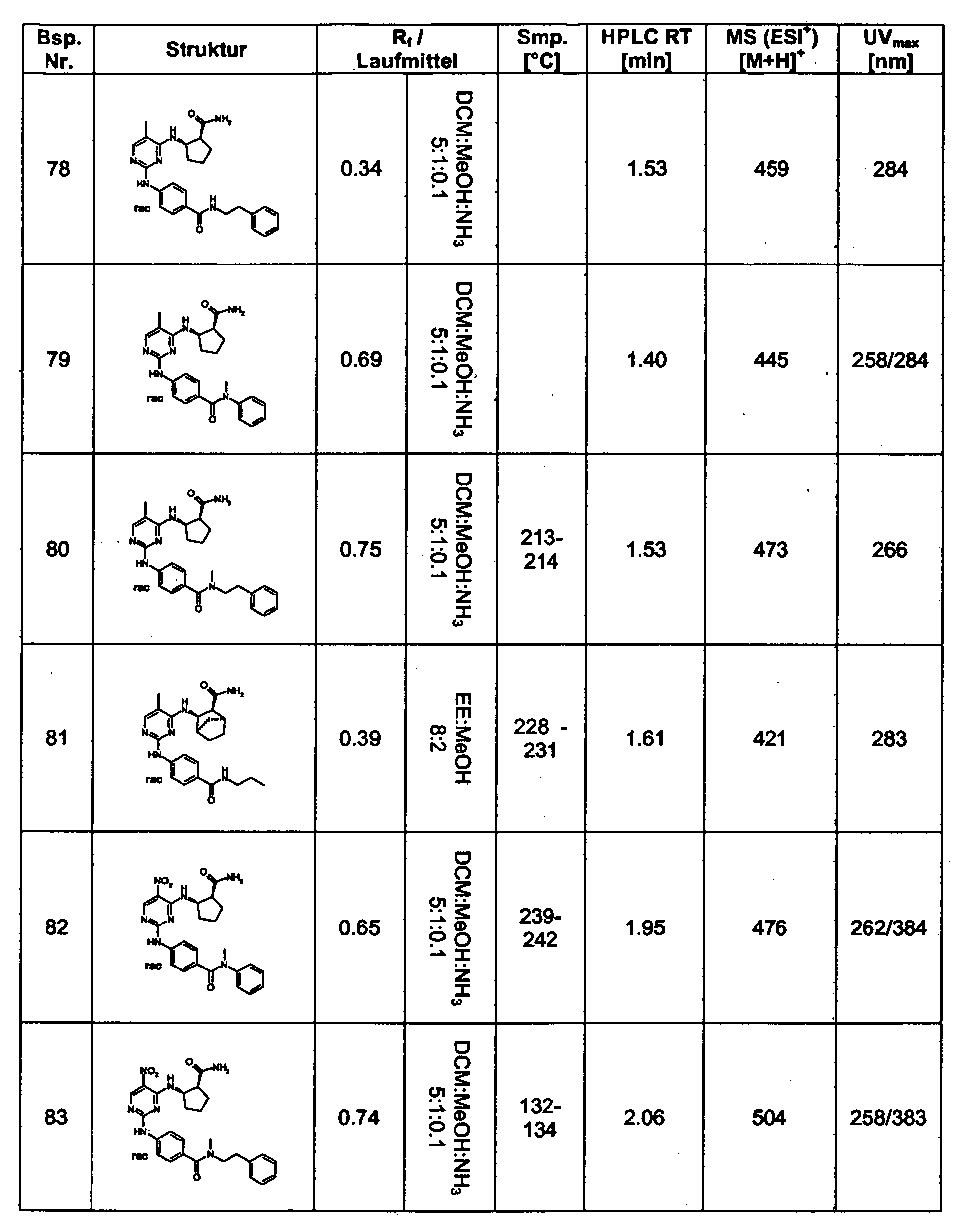

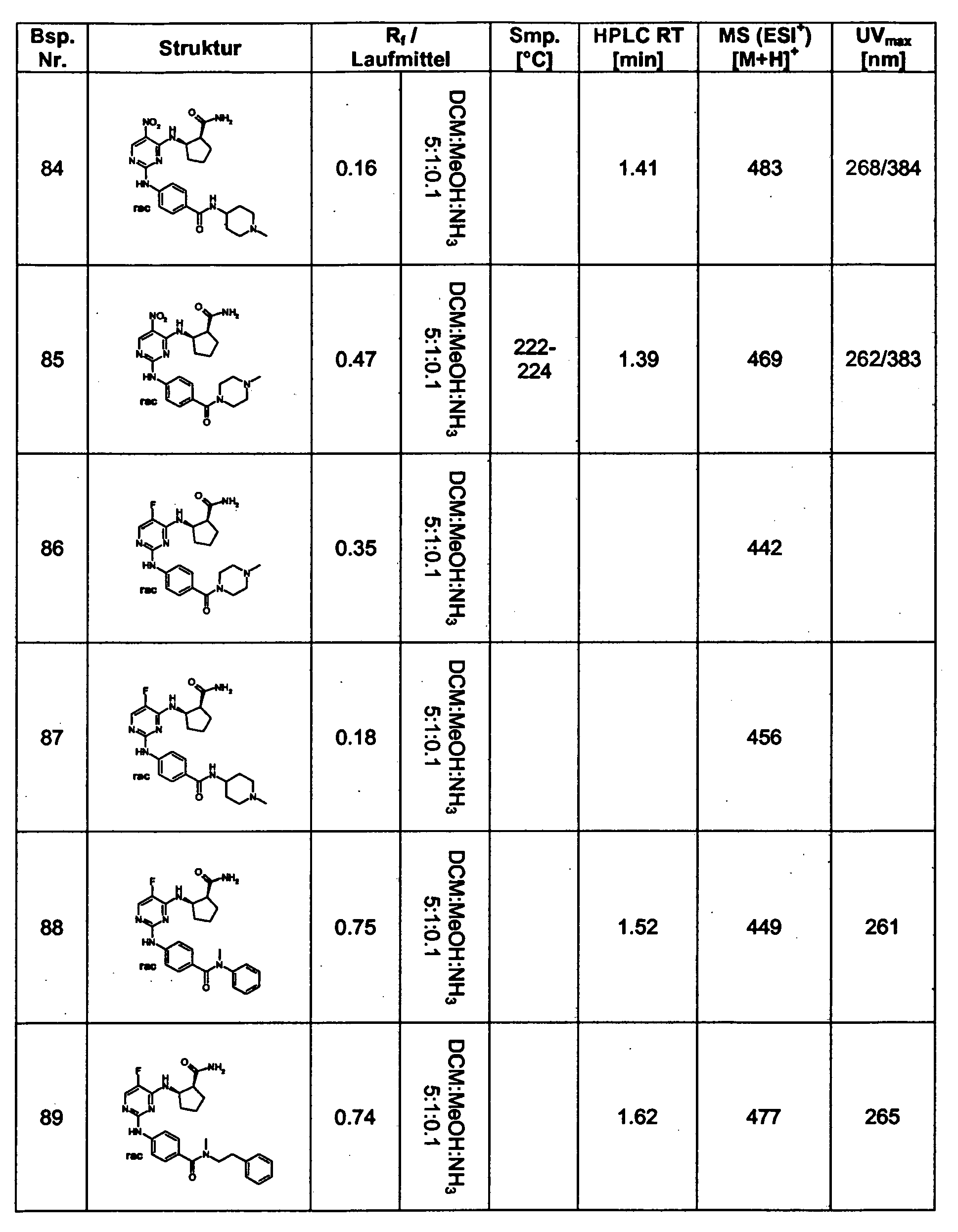

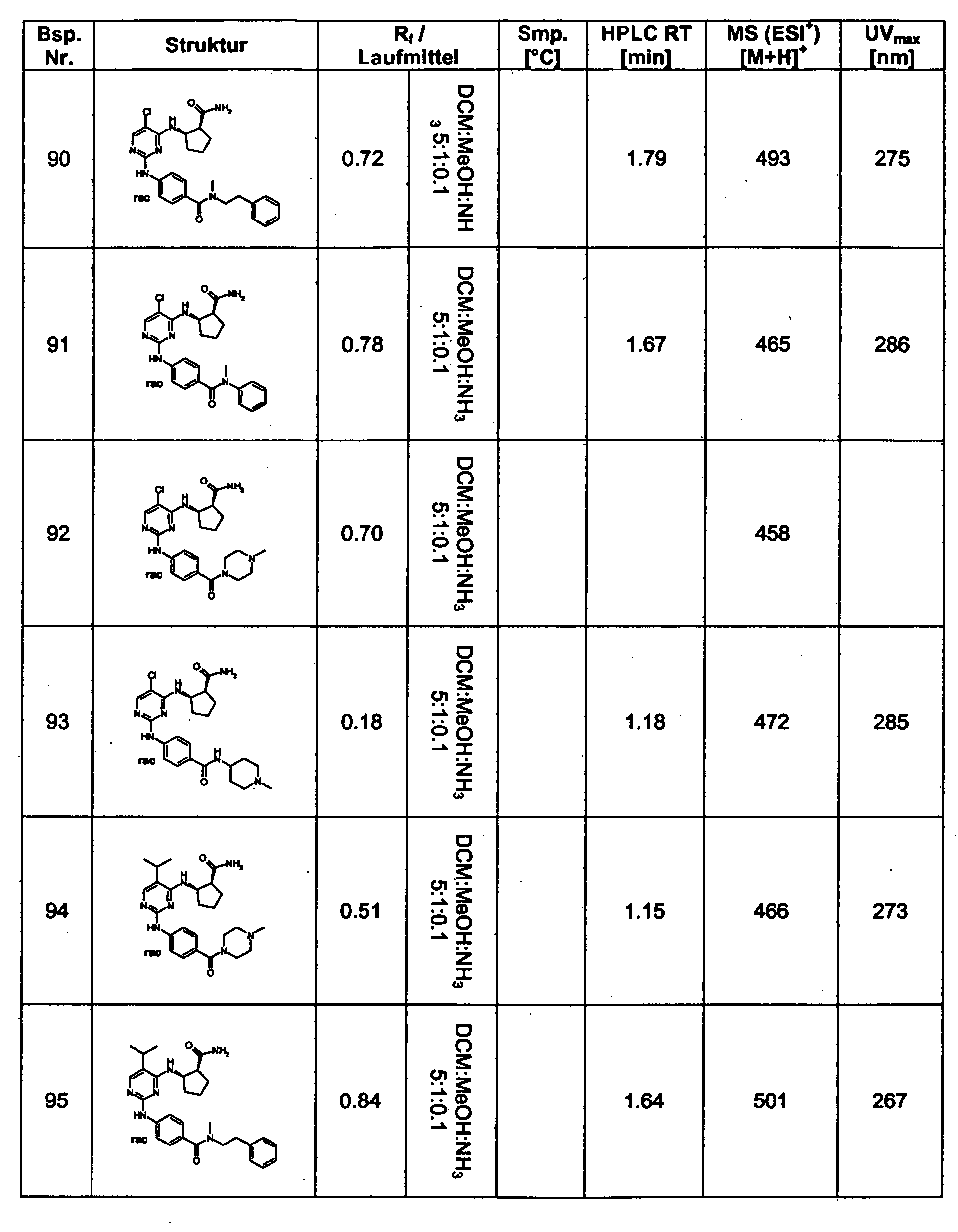

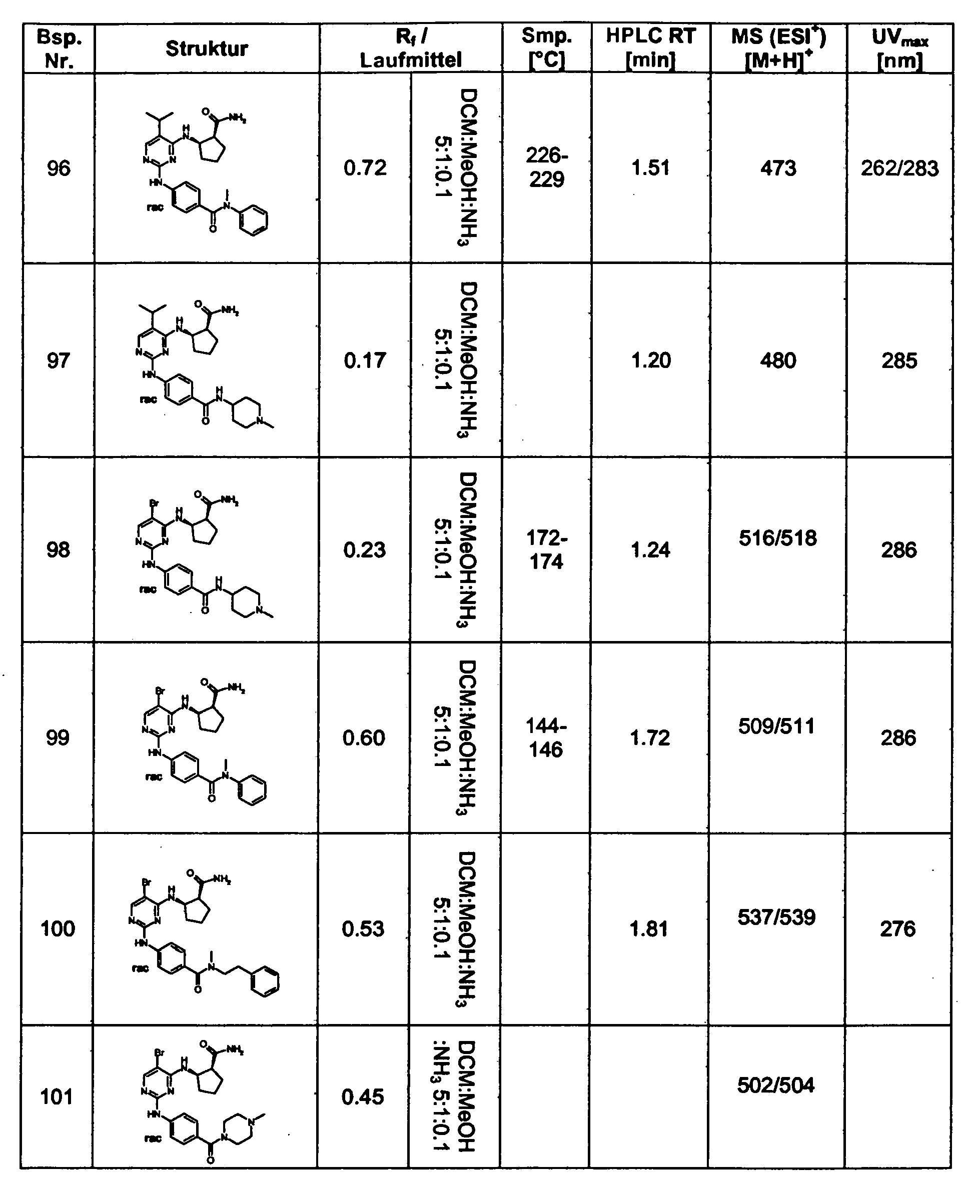

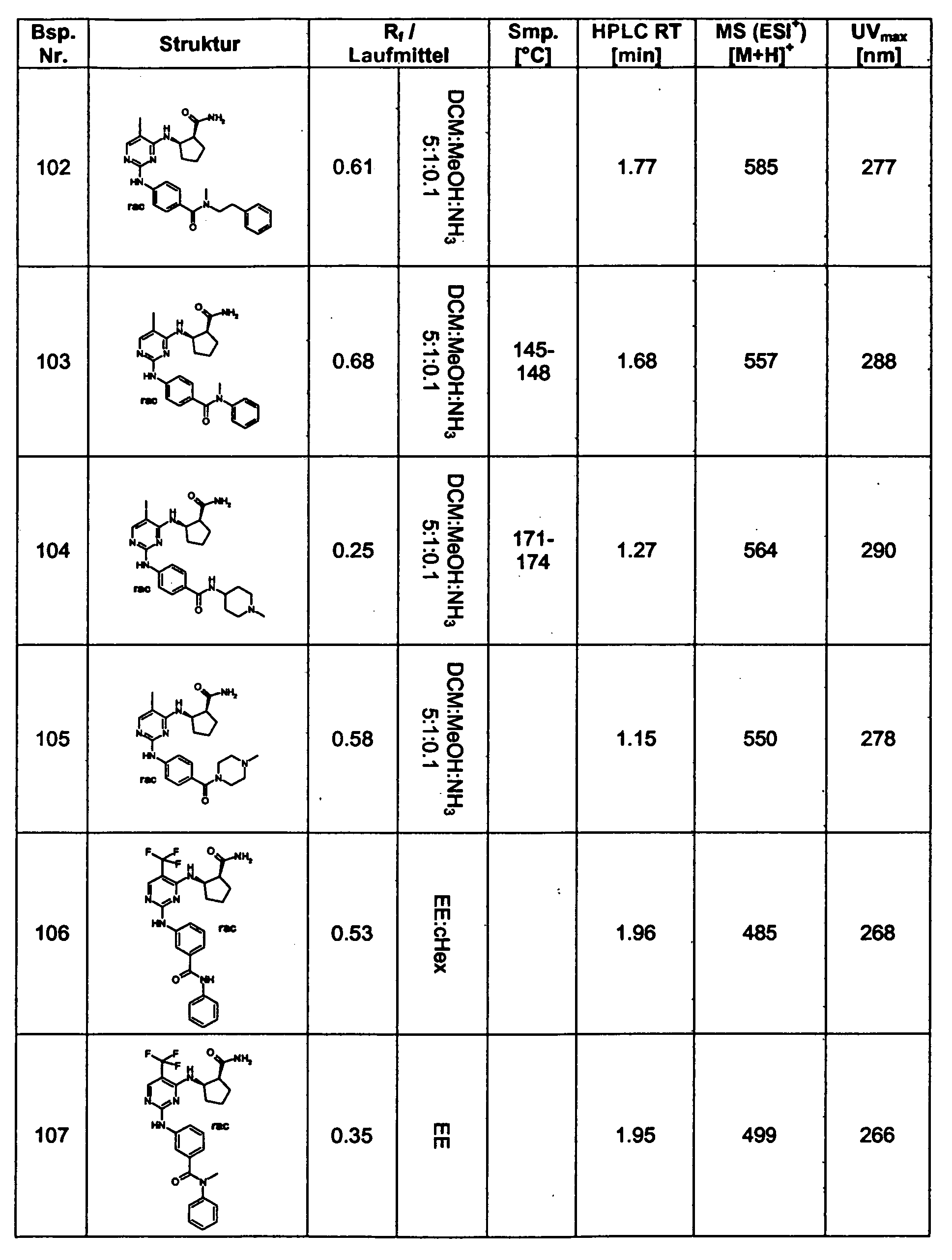

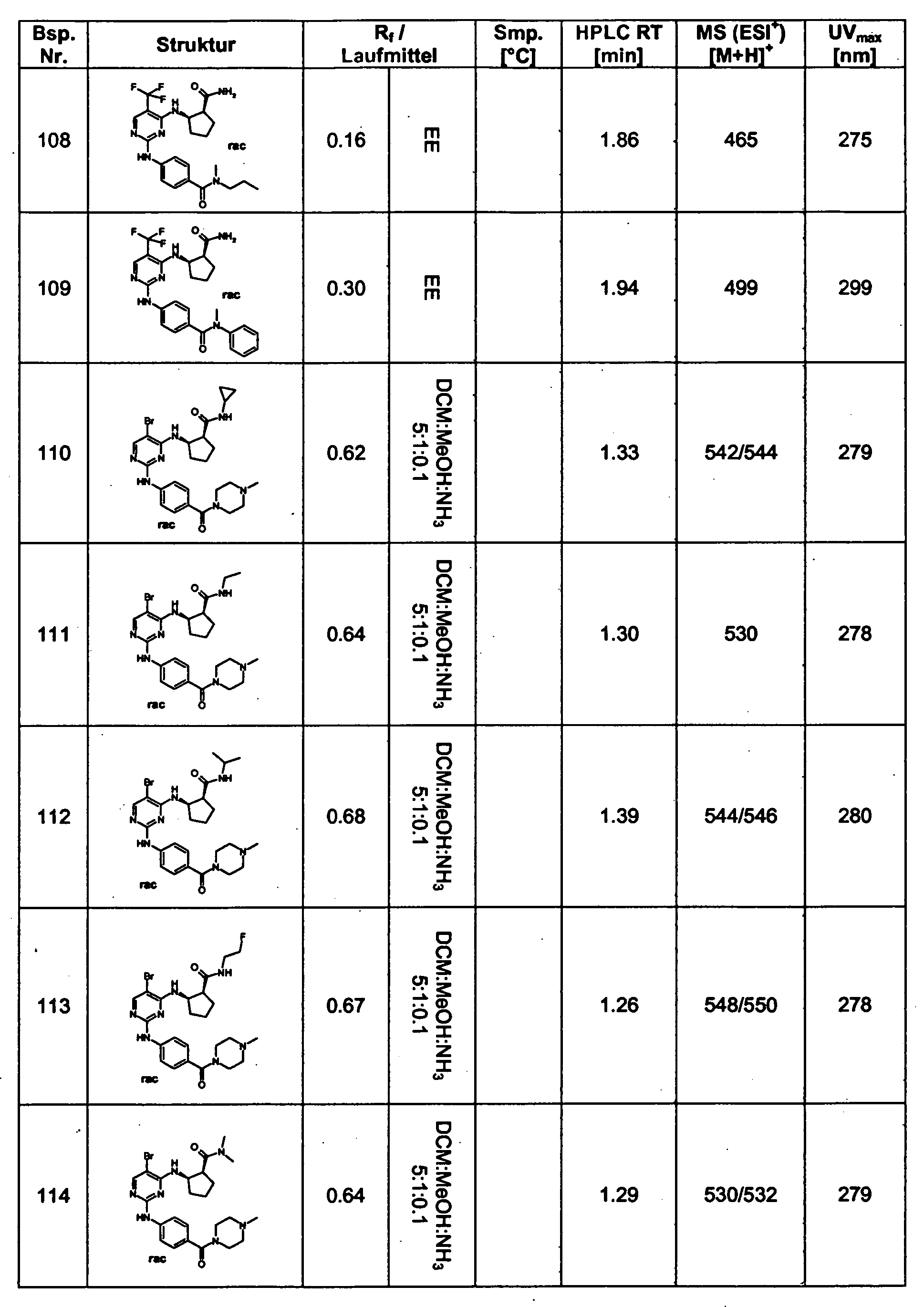

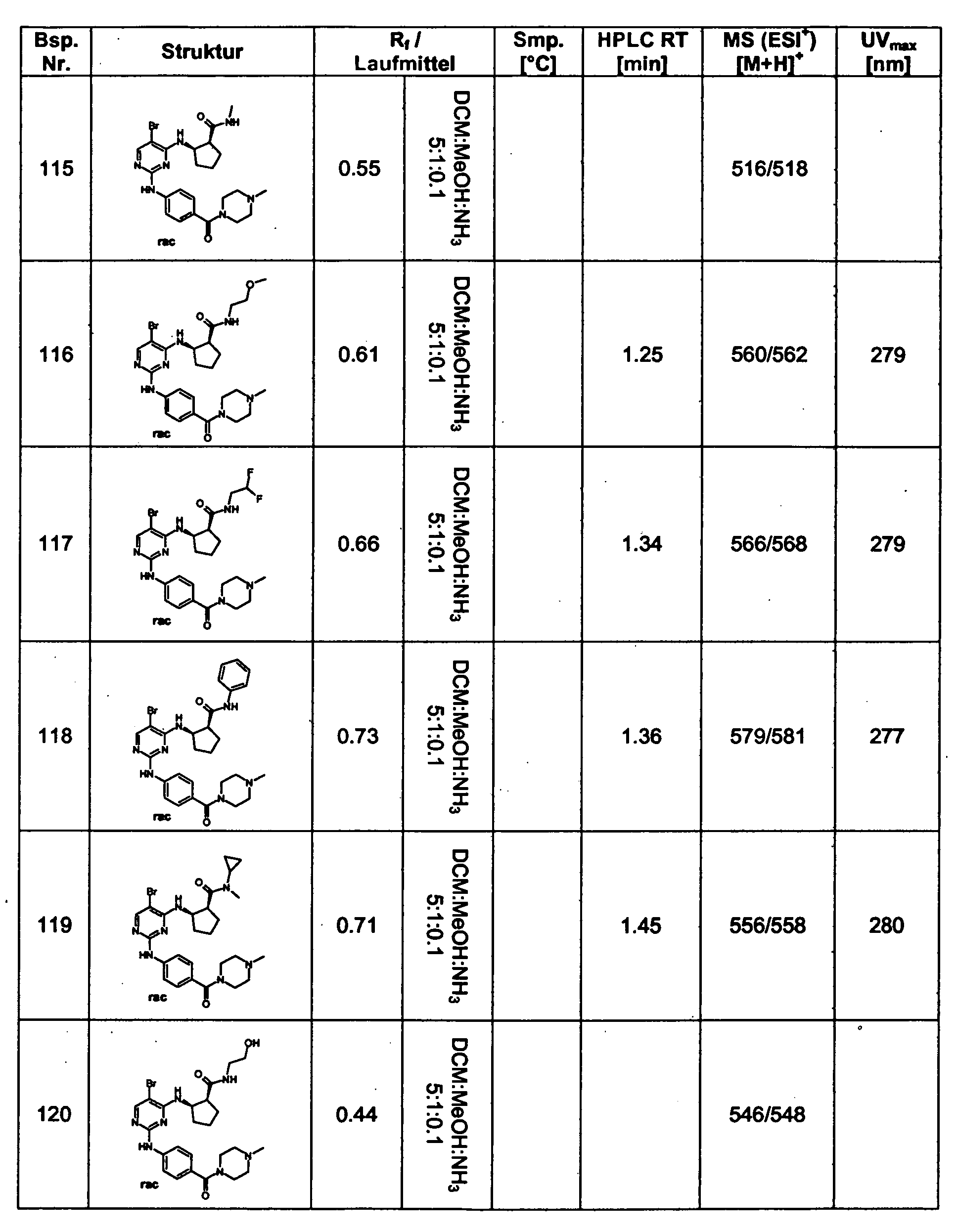

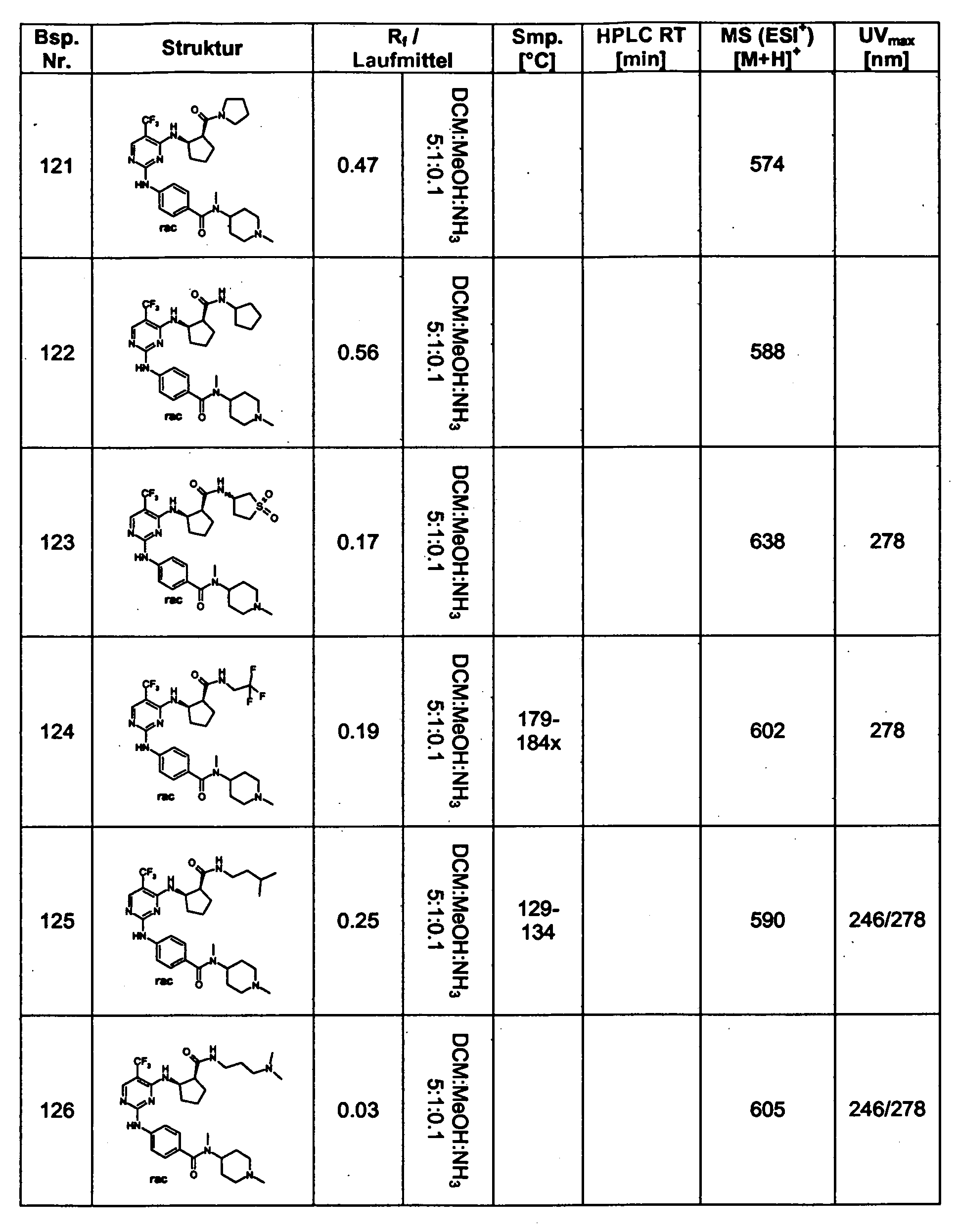

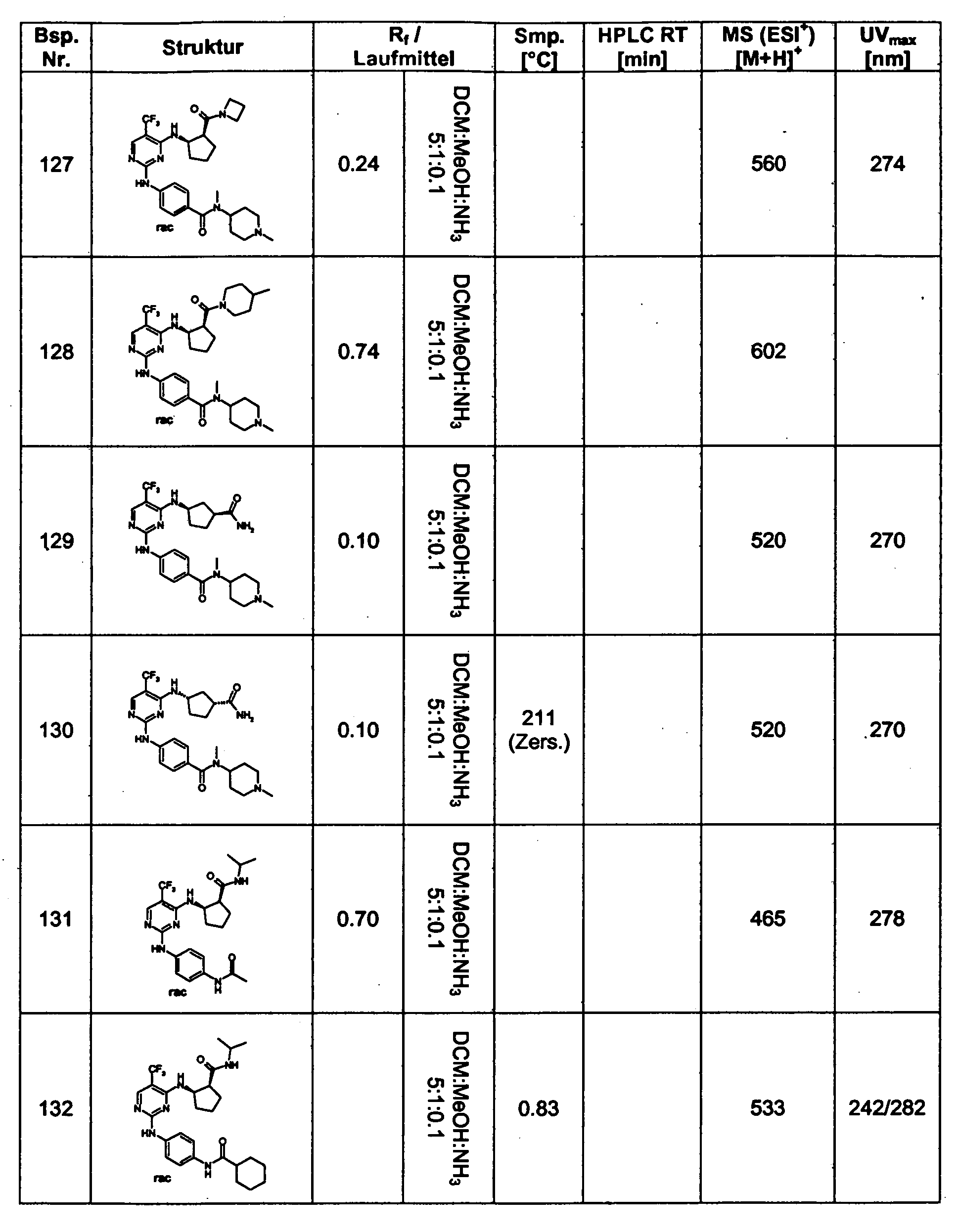

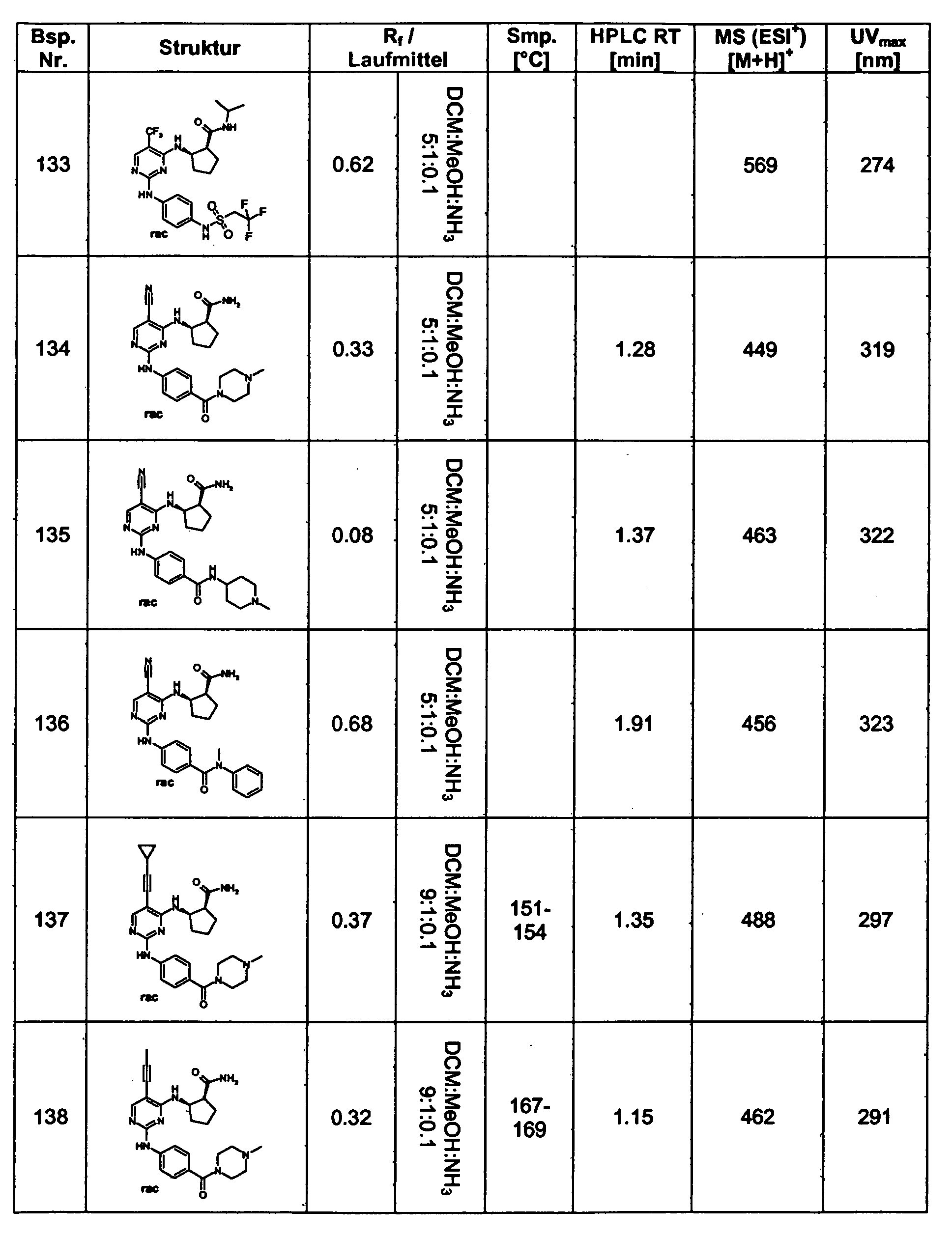

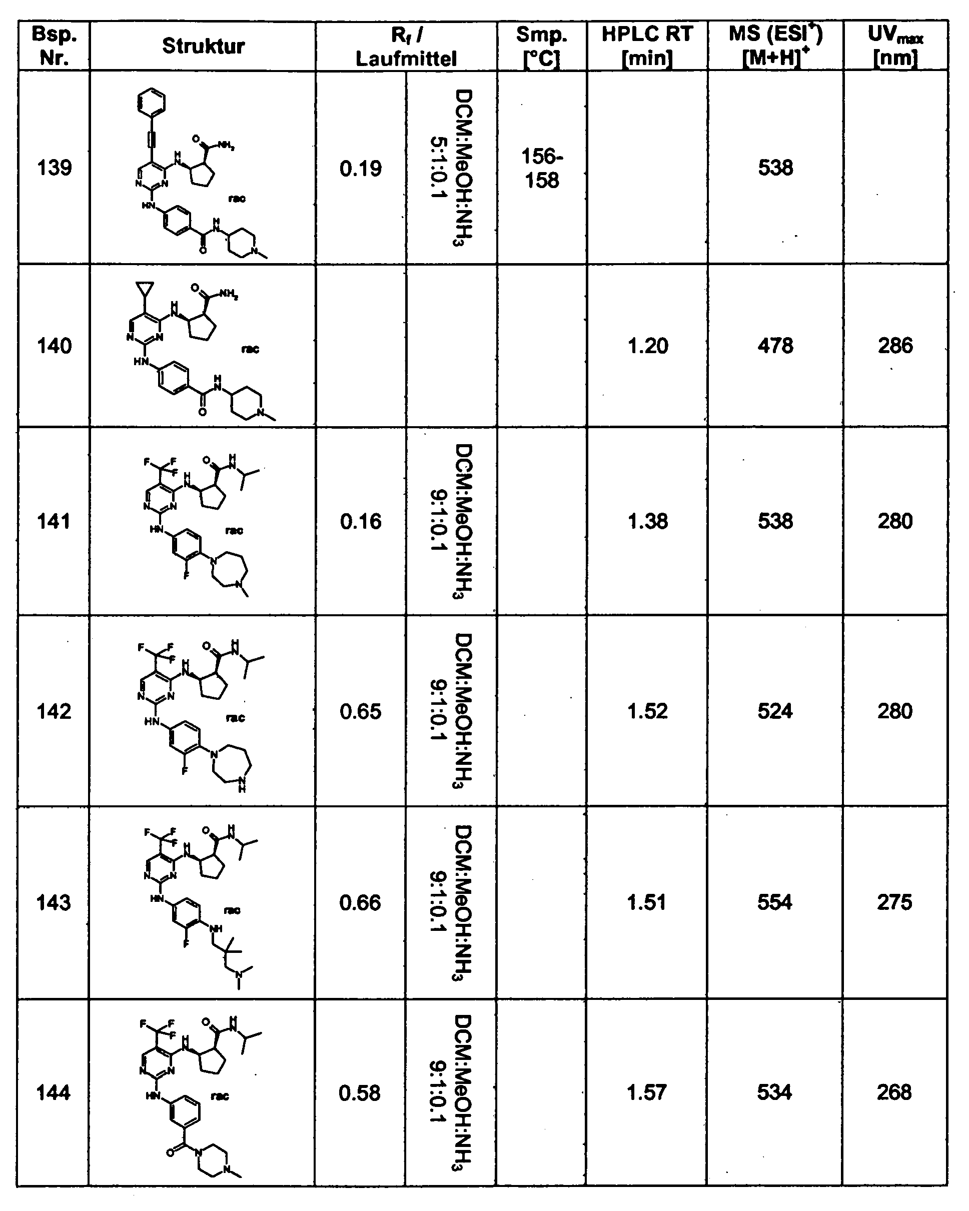

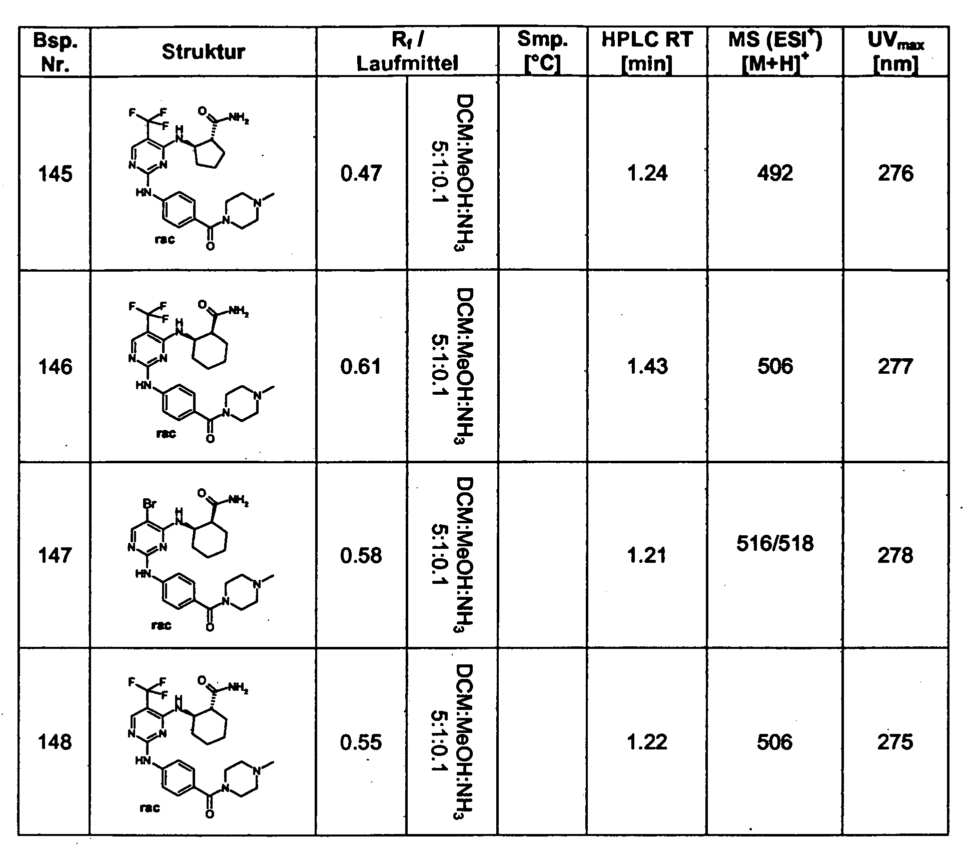

Die nachfolgenden Beispiele beschreiben die biologische Wirkung der erfindungsgemäßen Verbindungen ohne die Erfindung auf diese Beispiele einzuschränken.

Wie durch DNA-Färbung mit darauf folgender FACS oder Cellomics Array Scan Analysen gezeigt werden konnte, ist die durch die erfindungsgemäßen Verbindungen bewirkte Proliferationsinhibition vor allem durch Fehler in der Chromosomensegregation vermittelt. Wegen der Anhäufung von Fehlsegregationen kommt es zu einer massiven Polyploidie, die letztlich in einer Proliferationshemmung oder sogar Apoptose münden kann. Auf Grund ihrer biologischen Eigenschaften eignen sich die erfindungsgemäßen Verbindungen der allgemeinen Formel (1), deren Isomere und deren physiologisch verträgliche Salze sowie Polymorphe zur Behandlung von Erkrankungen, die durch exzessive oder abnormale Zellproliferation charakterisiert sind.

### Beispiel Aurora-B Kinaseassay

Es wurde ein radioaktiver Enyzmhemmungs-Assay entwickelt, der Baculovirusexprimiertes rekombinantes N-terminal mit einem Histidine(6)-Epitop (His-) ausgestattetes humanes Aurora B Wildtyp Protein verwendet, das aus infizierten Insekten Zellen (SF21) gewonnen und gereinigt wird.

### Expression und Reinigung

Hierzu werden beispielsweise 300x10⁶ SF21 Zellen in SF-900II Insektenzell-Medium (Invitrogen) mit einer passenden Menge Bakuloviruslösung für 1 h bei 27 °C inkubiert (Fernbachkolben-Schüttler, 50 rpm). Danach werden 250ml SF-900 II Medium zugegeben und für 3 Tage geschüttelt (100 rpm, 27 °C). Drei Stunden vor der Ernte wird der Kultur Okadainsäure (C₄₄H₆₈O₁₃, Calbiochem #495604) zugesetzt (Endkonzentration 0.1 µM) um Phosphorylierungsstellen auf rekombinantem Aurora B zu stabilisieren. Die Zellen werden durch Zentrifugation (1000 rpm, 5 min, 4 °C) pelletiert, der Überstand verworfen und das Pellet in flüssigem Stickstoff gefroren. Das Pellet wird aufgetaut (37 °C, 5 min) und in Lysispuffer resuspendiert. Für 200 mL Ausgangskulturvolumen werden 40 mL Lysispuffer (25 mM Tris/Cl, 10 mM MgCl₂, 300 mM NaCl, 20 mM Imidazol, pH 8.0, 0.07% 2-Mercaptoethanol und Protease-Inhibitor-Complete von Roche Diagnostics) verwendet Nach zwei zügigen Gefrier/Auftauzyklen (Flüssigstickstoff zu 37 °C), wird das Lysat für 30 min auf Eis gehalten, danach mit gewaschenen Ni-NTA Kügelchen (Ni-NTA Superflow Beads, 4 mL pro 200 mL Ausgangskultur) inkubiert (2 h, 4°C) und in eine Econo-Pac Säule (Biorad #732-1010) gefüllt. Fünf Waschungen mit jeweils 10 Säulenvolumen Waschpuffer (25 mM Tris/Cl, 10 mM MgCl₂, 1000 mM NaCl, 20 mM Imidazol, pH 8.0, 0.07% 2-Mercaptoethanol und Protease-Inhibitor-Complete von Roche Diagnostics) gehen der Elution in 8ml (pro 200ml Ausgangskultur) Elutionspuffer (25 mM Tris/Cl pH 8.0, 300 mM NaCl, 10 mM MgC12,0.03% Brij-35, 10% Glycerol , 0.07% 2-Mercaptoethanol, 400 mM Imidazol) voraus. Die vereinigten Eluatfraktionen werden mittels einer Sephadex G25-Säule entsalzt und in Einfrierpuffer (50 mM Tris/Cl pH 8.0, 150 mM NaCI, 0.1 mM EDTA, 0.03% Brij-35, 10% Glycerol, 1 mM DTT) überführt.

### Kinase-Assay

Testsubstanzen werden in eine Polypropylenplatte (96 Vertiefungen, Greiner #655 201) vorgelegt, um einen Konzentrationsrahmen von 10 µM - 0.0001 µM abzudecken. Die Endkonzentration von DMSO im Assay ist 5%. Zu vorgelegten 10 µl Testsubstanz in 25% UMSO werden 30 µL Protein-Mix (50 mM Tris/Cl pH 7.5,25 mM MgCl₂, 25 mM NaCl, 167 µM ATP, 200 ng His-Aurora B in Einfrierpuffer) pipettiert und 15 min bei RT inkubiert. Danach werden 10 µL Peptid-Mix (100 mM Tris/Cl pH 7.5, 50 mM MgCl₂, 50 mM NaCl, 5 µM NaF, 5 µM DTT, 1 µCi gamma-P33-ATP [Amersham], 50 µM Substratpeptid [Biotin-EPLERRLSLVPDS oder Multimere davon, oder Biotin-EPLERRLSLVPKM oder Multimere davon, bzw. Biotin-LRRWSLGLRRWSLGLRRWSLGLRRWSLG]) zugegeben. Die Reaktion wird 75 min inkubiert (Raumtemperatur) und durch Zugabe von 180 µL 6.4% Trichloressigsäure gestoppt und für 20 min auf Eis inkubiert. Eine Multiscreen Filtrationsplatte (Millipore, MAIP NOB10) wird mit zunächst 100 µL 70% Ethanol und danach mit 180 µL Trichloressigsäure äquilibriert und die Flüssigkeiten mit einem geeigneten Absauggerät entfernt. Danach wird die gestoppte Kinase-Reaktion aufgebracht. Nach 5 Waschschritten mit jeweils 180 µL 1% Trichloressigsäure wird der untere Teil der Platte getrocknet (10-20 min bei 55 °C) und 25 µL Scintillations-Cocktail (Microscint, Packard # 6013611) zugegeben. Inkorporiertes gamma-Phosphat wird mithilfe eines Wallac 1450 Microbeta Flüssigscintillations Zählers quantifiziert. Proben ohne Testsubstanz oder ohne Substratpeptid dienen als Kontrollen. IC₅₀ Werte werden mittels Graph Pad Prism Software ermittelt.

Die anti-proliferative Wirkung der erfindungsgemäßen Verbindungen wird im Proliferationstest an kultivierten humanen Tumorzellen und/oder in einer Zell Zyklus Analyse, beispielsweise an NCI-H460 Tumorzellen, bestimmt. Die Verbindungen zeigen in beiden Testmethoden eine gute bis sehr gute Wirksamkeit, d.h. beispielsweise einen EC₅₀-Wert im NCI-H460-Proliferationstest kleiner 5 µmol/L, in der Regel kleiner 1 µmol/L.

### Messung der Inhibierung der Proliferation an kultivierten humanen Tumorzellen

Zur Messung der Proliferation an kultivierten humanen Tumorzellen werden Zellen der Lungen Tumorzell-Linie NCI-H460 (erhalten von American Type Culture Collection (ATCC)) in RPMI 1640 Medium (Gibco) und 10% fötalem Rinderserum (Gibco) kultiviert und in der log-Wachstumsphase geerntet. Anschließend werden die NCI-H460 Zellen in 96-well Flachbodenplatten (Falcon) mit einer Dichte von 1000 Zellen pro well in RPMI 1640 Medium eingebracht und über Nacht in einem Inkubator (bei 37°C und 5 % CO₂) inkubiert. Die Wirksubstanzen werden in verschiedenen Konzentrationen (gelöst in DMSO; DMSO-Endkonzentration: 0.1%) zu den Zellen zugegeben. Nach 72 h Inkubation werden zu jedem well 20 µl AlamarBlue Reagenz (AccuMed International) zugesetzt, und die Zellen für weitere 5-7 h inkubiert. Nach Inkubation wird der Farbumsatz des . AlamarBlue Reagenz in einem Wallac Microbeta Fluoreszenzspektrophotometer bestimmt. Ec₅₀ Werte werden mittels Standard Levenburg Marquard Algorithmen (GraphPadPrizm) kalkuliert.

Zellzyklusanalysen werden beispielsweise mittels FACS Analysen (Fluorescence Activated Cell Sorter) oder mittels Cellomics Array Scan (CellCycle Analysis) durchgeführt

### FACS - Analyse

Propidium Iodid (PI) bindet stöchiometrisch an doppelsträngige DNA, und ist damit geeignet den Anteil an Zellen in der G1, S, und G2/M Phase des Zellzykluses auf der Basis des zellulären DNA Gehaltes zu bestimmen. Zellen in der G0 und G1 Phase haben einen diploiden DNA Gehalt (2N), während Zellen in der G2 oder Mitose einen 4N DNA Gehalt haben.

Für eine PI-Färbung werden beispielsweise 1,75x10⁶ NCI-H460 Zellen auf eine 75 cm² Zellkulturflasche ausgesät, nach 24 h wird entweder 0.1 % DMSO als Kontrolle zugesetzt, bzw. die Substanz in verschiedenen Konzentrationen (in 0.1 % DMSO). Die Zellen werden für 42 h mit der Substanz bzw. mit DMSO inkubiert. Dann werden die Zellen mit Trypsin abgelöst und zentrifugiert. Das Zellpellet wird mit gepufferter Kochsalzlösung (PBS) gewaschen und die Zellen anschließend mit 80% Ethanol bei -20 °C für mindestens 2 h fixiert. Nach einem weiteren Waschschritt mit PBS werden die Zellen mit Triton-X100 (Sigma; 0,25% in PBS) für 5 min auf Eis permeabilisiert und anschließen mit einer Lösung aus Propidium Iodid (Sigma; 10µg/ml)und RNAse (Serva; 1mg/mL1)im Verhältnis 9:1 für mindestens 20 min im Dunkeln inkubiert. Die DNA Messung erfolgt in einem Becton Dickinson FACS Analyzer, mit einem Argonlaser (500 mW, Emission 488 nm), Datenerhebung und Datenauswertung erfolgt mit dem DNA Cell Quest Programm (BD).

### Cellomics Array Scan

NCI-H460 Zellen werden in 96-well Flachbodenplatten (Falcon) in RPMI 1640 Medium (Gibco) mit 10% fötalem Rinderserum (Gibco) in einer Dichte von 2000 Zellen pro well ausgesät und über Nacht in einem Inkubator (bei 37 °C und 5 % CO₂) inkubiert. Die Wirksubstanzen werden in verschiedenen Konzentrationen (gelöst in DMSO; DMSO-Endkonzentration: 0.1 %) zu den Zellen zugegeben. Nach 42 h Inkubation wird das Medium abgesaugt, die Zellen 10 min mit 4% Formaldehydlösung und Triton X-100 (1:200 in PBS) bei Raumtemperatur fixiert und gleichzeitig permeabilisiert, und anschließend zweimal mit einer 0.3% BSA Lösung (Calbiochem) gewaschen. Anschließend erfolgt die Färbung der DNA durch Zugabe von 50 µL/well 4',6-Diamidino-2-phenylindole (DAPI; Molecular Probes) in einer Endkonzentration von 300 nM für 1 h bei Raumtemperatur, im Dunkeln. Danach wird zweimal sorgfältig mit PBS gewaschen, die Platten mit schwarzer Klebefolie abgeklebt und im Cellomics ArrayScan mittels CellCycle BioApplication Programm analysiert und via Spotfire visualisiert und ausgewertet.

Die Substanzen der vorliegenden Erfindung sind Aurora Kinaseinhibitoren. Auf Grund ihrer biologischen Eigenschaften eignen sich die neuen Verbindungen der allgemeinen Formel (1), deren Isomere und deren physiologisch verträgliche Salze zur Behandlung von Erkrankungen, die durch exzessive oder anomale Zellproliferation charakterisiert sind.

Zu solchen Erkrankungen gehören beispielsweise: Virale Infektionen (z.B. HIV und Kaposi Sarkoma), Entzündung und Autoimmunerkrankungen (z.B. Colitis, Arthritis, Alzheimer Erkrankung, Glomerulonephritis und Wundheilung), bakterielle, fungale und/oder parasitäre Infektionen, Leukämien, Lymphoma und solide Tumore (zB. Karzinome und Sarkome), Hauterkrankungen (z.B. Psoriasis), auf Hyperplasie beruhende Krankheiten die durch einen Anstieg der Zellzahl (z.B. Fibroblasten, Hepatozyten, Knochen und Knochenmarkzellen, Knorpel oder glatte Muskulaturzellen oder Epithelialzellen (z.B. endometrische Hyperplasie)) gekennzeichnet sind; Knochenerkrankungen und kardiovaskuläre Erkrankungen (z.B. Restenose und Hypertrophie).

Beispielsweise können folgende Krebserkrankungen mit erfindungsgemäßen Verbindungen behandelt werden, ohne jedoch darauf beschränkt zu sein: Hirntumore wie beispielsweise Akustikusneurinom, Astrozytome wie pilozytisches Astrozytome, fibrilläres Astrozytom, protoplasmatisches Astrozytom, gemistozytäres Astrozytom, anaplastisches Astrozytom und Glioblastome, Hirnlymphome, Hirnmetastasen, Hypophysentumor wie Prolaktinom, HGH (human growth hormon) produzierender Tumor und ACTH produzierender Tumor (adrenocorticotropes Hormon), Kraniopharyngiome, Medulloblastome, Meningeome und Oligodendrogliome; Nerventumore (Neoplasmen) wie zum Beispiel Tumore des vegetativen Nervensystems wie Neuroblastoma sympathicum, Ganglioneurom, Paragangliom (Phäochromozytom, Chromaffinom) und Glomuscaroticum-Tumor, Tumore am peripheren Nervensystem wie Amputationsneurom, Neurofibrom, Neurinom (Neurilemmom, Schwannom) und malignes Schwannom, sowie Tumore am zentralen Nervensystem als Hirn- und Rückenmarkstumoren; Darmkrebs wie zum Beispiel Rektumkarzinom, Kolonkarzinom, Analkarzinom, Dünndarmtumore und Zwölffingerdarmtumore; Lidtumore wie Basaliom oder Basalzellkarzinom; Bauchspeicheldrüsenkrebs oder Pankreaskarzinom; Blasenkrebs oder Blasenkarzinom; Lungenkrebs (Bronchialkarzinom) wie beispielsweise kleinzellige Bronchialkarzinome (Haferzellkarzinome) und nicht-kleinzellige Bronchialkarzinome wie Plattenepithelkarzinome, Adenokarzinome und großzellige Bronchialkarzinome; Brustkrebs wie zum Beispiel Mammakarzinom wie infiltrierend duktales Karzinom, Kolloidkarzinom, lobulär invasives Karzinom, tubuläres Karzinom, adenozystisches Karzinom und papilläres Karzinom; Non-Hodgkin-Lymphomen (NHL) wie beispielsweise Burkitt-Lymphom, niedrigmaligne Non-Hodgkin-Lymphomen (NHL) und Mucosis fungoides; Gebärmutterkrebs oder Endometriumkarzinom bzw. Corpuskarzinom; CUP-Syndrom (Cancer of Unknown Primary); Eierstockskrebs oder Ovarial-Karzinom wie mucinöse, endometriale oder seröse Krebs; Gallenblasenkrebs; Gallengangskrebs wie beispielsweise Klatskin-Tumor; Hodenkrebs wie zum Beispiel Seminome und Nicht-Seminome; Lymphom (Lymphosarkom) wie zum Beispiel malignes Lymphom, Morbus Hodgkin, Non-Hodgkin-Lymphomen (NHL) wie chronisch lymphatische Leukämie, Haarzell-Leukämie, Immunozytom, Plasmozytom (multiples Myelom), Immunoblastom, Burkitt-Lymphom, T-Zonen-Mycosis fungoides, großzellig-anaplastisches Lymphoblastom und Lymphoblastom; Kehlkopfkrebs wie zum Beispiel Stimmbandtumoren, supraglottisch, glottisch und subglottisch Kehlkopftumore; Knochenkrebs wie zum Beispiel Osteochondrom, Chondrom, Chondroblastom, Chondromyxoidfibrom, Osteom, Osteoid-Osteom, Osteoblastom, eosinophiles Granulom, Riesenzell-Tumor, Chondrosarkom, Osteosarkom, Ewing-Sarkom, Retikulo-Sarkom, Plasmozytom, Riesenzell-Tumor, fibröse Dysplasie, juvenile Knochenzyste und aneurysmatische Knochenzyste; Kopf Hals-Tumoren wie zum Beispiel Tumoren der Lippen, Zunge, Mundboden, Mundhöhle Zahnfleisch, Gaumen, Speicheldrüsen, Rachen, Nasenhöhlen, Nasennebenhöhlen, Kehlkopf und Mittelohr; Leberkrebs wie zum Beispiel das Leberzellkarzinom oder hepatozelluläres Karzinom (HCC); Leukämien wie zum Beispiel akute Leukämien wie akute lymphatische/lymphoblastische Leukämie (ALL), akute myeloische Leukämie (AML); chronische Leukämien wie chronisch lymphatische Leukämie (CLL), chronisch myeloische Leukämie (CML); Magenkrebs oder Magenkarzinom wie zum Beispiel papilläres, tubuläres und muzinöses Adenokarzinom, Siegelringzellkarzinom adenosquamöses Karzinom, kleinzelliges Karzinom und undifferenziertes Karzinom; Melanome wie zum Beispiel oberflächlich spreitendes, knotiges, lenitgo-maligna und akral-lentiginöse Melanom; Nierenkrebs wie zum Beispiel Nierenzellkarzinom oder Hypernephrom oder Grawitz-Tumor; Speiseröhrenkrebs oder Ösophaguskarzinom; Peniskrebs; Prostatakrebs; Rachenkrebs oder Pharynxkarziriome wie zum Beispiel Nasopharynxkarzinome, Oropharynxkarzinome und Hypopharynxkarainome; Retinoblastom wie zum Beispiel Scheidenkrebs oder Vaginalkarzinom; Plattenepithelkarzinome, Adenokarzinome, in situ Karzinome, maligne Melanome und Sarkome; Schilddrüsen-Karzinome wie zum Beispiel papilläre, follikuläre und medulläre Schilddrüsen-Karzinom, sowie anaplastische Karzinome; Spinaliom, Stachelzellkarzinom und Plattenepithelkarzinom der Haut; Thymome, Urethrakrebs und Vulvakrebs.

Die neuen Verbindungen können zur Prävention, Kurz- oder Langzeitbehandlung der vorstehend erwähnten Krankheiten gegebenenfalls auch in Kombination mit Radiotherapie oder mit anderen "State-of-art" Verbindungen, wie z.B. zytostatische oder zytotoxische Substanzen, Zellproliferationshemmer, anti-angiogene Substanzen, Steroide oder Antikörper, verwendet werden.

Die Verbindungen der allgemeinen Formel (1) können allein oder in Kombination mit anderen erfindungsgemäßen Wirkstoffen, gegebenenfalls auch in Kombination mit weiteren pharmakologisch aktiven Wirkstoffen, zur Anwendung gelangen.

Chemotherapeutica welche in Kombination mit den erfindungsgemäßen Verbindungen verabreicht werden können, umfassen ohne darauf eingeschränkt zu sein, Hormone, Hormonanaloge und Antihormone (z.B. Tamoxifen, Toremifen, Raloxifen, Fulvestrant, Megestrol Acetat, Flutamid, Nilutamid, Bicalutamid, Aminoglutethimid, Cyproteron Acetat, Finasterid, Buserelin Acetat, Fludrocortinson, Fluoxymesteron, Medroxyprogesteron, Octreotid), Aromatase Inhibitoren (z.B. Anastrozol, Letrozol, Liarozol, Vorozol, Exemestan, Atamestan,), LHRH Agonisten und Antagonisten (z.B. Goserelin Acetat, Luprolid), Inhibitoren von Wachstumsfaktoren (growth factors wie zum Beispiel "platelet derived growth factor" und "hepatocyte growth factor", Inhibitoren sind z. B. "growth factor"-Antikörper, "growth factor receptor"-Antikörper und Tyrosinekinase Inhibitoren, wie zum Beispiel Gefitinib, Imatinib, Lapatinib und Trashizumab); Antimetabolite (z.B. Antifolate wie Methotrexat, Raltitrexed, Pyrimidinanaloge wie 5-Fluorouracil, Capecitabin und Gemcitabin, Purin- und Adenosinanaloge wie Mercaptopurin, Thioguanin, Cladribin und Pentostatin, Cytarabin, Fludarabin); Antitumor-Antibiotica (z.B. Anthracycline wie Doxorubicin, Daunorubicin, Epirubicin und Idarubicin, Mitomycin-C, Bleomycin, Dactinomycin, Plicamycin, Streptozocin); Platinderivative (z.B. Cisplatin, Oxaliplatin, Carboplatin); Alkylierungsagentien (z.B. Estramustin, Meclorethamin, Melphalan, Chlorambucil, Busulphan, Dacarbazin, Cyclophosphamid, Ifosfamid, Temozolomid, Nitrosoharnstoffe wie zum Beispiel Carmustin und Lomustin, Thiotepa); antimitotische Agentien (z.B. Vinca-Alkaloide wie zum Beispiel Vinblastin, Vindesin, Vinorelbin und Vincristin; und Taxane wie Paclitaxel, Docetaxel); Topoisomerase Inhibitoren (z.B. Epipodophyllotoxine wie zum Beispiel Etoposid und Etopophos, Teniposid, Amsacrin, Topotecan, Irinotecan; Mitoxantron) und verschiedene Chemotherapeutica wie Amifostin, Anagrelid, Clodronat, Filgrastin, Interferon alpha, Leucovorin, Rituximab, Procarbazin, Levamisol, Mesna, Mitotan, Pamidronat und PorFimer.

Geeignete Anwendungsformen sind beispielsweise Tabletten, Kapseln, Zäpfchen, Lösungen, - insbesondere Lösungen zur Injektion (s.c., i.v., i.m.) und Infusion - Säfte, Emulsionen oder dispersible Pulver. Hierbei soll der Anteil der pharmazeutisch wirksamen Verbindung(en) jeweils im Bereich von 0.1 - 90 Gew.%, bevorzugt 0.5 - 50 Gew.-% der Gesamtzusammensetzung liegen, das heißt ein Mengen die ausreichend sind, um den unten angegebenen Dosierungsbereich zu erreichen. Die genannten Dosen können, falls erforderlich, mehrmals täglich gegeben werden.

Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektions- und Infusionslösungen werden in üblicher Weise, z.B. unter Zusatz von Isotonantien, Konservierungsmitteln, wie p-Hydroxybenzoate, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösemittel als Lösevermittler bzw. Hilfslösemittel eingesetzt werden können, hergestellt und in Injektionsflaschen oder Ampullen oder Infusionsflaschen abgefüllt.
Die eine oder mehrere Wirkstoffe beziehungsweise Wirkstoffkombinationen enthaltenden Kapseln können beispielsweise hergestellt werden indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuss- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Die Applikation erfolgt in üblicher Weise, vorzugsweise oral oder transdermal, insbesondere bevorzugt oral. Im Falle der oralen Anwendung können die Tabletten selbstverständlich außer den genannten Trägerstoffen auch Zusätze, wie z.B. Natriumcitrat, Calciumcarbonat und Dikalziumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wässriger Suspensionen können die Wirkstoffe außer den oben genannten Hilfsstoffen mit verschiedenen Geschmacksautbesserern oder Farbstoffen versetzt werden.

Für den Fall der parenteralen Anwendung können Lösungen der Wirkstoffe unter Verwendung geeigneter flüssiger Trägermaterialien eingesetzt werden.

Die Dosierung für die intravenöse Anwendung liegt bei 1 - 1000 mg pro Stunde, vorzugsweise zwischen 5 - 500 mg pro Stunde.

Trotzdem kann es gegebenenfalls notwendig sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein,'mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Formulierungsbeispiele illustrieren die vorliegende Erfindung ohne sie jedoch in ihrem Umfang zu beschränken:

### Pharmazeutische Formulierungsbeispiele

| A) | Tabletten | pro Tablette |
|---|---|---|
| | Wirkstoff nach Formel (1) | 100 mg |
| | Milchzucker | 140 mg |
| | Maisstärke | 240 mg |
| | Polyvinylpyrrolidon | 15 mg |
| | Magnesiumstearat | 5mg |
| | | 5̅0̅0̅ m̅g̅ |

Der fein gemahlene Wirkstoff, Milchzucker und ein Teil der Maisstärke werden miteinander vermischt. Die Mischung wird gesiebt, danach mit einer Lösung von Polyvinylpyrrolidon in Wasser befeuchtet, geknetet, feucht granuliert und getrocknet. Das Granulat, der Rest der Maisstärke und das Magnesiumstearat werden gesiebt und miteinander vermischt. Das Gemisch wird zu Tabletten geeigneter Form und Größe verpresst.

| B) | Tabletten | pro Tablette |
|---|---|---|
| | Wirkstoff nach Formel (1) | 80 mg |
| | Milchzucker | 55 mg |
| | Maisstärke | 190 mg |
| | Mikrokristalline Cellulose | 35 mg |
| | Polyvinylpyrrolidon | 15 mg |
| | Natrium-carboxymethylstärke | 23 mg |
| | Magnesiumstearat | 2 mg |
| | | 4̅0̅0̅ m̅g̅ |

Der fein gemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natriumcarboxymethylstärke und das Magnesiumstearat, vermischt und verpresst das Gemisch zu Tabletten geeigneter Größe.

| C) | Ampullenlösung | |
|---|---|---|
| | Wirkstoff nach Formel (1) | 50 mg |
| | Natriumchlorid | 50 mg |
| | Aqua pro inj. | 5 ml |

Der Wirkstoff wird bei Eigen-pH oder gegebenenfalls bei pH 5,5 - 6,5 in Wasser gelöst und mit Natriumchlorid als Isotonans versetzt. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff

### Referenzliste

Adams, RR., Wheatley,S.P., Gouldsworthy,A.M., Kandels-Lewis,S.E., Carmena,M., Smythe,C., Gerloff,D.L., and Earnshaw, W.C. (2000). INCENP binds the Aurora-related kinase AIRK2 and is required to target it to chromosomes, the central spindle and cleavage furrow. Curr. Biol. 10, 1075-1078.
Andrews,P.D., Knatko,E., Moore,W.J., and Swedlow,J.R (2003). Mitotic mechanics: the auroras come into view. Curr. Opin. Cell Biol. 15, 672-683.
Bayliss,R., Sardon,T., Vemos,I., and Conti,E. (2003). Structural basis of Aurora-A activation by TPX2 at the mitotic spindle. Mol. Cell 12, 851-862.
Bischoff,J.R., Anderson,L., Zhu,Y., Mossie,K., Ng,L., Souza,B., Schryver,B., Flanagan,P., Clairvoyant,F., Ginther,C., Chan,C.S., Novotny,M., Slamon,D.J., and Plowman,G.D. (1998). A homologue of Drosophila aurora kinase is oncogenic and amplified in human colorectal cancers. EMBO J. 17, 3052-3065.
Bishop,J.D. and Schumacher,J.M. (2002). Phosphorylation of the carboxyl terminus of inner centromere protein (INCENP) by the Aurora B Kinase stimulates Aurora B kinase activity. J. Biol. Chem 277, 27577-27580.
Bolton,M.A., Lan,W., Powers,S.E., McCleland,M.L., Kuang,J., and Stukenberg,P.T. (2002). Aurora B kinase exists in a complex with survivin and INCENP and its kinase activity is stimulated by survivin binding and phosphorylation. Mol. Biol. Cell 13, 3064-3077.
Carmena,M. and Earnshaw, W.C: (2003). The cellular geography of aurora kinases. Naht Rev. Mol. Cell Biol. 4, 842-854..
Csomos,P., Bernath,G., and Fueloep,F. (2002). A novel preparation of 2-aminocyclopentanecarboxamides. Monatsh. Chem., 133(8), 1077-1084
Ditchfield,C., Johnson,V.L., Tighe,A., Ellston, R., Haworth,C., Johnson,T., Mortlock,A., Keen,N., and Taylor,S.S. (2003). Aurora B couples chromosome alignment with anaphase by targeting BubRl, Mad2, and Cenp-E to kinetochores. J. Cell Biol. 161, 267-280.
Eyers,P.A. and Maller,J.L. (2004). Regulation of Xenopus Aurora A activation by TPX2. J. Biol. Chem. 279, 9008-9015.
Forro,E., and Fueloep,F.(2003) Lipase-Catalyzed Enantioselective Ring Opening of Unactivated Alicyclic-Fused β-Lactams in an Organic Solvent. Org. Lett. 5, 1209-1211.
Glover,D.M., Hagan,LM., and Tavares,A.A. (1998). Polo-like kinases: a team that plays throughout mitosis. Genes Dev. 12, 3777-3787.
Gorbsky,G.J. (2004). Mitosis: MCAK under the aura of Aurora B. Curr. Biol. 14, R346-R348.
Gruneberg,U., Neef, R, Honda,R, Nigg,E.A., and Barr,F.A. (2004). Relocation of Aurora B from centromeres to the central spindle at the metaphase to anaphase transition requires MKlp2. J. Cell Biol. 166, 167-172.
Hauf,S., Cole,R.W., LaTerra,S., Zimmer,C., Schnapp,G., Walter,R, Heckel,A., van Meel,J., Rieder,C.L., and Peters,J.M. (2003). The small molecule Hesperadin reveals a role for Aurora B in correcting kinetochore-microtubule attachment and in maintaining the spindle assembly checkpoint. J. Cell Biol. 161, 281-294.
Hirota,T., Kunitoku,N., Sasayama,T., Marumoto,T., Zhang,D., Nitta,M., Hatakeyama,K., and Saya,H. (2003). Aurora-A and an interacting activator, the LIM protein Ajuba, are required for mitotic commitment in human cells. Cell 114, 585-598.
Honda,R., Korner,R., and Nigg,E.A. (2003). Exploring the functional interactions between Aurora B, INCENP, and survivin in mitosis. Mol. Biol. Cell 14, 3325-3341.
Kaitna,S., Mendoza,M., Jantsch-Plunger,V., and Glotzer,M. (2000). Incenp and an auroralike kinase form a complex essential for chromosome segregation and efficient completion of cytokinesis. Curr. Biol. 10, 1172-1181.
Katayama,H., Ota,T., Jisaki,F., Ueda,Y., Tanaka,T., Odashima,S., Suzuki,F., Terada,Y., and Tatsuka,M. (1999). Mitotic kinase expression and colorectal cancer progression. J. Natl. Cancer Inst. 91, 1160-1162.
Keen,N. and Taylor,S. (2004). Aurora-kinase inhibitors as anticancer agents. Nat. Rev. Cancer 4, 927-936.
Kufer,T.A., Sillje,H.H., Korner,R., Gruss,O.J., Meraldi,P., and Nigg,E.A: (2002). Human TPX2 is required for targeting Aurora-A kinase to the spindle. J. Cell Biol. 158, 617-623.
Lane,H.A. and Nigg,E.A. (1996). Antibody microinjection reveals an essential role for human polo-like kinase 1 (Plk1) in the functional maturation of mitotic centrosomes. J. Cell Biol. 135, 1701-1713.
Li,X., Sakashita,G., Matsuzaki,H., Sugimoto,K., Kimura,K., Hanaoka,F., Taniguchi,H., Furukawa,K., and Urano,T. (2004). Direct association with inner centromere protein (INCENP) activates the novel chromosomal passenger protein, Aurora-C. J. Biol. Chem. 279, 47201-47211.
Mayer,T.U., Kapoor,T.M., Haggarty,S.J., King,R.W., Schreiber,S.L., and Mitchison,T.J. (1999). Small molecule inhibitor of mitotic spindle bipolarity identified in a phenotypebased screen. Science 286, 971-974.
Meraldi,P., Honda,R., and Nigg,E.A. (2004). Aurora kinases link chromosome segregation and cell division to cancer susceptibility. Curr. Opin. Genet. Dev. 14, 29-36.
Murata-Hori,M. and Wang,Y.L. (2002). The kinase activity of aurora B is required for kinetochore-microtubule interactions during mitosis. Curr. Biol. 12, 894-899.
Nigg,E.A. (2001). Mitotic kinases as regulators of cell division and its checkpoints. Nat. Rev. Mol. Cell Biol. 2, 21-32.
Nishio,K., Ishida,T., Arioka,H., Kurokawa,H., Fukuoka,K., Nomoto,T., Fukumoto,H., Yokote,H., and Saijo,N. (1996). Antitumor effects ofbutyrolactone I, a selective cdc2 kinase inhibitor, on human lung cancer cell lines. Anticancer Res. 16, 3387-3395.
Nurse,P. (1990). Universal control mechanism regulating onset of M-phase. Nature 344, 503-508.
Ota,T., Suto,S., Katayama.H., Han,Z.B., Suzuki,F., Maeda,M., Tanino,M., Terada,Y., and Tatsuka,M. (2002). Increased mitotic phosphorylation of histone H3 attributable to AIM-1/Aurora-B overexpression contributes to chromosome number instability. Cancer Res. 62, 5168-5177.
Qian,Y.W., Erikson,E., Taieb,F.E., and Maller,J.L. (2001). The polo-like kinase Plx1 is required for activation of the phosphatase Cdc25C and cyclin B-Cdc2 in Xenopus oocytes. Mol. Biol. Cell 12, 1791-1799.
Sasai,K., Katayama,H., Stenoien,D.L., Fujii,S., Honda,R., Kimura,M., Okano,Y., Tatsuka,M., Suzuki,F., Nigg,E.A., Earnshaw,W.C., Brinkley,W.R., and Sen,S. (2004). Aurora-C kinase is a novel chromosomal passenger protein that can complement Aurora-B kinase function in mitotic cells. Cell Motil. Cytoskeleton 59, 249-263.
Satinover,D.L., Leach,C.A., Stukenberg,P.T., and Brautigan,D.L. (2004). Activation of Aurora-A kinase by protein phosphatase inhibitor-2, a bifunctional signaling protein. Proc. Natl. Acad. Sci. U. S. A 101, 8625-8630.
Schiff,P.B. and Horwitz,S.B. (1980). Taxol stabilizes microtubules in mouse fibroblast cells. Proc. Natl. Acad. Sci. U. S. A 77, 1561-1565.
Vigneron,S., Prieto,S., Bernis,C., Labbe,J.C., Castro,A., and Lorca,T. (2004). Kinetochore localization of spindle checkpoint proteins: who controls whom? Mol. Biol. Cell 15, 4584-4596.
Zhou,H., Kuang,J., Zhong,L., Kuo,W.L., Gray,J.W., Sahin,A., Brinkley,B.R., and Sen,S. (1998). Tumour amplified kinase STK15/BTAK induces centrosome amplification, aneuploidy and transformation. Nat Genet. 20, 189-193.

## Patentansprüche

1. Verbindungen der allgemeinen Formel (1), 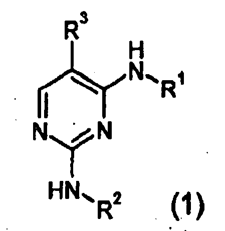 worin
R¹ ein Rest, substituiert mit R⁵ und gegebenenfalls mit einem oder mehreren R⁴, ausgewählt aus der Gruppe bestehend aus C₃₋₁₀Cycloalkyl und 3-8 gliedriges Heterocycloalkyl;
R² ein Rest, gegebenenfalls substituiert mit einem oder mehreren R⁴, ausgewählt aus der Gruppe bestehend aus C₁₋₆Alkyl, C₃₋₁₀Cycloalkyl, 3-8 gliedriges Heterocycloalkyl, C₆₋₁₅Aryl und 5-12 gliedriges Heteroaryl;
R³ ein Rest ausgewählt aus der Gruppe bestehend aus Wasserstoff, Halogen, -CN, -NO₂, C₁₋₄Alkyl, C₁₋₄Haloalkyl, C₃₋₁₀Cycloalkyl, C₄₋₁₆Cycloalkylalkyl und C₇₋₁₆Arylalkyl;
R⁴ ein Rest ausgewählt aus der Gruppe bestehend aus R^{a}, R^{b} und R^{a} substituiert mit einem oder mehreren, gleich oder verschiedenen R^{e} und/oder R^{b};
R⁵ ein geeigneter Rest ausgewählt aus der Gruppe bestehend aus -C(O)R^{c}, -C(O)NR^{c}R^{c}, -S(O)₂R^{c}, -N(R^{f})S(O)₂R^{c}, -N(R^{f})C(O)R^{c}, -N(R^{f})C(O)OR^{c}, und -N(R^{f})C(O)NR^{c}R^{c};
jedes R^{a} ist unabhängig voneinander ausgewählt aus der Gruppe bestehend aus C₁₋₆Alkyl, C₃₋₁₀Cycloalkyl, C₄₋₁₆Cycloalkylalkyl, C₆₋₁₀Aryl, C₇₋₁₆Arylalkyl, 2-6 gliedriges Heteroalkyl, 3-8 gliedriges Heterocycloalkyl, 4-14 gliedriges Heterocycloalkylalkyl, 5-12 gliedriges Heteroaryl und 6-18 gliedriges Heteroarylalkyl;
jedes R^{b} ist eine geeigneter Rest und jeweils unabhängig voneinander ausgewählt aus der Gruppe bestehend aus =O, -OR^{c}, C₁₋₃Haloalkyloxy, -OCF₃, =S, -SR^{c}, =NR^{c}, =NOR^{c}, -NR^{c}R^{c}, Halogen, -CF₃, -CN, -NC, -OCN, -SCN, -NO₂, -S(O)R^{c}, -S(O)₂R^{c}, S(O)₂OR^{c}, -S(O)NR^{c}R^{c}, -S(O)₂NR^{c}R^{c}, -OS(O)R^{c}, -OS(O)₂R^{c}, -OS(O)₂OR^{c}, OS(O)₂NR^{c}R^{c}, -C(O)R^{c}, -C(O)OR^{c}, -C(O)NR^{c}R^{c}, -CN(R^{f})NR^{c}R^{c}, -CN(OH)R^{c}, -CN(OH)NR^{c}R^{c}, -OC(O)R^{c}, -OC(O)OR^{c}, -OC(O)NR^{c}R^{c}, -OCN(R^{f})NR^{c}R^{c}, -N(R^{f})C(O)R^{c}, -N(R^{f})C(S)R^{c}, -N(R^{f})S(O)₂R^{c}, -N(R^{f})C(O)OR^{c}, -N(R^{f})C(O)NR^{c}R^{c}, -[N(R^{f})C(O)]₂R^{c}, -N[C(O)]₂R^{c}, -N[C(O)]₂OR^{c}, -[N(R^{f})C(O)]₂OR^{c} und -N(R^{f})CN(R^{f})NR^{c}R^{c};
jedes R^{c} ist unabhängig voneinander Wasserstoff oder ein gegebenenfalls mit einem oder mehreren, gleich oder verschiedenen R^{d} und/oder R^{e} substituierter Rest ausgewählt aus der Gruppe bestehend aus C₁₋₆Alkyl, C₃₋₁₀Cycloalkyl, C₄₋₁₁Cycloalkylalkyl, C₆₋₁₀Aryl, C₇₋₁₆Arylalkyl, 2-6 gliedriges Heteroalkyl, 3-8 gliedriges Heterocycloalkyl, 4-14 gliedriges Heterocycloalkylalkyl, 5-12 gliedriges Heteroaryl und 6-18 gliedriges Heteroarylalkyl,
jedes R^{d} ist unabhängig voneinander Wasserstoff oder ein gegebenenfalls mit einem oder mehreren, gleich oder verschiedenen R^{e} und/oder R^{f} substituierter Rest ausgewählt aus der Gruppe bestehend aus C₁₋₆Alkyl, C₃₋₈Cycloalkyl, C₄₋₁₁Cycloalkylalkyl, C₆₋₁₀Aryl, C₇₋₁₆Arylalkyl, 2-6 gliedriges Heteroalkyl, 3-8 gliedriges Heterocycloalkyl, 4-14 gliedriges Heterocycloallcylalkyl, 5-12 gliedriges Heteroaryl und 6-18 gliedriges Heteroarylalkyl;
jedes R^{e} ist eine geeigneter Rest und jeweils unabhängig voneinander ausgewählt aus der Gruppe bestehend aus =O, -OR^{f}, C₁₋₃Haloalkyloxy, -OCF₃, =S, -SR^{f}, =NR^{f}, NOR^{f}, -NR^{f}R^{f}, Halogen, -CF₃, -CN, -NC, -OCN, -SCN, -NO₂, -S(O)R^{f}, -S(O)₂R^{f}, -S(O)₂OR^{f},-S(O)NR^{f}R^{f}, -S(O)₂NR^{f}R^{f}, -OS(O)R^{f}, -OS(O)₂R^{f}, -OS(O)₂OR^{f}, -OS(O)₂NR^{f}R^{f}, -C(O)R^{f}, -C(O)OR^{f}, -C(O)NR^{f}R^{f}, -CN(R^{g})NR^{f}R^{f}, -CN(OH)R^{f}, -C(NOH)NR^{f}R^{f}, -OC(O)R^{f}, -OC(O)OR^{f}, -OC(O)NR^{f}R^{f}, -OCN(R^{g})NR^{f}R^{f}, -N(R^{g})C(O)R^{f}, -N(R^{g})C(S)R^{f}, -N(R^{g})S(O)₂R^{f}, -N(R^{d})C(O)OR^{f}, -N(R^{g})C(O)NR^{f}R^{f}, und -N(R^{g})CN(R^{f})NR^{f}R^{f};
jedes R^{f} ist unabhängig voneinander Wasserstoff oder ein gegebenenfalls mit einem oder mehreren, gleich oder verschiedenen R^{g} substituierter Rest ausgewählt aus der Gruppe bestehend aus , C₁₋₆Alkyl, C₃₋₈Cycloalkyl, C₄₋₁Cycloalkylalkyl, C₆₋₁₀Aryl, C₇₋₁₆Arylalkyl, 2-6 gliedriges Heteroalkyl, 3-8 gliedriges Heterocycloalkyl, 4-14 gliedriges Heterocycloalkylalkyl, 5-12 gliedriges Heteroaryl und 6-18 gliedriges Heteroarylalkyl;
jedes R^{g} ist unabhängig voneinander Wasserstoff, C₁₋₆Alkyl, C₃₋₈Cycloalkyl, C₄₋₁₁Cycloalkylalkyl, C₆₋₁₀Aryl, C₇₋₁₆Arylalkyl, 2-6 gliedriges Heteroalkyl, 3-8 gliedriges Heterocycloalkyl, 4-14 gliedriges Heterocycloalkyl, 5-12 gliedriges Heteroaryl und 6-18 gliedriges Heteroarylalkyl bedeuten, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

2. Verbindungen nach Anspruch 1, wobei R³ ein Rest ausgewählt aus der Gruppe bestehend aus Halogen und C₁₋₄Haloalkyl bedeutet.

3. Verbindungen nach Anspruch 2, wobei R³ -CF₃ bedeutet.

4. Verbindungen nach Anspruch 1 bis 3, wobei R² C₆₋₁₀Aryl oder 5-12 gliedriges Heteroaryl, gegebenenfalls substituiert mit einem oder mehreren R⁴, bedeutet

5. Verbindungen nach Anspruch 4, wobei R² Phenyl, gegebenenfalls substituiert mit einem oder mehreren R⁴, bedeutet.

6. Verbindungen der allgemeinen Formel (1A), 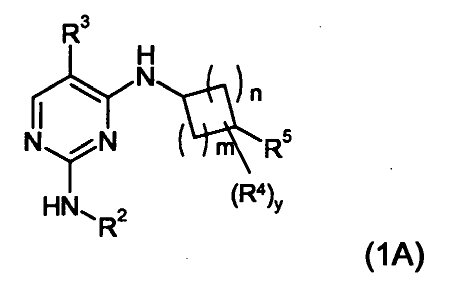 wobei
n gleich 0 oder 1, und
m gleich 1 *-* 5, und
y gleich 0 bis 6, bedeuten und die übrigen Reste wie vorstehend definiert sind.

7. Verbindungen nach Anspruch 6, wobei R³ ein Rest ausgewählt aus der Gruppe bestehend aus Halogen und C₁₋₄Haloalkyl bedeutet.

8. Verbindungen nach Anspruch 7, wobei R³ CF₃ bedeutet.

9. Verbindungen nach Anspruch 6 - 8, wobei R² C₆₋₁₀Aryl oder 5-12 gliedriges Heteroaryl, gegebenenfalls substituiert mit einem oder mehreren R⁴, bedeutet.

10. Verbindungen nach Anspruch 6 - 9, wobei R² Phenyl, gegebenenfalls substituiert mit einem oder mehreren R⁴, bedeutet.

11. Verbindungen - oder deren pharmazeutisch wirksamen Salze - nach Anspruch 1 bis 10 zur Verwendung als Arzneimittel.

12. Verbindungen - oder deren pharmazeutisch wirksamen Salze - nach Anspruch 1 bis 10 zur Herstellung eines Arzneimittels mit antiproliferativer Wirkung.

13. Pharmazeutische Zubereitungen, enthaltend als Wirkstoff eine oder mehrere Verbindungen der allgemeinen Formel (1) oder (1A) gemäß einem der Ansprüche 1 bis 10 oder deren physiologisch verträglichen Salze gegebenenfalls in Kombination mit üblichen Hilfs- und/oder Trägerstoffen.

14. Verwendung von Verbindungen der allgemeinen Formel (1) oder (1A) gemäß Anspruch 1 bis 10 zur Herstellung eines Arzneimittels zur Behandlung und/oder Prävention von Krebs, Infektionen, Entzündungs- und Autoimmunerkrankungen.

15. Pharmazeutische Präparation umfassend eine Verbindung der allgemeinen Formel (1) oder (1A) gemäß Anspruch 1 bis 10 und mindestens eine weitere zytostatische oder zytotoxische, von Formel (1) oder (1A) verschiedene Wirksubstanz, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

## Claims

1. Compounds of general formula (1), 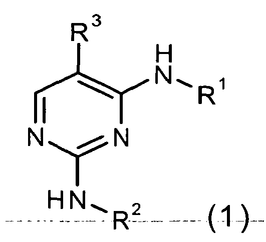 wherein
R¹ denotes a group, substituted by R⁵ and optionally by one or more R⁴, selected from among C₃₋₁₀-cycloalkyl and 3-8-membered heterocycloalkyl;
R² denotes a group, optionally substituted by one or more R⁴, selected from among C₁₋₆-alkyl, C₃₋₁₀-cycloalkyl, 3-8-membered heterocycloalkyl, C₆₋₁₅ aryl and 5-12-membered heteroaryl;
R³ denotes a group selected from among hydrogen, halogen, -CN, -NO₂, C₁₋₄ alkyl, C₁₋₄ haloalkyl, C₃₋₁₀-cycloalkyl, C₄₋₁₆-cycloalkylalkyl and C₇₋₁₆ arylalkyl;
R⁴ denotes a group selected from among R^{a}, R^{b} and R^{a} substituted by one or more identical or different R^{c} and/or R^{b};
R⁵ denotes a suitable group selected from among -C(O)R^{c}, -C(O)NR^{c}R^{c}, -S(O)₂R^{c}, -N(R^{f})S(O)₂R^{c}, -N(R^{f})C(O)R^{c}, -N(R^{f})C(O)OR^{c}, and -N(R^{f})C(O)NR^{c}R^{c};
each R^{a} is selected independently of one another from among C₁₋₆ alkyl, C₃₋₁₀-cycloalkyl, C₄₋₆-cycloalkylalkyl, C₆₋₁₀ aryl, C₇₋₁₆ arylalkyl, 2-6-membered heteroalkyl, 3-8-membered heterocycloalkyl, 4-14-membered heterocycloalkylalkyl, 5-12-membered heteroaryl and 6-18-membered heteroarylalkyl;
each R^{b} is a suitable group and each selected independently of one another from among =O, -OR^{c}, C₁₋₃ haloalkyloxy, -OCF₃, =S, -SR^{c}, =NR^{c}, =NOR^{c}, -NR^{c}R^{c}, halogen, -CF₃, -CN, -NC, -OCN, -SCN, -NO₂, -S(O)R^{c}, -S(O)₂R^{c}, -S(O)₂OR^{c},-S(O)NR^{c}R^{c}, -S(O)₂NR^{c}R^{c}, -OS(O)R^{c}, -OS(O)₂R^{c}, -OS(O)₂OR^{c}, -OS(O)₂NR^{c}R^{c},-C(O)R^{c}, -C(O)OR^{c}, -C(O)NR^{c}R^{c}, -CN(R^{f})NR^{c}R^{c}, -CN(OH)R^{c}, -CN(OH)NR^{c}R^{c},-OC(O)R^{c}, -OC(O)OR^{c}, -OC(O)NR^{c}R^{c}, -OCN(R^{f})NR^{c}R^{c}, -N(R^{f})C(O)R^{c}, -N(R^{f})C(S)R^{c}, -N(R^{f})S(O)₂R^{c}, -N(R^{f})C(O)OR^{c}, -N(R^{f})C(O)NR^{c}R^{c}, -[N(R^{f})C(O)]₂R^{c}, -N[C(O)]₂R^{c}, -N[C(O)]₂OR^{c}, -[N(R^{f})C(O)]₂OR^{c} and -N(R^{f})CN(R^{f})NR^{c}R^{c};
each R^{c} independently of one another is hydrogen or a group optionally substituted by one or more identical or different R^{d} and/or R^{e} selected from among C₁₋₆ alkyl, C₃₋₁₀-cycloalkyl, C₄₋₁₁-cycloalkylalkyl, C₆₋₁₀ aryl, C₇₋₁₆ arylalkyl, 2-6-membered heteroalkyl, 3-8-membered heterocycloalkyl, 4-14-membered heterocycloalkylalkyl, 5-12-membered heteroaryl and 6-18-membered heteroarylalkyl,
each R^{d} independently of one another is hydrogen or a group optionally substituted by one or more identical or different R^{e} and/or R^{f} selected from among C₁₋₆ alkyl, C₃₋₈-cycloalkyl, C₄₋₁₁-cycloalkylalkyl, C₆₋₁₀ aryl, C₇₋₁₆ arylalkyl, 2-6-membered heteroalkyl, 3-8-membered heterocycloalkyl, 4-14-membered heterocycloalkylalkyl, 5-12-membered heteroaryl and 6-18-membered heteroarylalkyl;
each R^{e} is a suitable group and each selected independently of one another from among =O, -OR^{f}, C₁₋₃ haloalkyloxy, -OCF₃, =S, -SR^{f}, =NR^{f}, =NOR^{f}, -NR^{f}R^{f}, halogen, -CF₃, -CN, -NC, -OCN, -SCN, -NO₂ -S(O)R^{f}, -S(O)₂R^{f}, -S(O)₂OR^{f}, -S(O)NR^{f}R^{f}, -S(O)₂NR^{f}R^{f}, -OS(O)R^{f}, -OS(O)₂R^{f}, -OS(O)₂OR^{f}, -OS(O)₂NR^{f}R^{f}, -C(O)R^{f}, -C(O)OR^{f}, -C(O)NR^{f}R^{f}, -CN(R^{g})NR^{f}R^{f}, -CN(OH)R^{f}, -C(NOH)NR^{f}R^{f}, -OC(O)R^{f}, -OC(O)OR^{f}, -OC(O)NR^{f}R^{f}, -OCN(R^{g})NR^{f}R^{f}, -N(R^{g})C(O)R^{f}, -N(R^{g})C(S)R^{f}, -N(R^{g})S(O)₂R^{f}, -N(R^{d})C(O)OR^{f}, -N(R^{g})C(O)NR^{f}R^{f}, and -N(R^{g})CN(R^{f})NR^{f}R^{f};
each R^{f} independently of one another is hydrogen or a group optionally substituted by one or more identical or different R^{g} selected from among C₁₋₆ alkyl, C₃₋₈-cycloalkyl, C₄₋₁₁-cycloalkylalkyl, C₆₋₁₀ aryl, C₇₋₁₆ arylalkyl, 2-6-membered heteroalkyl, 3-8-membered heterocycloalkyl, 4-14-membered heterocycloalkylalkyl, 5-12-membered heteroaryl and 6-18-membered heteroarylalkyl;
each R^{g} independently of one another is hydrogen, C₁₋₆ alkyl, C₃₋₈-cycloalkyl, C₄₋₁₁-cycloalkylalkyl, C₆₋₁₀ aryl, C₇₋₁₆ arylalkyl, 2-6-membered heteroalkyl, 3-8-membered heterocycloalkyl, 4-14-membered heterocycloalkyl, 5-12-membered heteroaryl and 6-18-membered heteroarylalkyl, optionally in the form of the tautomers, the racemates, the enantiomers, the diastereomers and the mixtures thereof, and optionally the pharmacologically acceptable acid addition salts thereof.

2. Compounds according to claim 1, wherein R³ denotes a group selected from among halogen and C₁₋₄haloalkyl.

3. Compounds according to claim 2, wherein R³ denotes -CF₃.

4. Compounds according to claim 1 to 3, wherein R² denotes C₆₋₁₀ aryl or 5-12-membered heteroaryl, optionally substituted by one or more R⁴.

5. Compounds according to claim 4, wherein R² denotes phenyl, optionally substituted by one or more R⁴.

6. Compounds of general formula (1A), 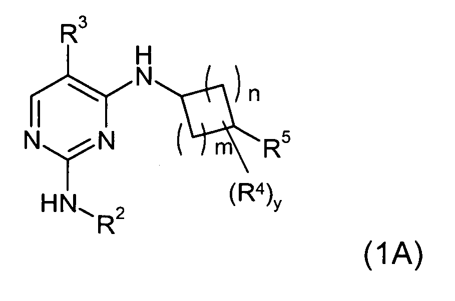 wherein
n is equal to 0 or 1, and
m is equal to 1 - 5, and
y is equal to 0 to 6, and the remaining groups are as hereinbefore defined.

7. Compounds according to claim 6, wherein R³ denotes a group selected from among halogen and C₁₋₄haloalkyl.

8. Compounds according to claim 7, wherein R³ denotes CF₃.

9. Compounds according to claim 6 - 8, wherein R² denotes C₆₋₁₀ aryl or 5-12-membered heteroaryl, optionally substituted by one or more R⁴.

10. Compounds according to claim 6 - 9, wherein R² denotes phenyl, optionally substituted by one or more R⁴.

11. Compounds - or the pharmaceutically active salts thereof - according to claim 1 to 10 for use as pharmaceutical compositions.

12. Compounds - or the pharmaceutically active salts thereof - according to claim 1 to 10 for preparing a pharmaceutical composition with an antiproliferative activity.

13. Pharmaceutical preparations, containing as active substance one or more compounds of general formula (1) or (1A) according to one of claims 1 to 10 or the physiologically acceptable salts thereof optionally in conjunction with conventional excipients and/or carriers.

14. Use of compounds of general formula (1) or (1A) according to claim 1 to 10 for preparing a pharmaceutical composition for the treatment and/or prevention of cancer, infections, inflammatory and autoimmune diseases.

15. Pharmaceutical preparation comprising a compound of general formula (1) or (1A) according to claim 1 to 10 and at least one other cytostatic or cytotoxic active substance, different from formula (1) or (1A), optionally in the form of the tautomers, the racemates, the enantiomers, the diastereomers and the mixtures thereof, and optionally the pharmacologically acceptable acid addition salts thereof.

## Revendications

1. Composés de formule générale (1) 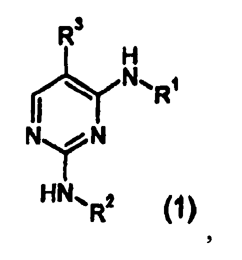 où
R¹ représente un radical, substitué par R⁵ et éventuellement par un ou plusieurs R⁴, choisi parmi le groupe constitué par un cycloalkyle en C₃₋₁₀ et un hétérocycloalkyle à 3-8 membres ;
R² représente un radical, éventuellement substitué par un ou plusieurs R⁴, choisi parmi le groupe constitué par un alkyle en C₁₋₆, un cycloalkyle en C₃₋₁₀, un hétérocycloalkyle à 3-8 membres, un aryle en C₆₋₁₅ et un hétéroaryle à 5-12 membres ;
R³ représente un radical choisi parmi le groupe constitué par un hydrogène, un halogène, un -CN, un -NO₂, un alkyle en C₁₋₄, un haloalkyle en C₁₋₄, un cycloalkyle en C₃₋₁₀, un cycloalkylalkyle en C₄₋₁₆ et un arylalkyle en C₇₋₁₆ ;
R⁴ représente un radical choisi parmi le groupe constitué par R^{a}, R^{b} et R^{a} substitué par un ou plusieurs R^{c} et/ou R^{b} identiques ou différents ;
R⁵ représente un radical approprié choisi parmi le groupe constitué par -C(O)R^{c},-C(O)NR^{c}R^{c}, -S(O)₂R^{c}, -N(R^{f})S(O)₂R^{c}, -N(R^{f})C(O)R^{c}, -N(R^{f})C(O)OR^{c} et-N(R^{f})C(O)NR^{c}R^{c} ;
chaque R^{a} est choisi indépendamment parmi le groupe constitué par un alkyle en C₁₋₆, un cycloalkyle en C₃₋₁₀, un cycloalkylalkyle en C₄₋₁₆, un aryle en C₆₋₁₀, un arylalkyle en C₇₋₁₆, un hétéroalkyle à 2-6 membres, un hétérocycloalkyle à 3-8 membres, un hétérocycloalkylalkyle à 4-14 membres, un hétéroaryle à 5-12 membres et un hétéroarylalkyle à 6-18 membres ;
chaque R^{b} est un radical approprié, chacun choisi indépendamment parmi le groupe constitué par =O, -OR^{c}, haloalkyloxy en C₁₋₃, -OCF₃, =S, -SR^{c}, =NR^{c}, =NOR^{c},-NR^{c}R^{c}, halogène, -CF₃, -CN, -NC, -OCN, -SCN, -NO₂, -S(O)R^{c}, -S(O)₂R^{c},-S(O)₂OR^{c}, -S(O)NR^{c}R^{c}, -S(O)₂NR^{c}R^{c}, -OS(O)R^{c}, -OS(O)₂R^{c}, -OS(O)₂OR^{c},-OS(O)₂NR^{c}R^{c}, -C(O)R^{c}, -C(O)OR^{c}, -C(O)NR^{c}R^{c}, -CN(R^{f})NR^{c}R^{c}, -CN(OH)R^{c},-CN(OH)NR^{c}R^{c}, -OC(O)R^{c}, -OC(O)OR^{c}, -OC(O)NR^{c}R^{c}, -OCN(R^{f})NR^{c}R^{c},-N(R^{f})C(O)R^{c}, -N(R^{f})C(S)R^{c}, - N(R^{f})S(O)2R^{c}, -N(R^{f})C(O)OR^{c}, -N(R^{f})C(O)NR^{c}R^{c},-[N(R^{f})C(O)]₂R^{c}, -N[C(O)]₂R^{c}, -N[C(O)]₂OR^{c}, -[N(R^{f})C(O)]₂OR^{c} et-N(R^{f})CN(R^{f})NR^{c}R^{c};
chaque R^{c} est indépendamment un hydrogène ou un radical éventuellement substitué avec un ou plusieurs R^{d} et/ou R^{e} identique ou différent, choisi parmi le groupe constitué par un alkyle en C₁₋₆, un cycloalkyle en C₃₋₁₀, un cycloalkylalkyle en C₄₋₁₁, un aryle en C₆₋₁₀, un arylalkyle en C₇₋₁₆, un hétéroalkyle à 2-6 membres, un hétérocycloalkyle à 3-8 membres, un hétérocycloalkylalkyle à 4-14 membres, un hétéroaryle à 5-12 membres et un hétéroarylalkyle à 6-18 membres ;
chaque R^{d} est indépendamment un hydrogène ou un radical éventuellement substitué avec un ou plusieurs R^{e} et/ou R^{f} identique ou différent, choisi parmi le groupe constitué par un alkyle en C₁₋₆, un cycloalkyle en C₃₋₈, un cycloalkylalkyle en C₄₋₁₁, un aryle en C₆₋₁₀, un arylalkyle en C₇₋₁₆, un hétéroalkyle à 2-6 membres, un hétérocycloalkyle à 3-8 membres, un hétérocycloalkylalkyle à 4-14 membres, un hétéroaryle à 5-12 membres et un hétéroarylalkyle à 6-18 membres ;
chaque R^{e} est un radical déterminé, chacun choisi indépendamment parmi le groupe constitué par =O, -OR^{f}, haloalkyloxy en C₁₋₃, -OCF₃, =S, -SR^{f}, =NR^{f}, =NOR^{f},-NR^{f}R^{f}, halogène, -CF₃, -CN, -NC, -OCN, -SCN, -NO₂, -S(O)R^{f}, -S(O)₂R^{f}, -S(O)₂OR^{f}, -S(O)NR^{f}R^{f}, -S(O)₂NR^{f}R^{f}, -OS(O)R^{f}, -OS(O)₂R^{f}, -OS(O)₂OR^{f}, -OS(O)₂NR^{f}R^{f},-C(O)R^{f}, -C(O)OR^{f}, -C(O)NR^{f}R^{f}, -CN(R^{g})NR^{f}R^{f}, -CN(OH)R^{f}, -C(NOH)NR^{f}R^{f},-OC(O)R^{f}, -OC(O)OR^{f}, -OC(O)NR^{f}R^{f}, -OCN(R^{g})NR^{f}R^{f}, -N(R^{g})C(O)R^{f},-N(R^{g})C(S)R^{f}, - N(R^{g})S(O)₂R^{f}, -N(R^{d})C(O)OR^{f}, -N(R^{g})C(O)NR^{f}R^{f} et-N(R^{g})CN(R^{f})NR^{f}R^{f} ;
chaque R^{f} est indépendamment un hydrogène ou un radical éventuellement substitué avec un ou plusieurs R^{g} identique ou différent, choisi parmi le groupe constitué par un alkyle en C₁₋₆, un cycloalkyle en C₃₋₈, un cycloalkylalkyle en C₄₋₁₁, un aryle en C₆₋₁₀, un arylalkyle en C₇₋₁₆, un hétéroalkyle à 2-6 membres, un hétérocycloalkyle à 3-8 membres, un hétérocycloalkylalkyle à 4-14 membres, un hétéroaryle à 5-12 membres et un hétéroarylalkyle à 6-18 membres ;
chaque R^{g} est indépendamment un hydrogène, un alkyle en C₁₋₆, un cycloalkyle en C₃₋₈, un cycloalkylalkyle en C₄₋₁₁, un aryle en C₆₋₁₀, un arylalkyle en C₇₋₁₆, un hétéroalkyle à 2-6 membres, un hétérocycloalkyle à 3-8 membres, un hétérocycloalkylalkyle à 4-14 membres, un hétéroaryle à 5-12 membres et un hétéroarylalkyle à 6-18 membres, éventuellement sous la forme des tautomères, des racémates, des énantiomères, des diastéréomères et des mélanges de ceux-ci, et éventuellement des sels d'addition acides de ceux-ci pharmacologiquement neutres.

2. Composés selon la revendication 1, dans lesquels R³ représente un radical choisi parmi le groupe constitué par un halogène et un haloalkyle en C₁₋₄.

3. Composés selon la revendication 2, dans lesquels R³ représente -CF₃.

4. Composés selon les revendications 1 à 3, dans lesquels R² représente un aryle en C₆₋₁₀ ou un hétéroaryle à 5-12 membres, éventuellement substitué par un ou plusieurs R⁴.

5. Composés selon la revendication 4, dans lesquels R² représente un phényle, éventuellement substitué par un ou plusieurs R⁴.

6. Composés de formule générale (1A), 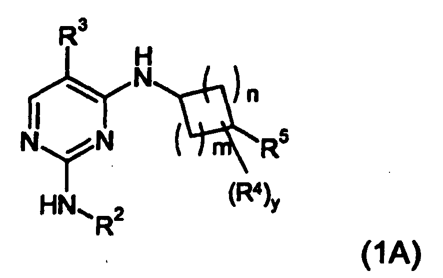 dans lesquels
n est égal à 0 ou 1, et m est égal à 1-5, et
y est égal à 0 à 6, et les autres radicaux sont définis comme précédemment.

7. Composés selon la revendication 6, dans lesquels R³ représente un radical choisi parmi le groupe constitué par un halogène et un haloalkyle en C₁₋₄.

8. Composés selon la revendication 7, dans lesquels R³ représente -CF₃.

9. Composés selon les revendications 6 à 8, dans lesquels R² représente un aryle en C₆₋₁₀ ou un hétéroaryle à 5-12 membres, éventuellement substitué par un ou plusieurs

10. Composés selon les revendications 6 à 9, dans lesquels R² représente un phényle, éventuellement substitué par un ou plusieurs R⁴.

11. Composés, ou sels pharmaceutiquement efficaces de ceux-ci, selon les revendications 1 à 10 pour une utilisation comme médicaments.

12. Composés, ou sels pharmaceutiquement efficaces de ceux-ci, selon les revendications 1 à 10 pour la fabrication de médicaments ayant un effet antiprolifératif.

13. Préparations pharmaceutiques, contenant comme substance active un ou plusieurs composés de formule générale (1) ou (1A) selon l'une des revendications 1 à 10 ou les sels physiologiquement acceptables de ceux-ci, éventuellement en association avec des adjuvants et/ou excipients habituels.

14. Utilisation de composés de formule générale (1) ou (1A) selon les revendications 1 à 10 pour la fabrication d'un médicament destiné au traitement et/ou à la prévention des cancers, des infections, des maladies inflammatoires et autoimmunes.

15. Préparation pharmaceutique comprenant un composé de formule générale (1) ou (1A) selon les revendications 1 à 10 et au moins une autre substance active cytostatique ou cytotoxique, différente de formule (1) ou (1A), éventuellement sous la forme de ses tautomères, de ses racémates, de ses énantiomères, de ses diastéréomères et des mélanges de ceux-ci, et éventuellement de ses sels d'addition acides pharmacologiquement neutres.
